(19) Europäisches Patentamt
European Patent Office
Office européen des brevets

(11) Numéro de publication: **0 034 536**
**B1**

# (12) FASCICULE DE BREVET EUROPEEN

(45) Date de publication du fascicule du brevet: **27.12.84**

(51) Int. Cl.$^3$: **C 07 D 501/36,** C 07 D 277/20, A 61 K 31/545

(21) Numéro de dépôt: **81400224.2**

(22) Date de dépôt: **13.02.81**

**(54) Nouvelles oximes dérivées de l'acide 3-alkyloxy ou 3-alkylthiométhyl 7-amino thiazolylacétamido céphalosporanique, leur préparation, leur application comme médicaments et les compositions les renfermant.**

(30) Priorité: **18.02.80 FR 8003479**

(43) Date de publication de la demande:
**26.08.81 Bulletin 81/34**

(45) Mention de la délivrance du brevet:
**27.12.84 Bulletin 84/52**

(84) Etats contractants désignés:
**AT BE CH DE GB IT LI LU NL SE**

(56) Documents cités:
**EP-A-0 029 557**
**EP-A-0 049 119**
**FR-A-2 119 074**
**FR-A-2 137 899**
**FR-A-2 348 218**
**FR-A-2 379 540**

**Le dossier contient des informations techniques présentées postérieurement au dépôt de la demande et ne figurant pas dans le présent fascicule.**

(73) Titulaire: **ROUSSEL-UCLAF**
**35, boulevard des Invalides**
**F-75007 Paris (FR)**

(72) Inventeur: **Heymes, René**
**72, rue Jean Jaurès**
**F-93230-Romainville (FR)**
Inventeur: **Pronine, Didier**
**65 bis, rue de la Feronne**
**F-93110 Rosny-sous-Bois (FR)**

(74) Mandataire: **Bourgouin, André et al**
**ROUSSEL-UCLAF Boîte postale no 9**
**102, route de Noisy F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 034 536 B1

## Description

La présente invention concerne de nouvelles oximes dérivées de l'acide 3-alkyloxy ou 3-alkylthio-méthyl 7-amino thiazolyl acétamido céphalosporanique, leur procédé de préparation, leur application comme médicaments et les compositions les renfermant.

Le brevet français 2.348.218 décrit des céphalosporines comportant un groupement amino thiazole et un radical méthoxyimino sur la chaîne latérale et en position 3 un groupement nucléophile attaché au noyau céphème par l'intermédiaire d'un groupement méthylène. En exemple figure le produit ayant un radical hydroxyméthyle en position 3.

Le brevet français 2.137.899 décrit de façon très générale des céphalosporines possédant sur la chaîne latérale un groupement hydroxyimino éthérifié. La Céfuroxime tombe dans cette formule générale.

Les produits de la présente demande possèdent une meilleure activité antibiotique que les produits des brevets cités.

L'invention a pour objet les produits de formule générale (I')

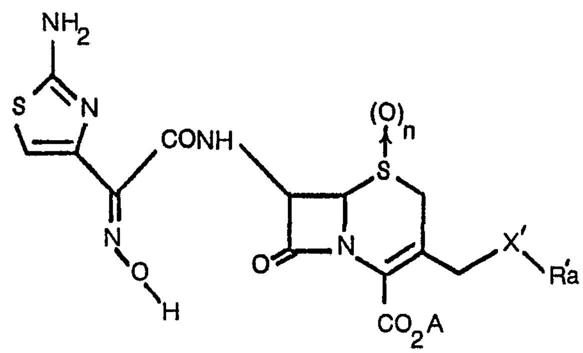

Isomères *syn* (I')

dans laquelle A représente un atome d'hydrogène, un équivalent de métal alcalin, alcalino-terreux, de magnésium, d'ammonium, ou d'une base organique aminée ou A représente le reste d'un groupement ester facilement clivable.

R'a représente un radical alkyle, éventuellement interrompu par un hétéroatome, alkényle ou alkynyle ayant au plus 6 atomes de carbone, linéaire ou ramifié ou un radical benzyle ou phényléthyle éventuellement substitué par un radical carboxy, amino, aminoalkyle, alkylamino, dialkylamino, dialkylaminoalkyle,

n est égal à 0, 1 ou 2,

X' représente un atome de soufre éventuellement oxydé sous forme de sulfoxyde de sulfone, ou un atome d'oxygène, ainsi que les sels des produits de formule (I') avec les acides minéraux ou organiques.

L'invention a notamment pour objet, parmi les produits de formule I', les produits répondant à la formule (I)

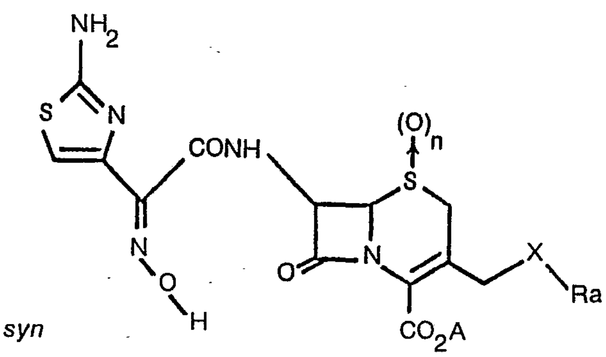

isomères *syn* (I)

dans laquelle A et n sont définis comme précédemment, Ra représente un radical alkyle, alkényle ou alkynyle ayant au plus 6 atomes de carbone, linéaire ou ramifié, ou un radical benzyle ou phényléthyle éventuellement substitué par un radical carboxy, amino, aminoalkyle, alkylamino, dialkylamino, dialkylaminoalkyle et X représente un atome de soufre ou un atome d'oxygène, ainsi que les sels des produits de formule (I) avec les acides minéraux ou organiques.

Parmi les valeurs de A on peut citer on équivalent de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements ester facilement clivables que peut représenter A, les groupements méthoxyméthyle, éthoxyméthyle, isopropyloxyméthyle, $\alpha$-méthoxyéthyle, $\alpha$-éthoxyéthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butyl carbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1-hexadécanoyloxyéthyle, 1-propionyloxypropyle, 1-méthoxycarbonyloxyéthyle, méthoxycarbonyloxyméthyle, 1-acétyloxybutyle, 1-acétyloxyhexyle, 1-acétyloxyheptyle, phtalidyle, 5,6-diméthoxyphtalidyle, tert-butylcarbonylméthyle, allyle, 2-chloroallyle, méthoxycarbonylméthyle, benzyle ou tert-butyle.

On peut encore citer entre autres restes de groupements esters qui peut représenter A, les groupements méthoxyéthoxyméthyle, diméthylaminoéthyle, cyanométhyle, tert-butyloxycarbonylméthyle, 2,2-éthylènedioxyéthyle, cyanoéthyle, 2,2-diméthoxyéthyle, 2-chloroéthoxyméthyle, 2-hydroxyéthoxyéthyle, 2,3-époxypropyle, 3-diméthylamino, 2-hydroxypropyle, 2-hydroxyéthyle, 2-méthylaminoethoxyméthyle, 2-aminoéthoxyméthyle, 3-méthoxy 2,4-thidiazol-5-yle, 2-tétrahydropyranyle, 2-méthoxyprop-2-yle, 1-hydroxyprop-2-yle, isopropyle, carbamoylméthyle, chlorométhyle, 2-chloroéthyle, acétyl méthyle, 2-méthylthioéthyle ou thiocyanatométhyle.

On peut encore citer entre autres restes de groupements esters que peut représenter A, les groupements 2-chloro 1-acétyloxyéthyle, 2-bromo 1-acétyloxyéthyle, 2-fluoro 1-acétyloxyéthyle, 2-méthoxy 1-acétyloxyéthyle, 2-méthyle 1-acétyloxypropyle, 2-acétyloxyprop-2-yle, 1-méthoxyacétyloxyéthyle, 1-acétylcarbonyloxyéthyle, 1-hydroxyacétyloxyéthyle, 1-formylcarbonyloxyéthyle, 1-(2-thiényl)carbonyloxyéthyle, 1-(2-furyl)carbonyloxyéthyle, 1-(5-nitro 2-furyl)carbonyloxyéthyle, 1-(2-pyrrolyl)carbonyloxyéthyle, 1-(propionyloxycarbonyloxyéthyle, 1-(propyloxycarbonyloxy)éthyle, 1-(isopropyloxycarbonyloxy)éthyle, 1-(méthoxyéthoxycarbonyloxy)éthyle, 1-(allyloxycarbonyloxy)éthyle 1-(2,3-époxy)propyloxycarbonyloxy éthyl, 1-(2-furyl)méthyloxycarbonyloxy éthyle, 1-(2-fluoro)éthyloxycarbonyloxy éthyle, 1-(méthoxycarbonyloxy)propyle, (2-méthoxycarbonyloxy)prop-2-yle, (méthoxycarbonyloxy)chlorométhyle, 1-(méthoxycarbonyloxy)2-chloroéthyle, 1-(méthoxycarbonyloxy)2-méthoxyéthyle, 1-(méthoxycarbonyloxy)1-allyle.

R'a peut représenter l'un des radicaux cités ci-dessous:

a) les radicaux méthyle, éthyle, propyle, isopropyle, butyle, sec-butyle, tert-butyle, pentyle, iso-pentyle, sec-pentyle, tert-pentyle, néo-pentyle, hexyle, iso-hexyle, sec-hexyle, tert-hexyle;
b) les radicaux vinyle, allyle, 1-propényle, butényle, pentényle, hexényle;
c) les radicaux éthynyle, propargyle, butynyle; en particulier méthyle, éthyle, propyle, isopropyle, allyle.

R'a peut également représenter un radical méthoxy méthyle, éthoxyméthyle.

Parmi les substituants éventuels des radicaux benzyle et phényléthyle que peut représenter R'a, on peut citer les radicaux carboxy, amino, aminoalkyle, alkylamino ou dialkylamino, dialkylaminoalkyle tel que diméthylaminoéthyle.

Les produits de formule (I') peuvent également se présenter sous forme de sels d'acides organiques ou de minéraux.

Parmi les acides avec lesquels on peut salifier le ou les groupements amino des produits (I'), on peut citer entre autres, les acides acétique, trifluoroacétique, maléïque, tartrique, méthanesulfonique, benzène sulfonique, p-toluène sulfonique, phosphorique, sulfurique, chlorhydrique, bromhydrique.

Les produits peuvent également se présenter sous forme de sels internes.

L'invention a plus particulièrement pour objet les produits de formule générale (I) telle que définie ci-dessus; dans laquelle R représente un atome d'hydrogène ou un radical alkyle linéaire ou ramifié ayant de 1 à 4 atomes de carbone, éventuellement substitué par un radical carboxylique libre, estérifié ou salifié, ou par un radical amino et Ra représente un radical alkyle ayant au plus 6 atomes de carbone et, parmi ceux-ci, les produits de formule (I) dans laquelle Ra représente un radical méthyle et n représente le nombre 0.

L'invention a également en particulier pour objet les produits de formule générale (I) telle que définie ci-dessus, répondant à la formule générale $I_A$.

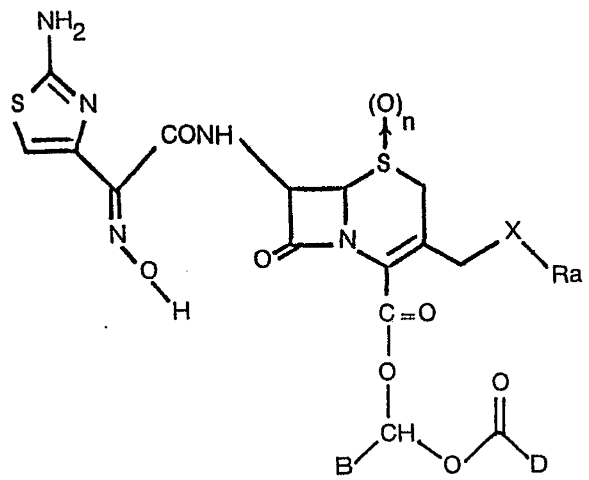

($I_A$)

dans laquelle n, X et Ra sont définis comme précédemment, B représente un atome d'hydrogène ou un radical alkyle linéaire ou ramifié éventuellement substitué, renfermant de 1 à 5 atomes de carbone et D représente un radical alcoyle ou alcoxy linéaire ou ramifié, éventuellement substitué, renferment de 1 à 15 atomes de carbone et notamment de 1 à 5 atomes de carbone, ainsi que leurs sels avec les acides minéraux ou organiques.

Parmi les produits de formule $I_A$, on retient tout particulièrement ceux dans lesquels B représente un atome d'hydrogène ou un radical méthyle ou éthyle et D représente un radical méthyle, éthyle, méthoxy ou éthoxy.

L'invention a plus particulièrement pour objet les produits définis ci-après dans les exemples et spécialement

— l'acide 3-méthoxyméthyl 7-//2-(2-aminothiazol-4-yl)2-hydroxyiminoacétyl/amino/ceph-3-ème 4-carboxylique, ses sels avec les métaux alcalins, alcalino-terreux, le magnésium, l'ammoniaque, les bases organiques aminées, les acides et ses esters facilement clivables,
— l'acide 3-méthylthiométhyl 7-//2-(2-aminothiazol-4-yl) 2-hydroxyiminoacétyl/amino/ceph-3-ème 4-carboxylique, ses sels avec les métaux alcalins, alcalino-terreux, le magnésium, l'ammoniaque, les bases organiques aminées, les acides et ses esters facilement clivables,
— l'acide 3-ethoxyméthyl 7-//2-(2-aminothiazol-4-yl) 2-hydroxyimino acétyl/amino/ceph-3-ème 4-carboxylique, ses sels avec les métaux alcalins, alcalino-terreux, le magnésium, l'ammoniaque, les bases organiques aminées, les acides et ses esters facilement clivables,
— le 3-méthylthiométhyl 7-//2-(2-aminothiazol-4-yl) 2-hydroxyimino acetyl/amino/ceph-3-ème 4-carboxylate de 1-acétyloxyéthyle,
— le 3-méthoxyméthyl 7-//2-(2-aminothiazol-4-yl) 2-hydroxyimino acétyl/amino/ceph-3-ème 4-carboxylate de 1-acétyloxyéthyle,
— le 3-éthoxyméthyl 7-//2-(2-aminothiazol-4-yl) 2-hydroxyimino acétyl/amino/ceph-3-ème 4-carboxylate de l'acétyloxyéthyle.

Il est entendu les produits de formule (I') précités peuvent exister,

— *soit* sous la forme indiquée par ladite formule (I'),
— *soit* sous la forme de produits de formule ($I'_z$):

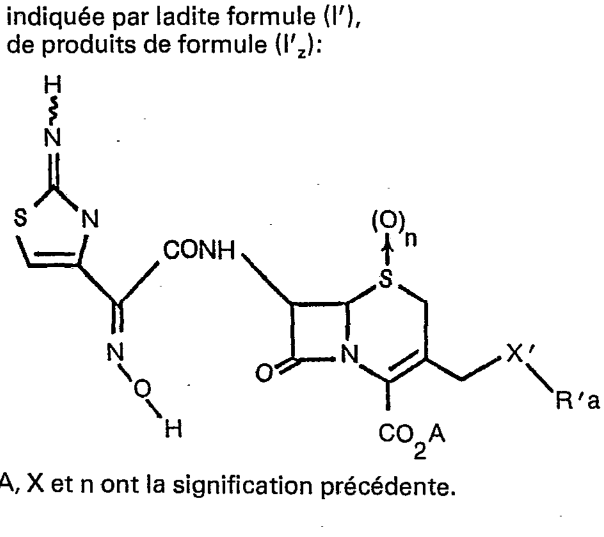

($I'_Z$)

dans laquelle R'a, A, X et n ont la signification précédente.

L'invention concerne également un procédé de préparation des produits de formule (I') telle que définie ci-dessus caractérisé en ce que:

A) soit l'on traite un produit de formule (II)

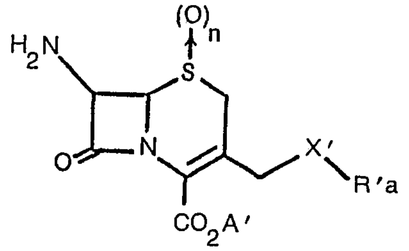

(II)

dans laquelle n, X' et R'a ont la signification précédente et A' représente un atome d'hydrogène ou le reste d'un groupement ester facilement éliminable, par un produit de formule (III)

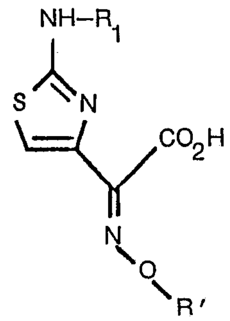

(III)

ou un dérivé fonctionnel de cet acide, formule (III) dans laquelle $R_1$ représente un atome d'hydrogène ou un groupement protecteur du radical amino et soit R' représente un atome d'hydrogène ou un groupement protecteur du radical hydroxyle, pour obtenir un produit de formule (IV):

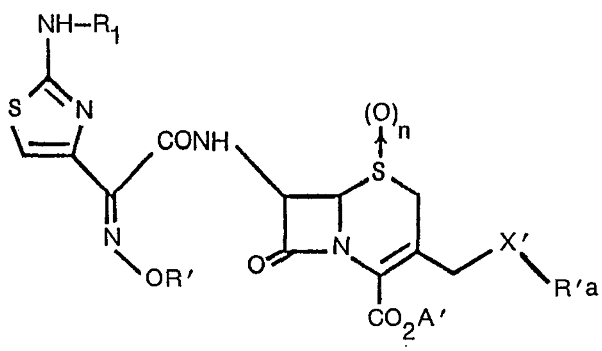

(IV)

dans laquelle $R_1$, R', A', R'a, X' et n ont la signification précédente,

B) soit l'on traite un produit de formule (V):

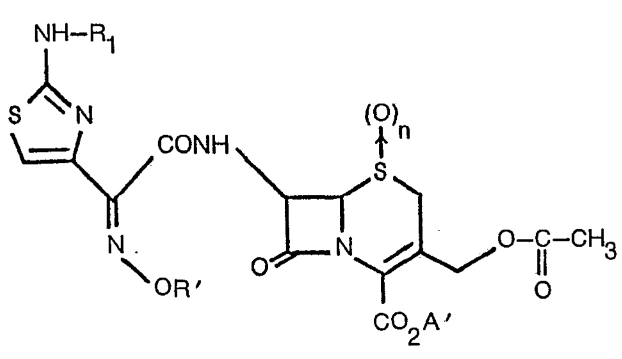

(V)

dans laquelle $R_1$, R', A' et n ont la signification précédente, *ou bien* par un produit de formule R's-SH dans laquelle R's a la signification précédente, *ou bien* d'abord par le 2-mercapto pyridine N-oxyde puis par un produit de formule R'aOH dans laquelle R'a a la signification précédente, pour obtenir un produit de formule (IV) tel que défini précedemment, produit de formule (IV) que, si désiré, dans le cas où n est

égal à 0 et X′ représente un atome de soufre ou d'oxygène, l'on traite par un agent d'oxydation pour obtenir un produit de formule (IV) dans laquelle n est égal à 1 ou 2 et X′ représente un atome de soufre oxydé sous forme de sulfoxyde ou de sulfone, ou un atome d'oxygène et, produit de formule IV que l'on soumet si nécessaire ou si désiré, à l'une ou plusieurs des réactions suivantes, dans un ordre quelconque,

a) coupure par hydrolyse, hydrogénolyse ou par action de la thiourée de tout en partie du groupement ester ou des groupements de protection du radical amino ou du radical hydroxyle,
b) estérification ou salification par une base du radical carboxylique,
c) salification par un acide du radical amino.

En plus des groupements cités ci-dessus, le groupement ester facilement éliminable que peut représenter A′ peut être par exemple l'ester formé avec les radicaux butyle, isobutyle, tert-butyle, pentyle, hexyle, acétoxyméthyle, propionyloxyméthyle, butyryloxyméthyle, valéryloxyméthyle, pivaloyloxyméthyle, 2-acétoxyméthyle, 2-propionyloxyéthyle, 2-butyryloxyéthyle.

On peut également citer les radicaux 2-iodoéthyle, $\beta\beta\beta$-trichloroéthyle, vinyle, allyle, éthynyle, propynyle, benzyle, 4-méthoxybenzyle, 4-nitrobenzyle, phényléthyle, trityle, diphénylméthyle, 3,4-diméthoxyphényle.

On peut également citer les radicaux phényle, 4-chlorophényle, tolyle, tert-butylphényle.

Le groupement protecteur du radical amino que peut représenter $R_1$ peut être par exemple un radical alkyle de 1 à 6 atomes de carbone tel que, préférentiellement, tert-butyle ou tert-amyle. $R_1$ peut également représenter un groupement acyle, aliphatique, aromatique ou hétérocyclique ou un groupe carbamoyle.

On peut citer les groupements alcanoyles inférieurs tel que par exemple formyle, acétyle, propionyle, butyryle, isobutyryle, valéryle, isovaléryle, oxalyle, succinyle, pivaloyle. $R_1$ peut également représenter un groupe alkoxy, ou cycloalkoxycarbonyle inférieur tel que par exemple, méthoxycarbonyle, éthoxycarbonyle, propoxycarbonyle, 1-cyclopropyléthoxycarbonyle, isopropyloxycarbonyle, butyloxycarbonyle, tertbutyloxycarbonyle, pentyloxycarbonyle, hexyloxycarbonyle, un groupe benzoyle, toluolyle, naphtolyle, phtaloyle, mésyle, phénylacétyle, phénylpropionyle, un groupe aralcoxycarbonyle, tel que benzyloxycarbonyle.

Les groupements acyle peuvent être substitués par exemple par un atome de chlore, de brome, d'iode ou de fluor. On peut citer les radicaux chloroacétyle, dichloroacétyle, trichloroacétyle, bromoacétyle ou trifluoroacétyle.

$R_1$ peut également représenter un groupement aralkyle inférieur tel que benzyle, 4-méthoxybenzyle ou phényléthyle, trityle, 3,4-di-méthoxybenzyle ou benzhydryle.

$R_1$ peut également représenter un groupe haloalkyle tel que trichloroéthyle.

$R_1$ peut également représenter un groupement chlorobenzoyle, para-nitrobenzoyle, para-tert-butylbenzoyle, phénoxyacétyle, caprylyle, n-décanoyle, acryloyle, trichloroéthoxycarbonyle.

$R_1$ peut également représenter un groupement méthylcarbamoyle, phénylcarbamoyle, naphtylcarbamoyle, ainsi que les thiocarbamoyles correspondants.

La liste ci-dessus n'est pas limitative, il est évident que d'autres groupements protecteurs des amines, groupements connus en particulier dans la chimie des peptides, peuvent également être utilisés.

Le groupement de protection du radical hydroxyle que peut représenter R′, peut être choisi dans la liste ci-dessous: R′ peut représenter un groupe acyle tel que par exemple formyle, acétyle, chloroacétyle, bromoacétyle, dichloroacétyle, trichloroacétyle, trifluoroacétyle, méthoxyacétyle, phénoxyacétyle, benzoyle, benzoylformyle, p-nitrobenzoyle. On peut citer également les groupements éthoxycarbonyle, méthoxycarbonyle, propoxycarbonyle, $\beta\beta\beta$-trichloroéthoxycarbonyle, benzyloxycarbonyle, tert-butoxycarbonyle, 1-cyclo propyléthoxycarbonyle, tétrahydropyrannyle, tétrahydrothiopyrannyle, méthoxytétrahydropyrannyle, trityle, benzyle, 4-méthoxybenzyle, benzhydryle, trichloroéthyle, 1-méthyle 1-méthoxyéthyle, phtaloyle.

On peut également citer d'autres acyles tels que propionyle, butyryle, isobutyryle, valéryle, isovaléryle, oxalyle, succinyle et pivaloyle.

On peut également citer les radicaux phénylacétyle, phénylpropionyle, mésyle, chlorobenzoyle, para-nitrobenzoyle, para-tert-butylbenzoyle, caprylyle, acryloyle, méthylcarbamoyle, phénylcarbamoyle, naphtylcarbamoyle.

Dans un mode préférentiel d'éxécution du procédé, on traite le produit de formule (II) par un dérivé fonctionnel d'un produit de formule (III). Ce dérivé fonctionnel peut être par exemple un halogénure, un anhydride symétrique ou mixte, l'amide, l'azide, ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formé par exemple avec le chlorure de para-toluène sulfonyle.

Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

L'anhydride peut être formé in situ par action de carbodiimide NN'di-substitué, par exemple la N,N-dicyclohexylcarbodiimide.

La réaction d'acylation est conduite de préférence dans un solvant organique tel que le chlorure de méthylène. On peut cependant utiliser d'autres solvants tels que le tétrahydrofuranne, le chloroforme ou le diméthylformamide.

Lorsqu'on utilise un halogènure d'acide et de manière générale lorsqu'une molécule d'acide halohydrique est libérée au cours de la réaction, on réalise la réaction de préférence un présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en général inférieure ou égale à la température ambiante.

Lorsque $R_1$ représente un atome d'hydrogène on utilise de préférence un anhydride mixte carboxylique sulfonique.

L'action du produit de formule R'aSH sur le produit de formule (V) est réalisée de préférence dans les conditions décrites dans le brevet français 2 379 540. On opère alors en présence d'éthérate de trifluorure de bore dans l'acide acétique ou le nitrométhane.

L'action, sur le produit de formule (V) d'abord du 2-mercapto pyridine N-oxyde puis de l'alcool R'aOH est effectuée de préférence dans les conditions indiquées dans le brevet français 2 119 074.

La formation de l'éther est effectuée de préférence en présence de sels cuivriques tels que le chlorure cuivrique.

L'oxydation du produit de formule (IV) peut être effectuée par un peracide, par exemple l'acide peracétique, perphtalique, m-chloroperbenzoïque, ou perbenzoïque, ou par l'eau oxygénée.

Selon les valeurs de $R_1$, R' et A', les produits de formule (IV) peuvent ou non constituer des produits de formule (I).

Les produits de formule (IV) constituent des produits de formule (I) lorsque $R_1$ et R' représentent chacun un atome d'hydrogène, et lorsque A' ne représente pas, parmi les groupements esters facilement clivables, l'un de ceux que l'on désirerait éliminer.

Dans les autres cas, l'action sur le produit de formule (IV) d'un ou plusieurs agents d'hydrolyse, d'hydrogénolyse ou de la thiourée a pour but d'éliminer le radical $R_1$ lorsque celui-ci représente un radical protecteur du radical amino, d'éliminer le radical R' lorsque celui-ci est différent de R et/ou d'éliminer le radical A' lorsque celui-ci représente, parmi les groupements ester facilement clivables l'un de ceux que l'on désire éliminer.

Cependant il est bien entendu possible d'éliminer $R_1$ sans toucher aux substituants R' et A' lorsque ceux-ci doivent être conservés. Il en est ainsi par exemple lorsque A' représente un groupement ester que l'on souhaite conserver tel qu'un groupement propionyloxyméthyle.

La nature des réactifs à mettre en jeu dans un tel cas est bien connu de l'homme de métier. Des exemples de telles réactions sont donnés plus loin dans la partie expérimentale.

On donne ci-après une énumération non exhaustive des moyens pouvant être mis en oeuvre pour éliminer les différents groupements.

L'élimination du groupe R1 peut être effectuée par hydrolyse, celle-ci étant acide, basique ou utilisant l'hydrazine.

On utilise préférentiellement l'hydrolyse acide pour éliminer les groupements alkoxy et cycloalkoxycarbonyles éventuellement substitués, tels que tert-pentyloxycarbonyle ou tert-butyloxycarbonyle, les groupements aralcoxycarbonyles éventuellement substitués tels que benzyloxycarbonyle, les groupements trityle, benzhydryle, tert-butyle ou 4-méthoxybenzyle.

L'acide que l'on utilise de préférence peut être choisi dans le groupe constitué par les acides chlorhydrique, benzène sulfonique ou para-toluène sulfonique, formique ou trifluoroacétique. On peut cependant utiliser d'autres acides minéraux ou organiques.

L'hydrolyse basique est utilisée préférentiellement pour éliminer les groupements acyle tels que trifluoroacétyle.

La base que l'on utilise de préférence est une base minérale telle que l'hydroxyde de sodium ou de potassium. On peut également utiliser la magnésie, la baryte ou un carbonate ou carbonate acide de métal alcalin tel que les carbonates et carbonates acides de sodium ou de potassium ou d'autres bases.

On peut également utiliser l'acétate de sodium ou de potassium.

L'hydrolyse utilisant l'hydrazine est utilisée de préférence pour éliminer des groupes tels que phtaloyle.

Le groupement $R_1$ peut également être éliminé par le système zinc-acide acétique (pour le groupement trichloroéthyle), les groupements benzhydryle, benzyloxycarbonyle sont éliminés de préférence par l'hydrogène en présence d'un catalyseur.

Le groupement chloroacétyle est éliminé par action de la thio-urée en milieu neutre ou acide selon le type de réaction décrit par MASAKI J.A.C.S., *90*, 4508, (1968).

On peut également utiliser d'autres méthodes de déprotection connues dans la littérature.

Parmi les groupes préférés, on peut citer les groupements formyle, acétyle, éthoxycarbonyle, mésyle, trifluoroacétyle, chloroacétyle, trityle.

L'acide que l'on utilise de préférence est l'acide trifluoroacétique.

L'élimination du radical A' ou R', lorsque celle-ci est nécessaire, est réalisée dans des conditions semblables à celles décrites précédemment pour l'élimination de $R_1$.

On peut utiliser, entre autres, l'hydrolyse acide pour éliminer les radicaux alkyle ou aralkyle éventuellement substitués.

On utilise préférentiellement un acide choisi dans le groupe formé par les acides chlorhydrique, formique, trifluoroacétique et para-toluène sulfonique.

Les autres valeurs des radicaux A' ou R' sont, lorsque cela est désiré, éliminées selon les procédés connus de l'homme de métier. On opère de préférence dans des conditions modérées, c'est-à-dire, à température ambiante ou en chauffant légèrement.

Naturellement, on peut, lorsque par exemple $R_1$ et A' ou R' sont das groupements éliminables appartenant à des types différents, faire agir sur les produits (IV) plusieurs agents envisagés dans les énumérations précédentes.

La salification des produits peut être effectuée selon les méthodes usuelles.

La salification peut, par example, être obtenue par action sur un produit sous forme acide ou sur un solvat, par exemple le solvat éthanolique ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate tri-sodique. On peut également faire appel à des sels d'acides organiques.

Comme sels d'acides organiques, on peut mentionner, par exemple, les sels de sodium d'acides carboxyliques aliphatiques linéaires ou ramifiés, saturés ou insaturés de 1 à 18 et de préférence de 2 à 10 atomes de carbone. Les chaines aliphatiques de ces acides peuvent être interrompues par un ou plusieurs hétéroatomes tels que l'oxygène ou le soufre ou substituées par des radicaux aryle, comme par exemple: phényle, thiényle, furyle, par un ou plusieurs radicaux hydroxyle ou par un ou plusieurs atomes d'halogène tels que fluor, chlore ou brome, préférentiellement chlore, par un ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme par exemple des acides benzoïques substitués, de préférence par des radicaux alkyles inférieurs.

Comme exemples de tels acides organiques on peut mentionner: les acides formique, acétique, acrylique, butyrique, adipique, isobutyrique, n-caproïque, isocaproïque, chloropropioniques, crotonique, phénylacétique, 2-thiénylacétique, 3-thiénylacétique, 4-éthylphénylacétique, glutarique, l'ester monoéthylique de l'acide adipique, les acides hexanoïque, heptanoïque, décanoïque, oléïque, stéarique, palmitique, 3-hydroxypropionique, 3-méthoxypropionique, 3-méthylthiobutyrique, 4-chlorobutyrique, 4-phénylbutyrique, 3-phénoxybutyrique, 4-éthylbenzoïque, 1-propylbenzoïque.

On utilise cependant de préférence comme sels de sodium l'acétate de sodium, le 2-éthyl héxanoate de sodium ou le diéthyl acétate de sodium.

La salification peut également être obtenue par action d'une base organique comme la triéthylamine, la diéthylamine, la triméthylamine, la propylamine, la N,N-diméthyléthanolamine, le tris (hydroxyméthyl) amino méthane, la méthylamine, l'éthanolamine, la pyridine, la picoline, la dicyclohexylamine, la morpholine et la benzylamine.

Elle peut également être obtenue par action de l'arginine, de la lysine, de la procaïne, de l'histidine, de la N-méthyle glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opére en général en faisant réagir l'acide de formule (I') ou un dérive fonctionnel, avec un dérivé de formule:

Z-Re

dans laquelle Z représente un radical hydroxyle ou un atome d'halogène tel que le chlore, le brome, l'iode et Re désignele groupement est à introduire, groupement dont une liste non exhaustive figure ci-dessus. Dans certains cas il peut être avantageux d'effectuer une estérification sur un produit dont l'amine et/ou un éventuel groupement oxyimino sont bloqués avant d'enlever le groupement protecteur de l'amine et du groupement oxyimino.

La présente invention concerne, plus spécialement, un procédé, tel que décrit ci-dessus, pour la préparation des produits de formule (I') telle que décrite précédemment, caractérisé en ce que l'on utilise, pour la mise en oeuvre du procédé, un produit de formule (III) dans laquelle $R_1$ représente un

groupement protecteur du radical amino et en ce que le dérivé fonctionnel de l'acide de formule (III) est un anhydride mixte carboxylique sulfonique.

L'anhydride carboxylique sulfonique utilisé est de préférence formé avec l'acide para-toluène sulfonique.

Le groupement protecteur que représente $R_1$ est de préférence le groupement trityle.

La présente demande a également pour objet un procédé de préparation du produit de formule (I'), telle que définie ci-dessus, caractérisé en ce que l'on traite un produit de formule (II')

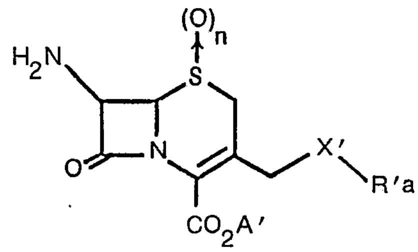

(II')

dans laquelle R'a, n, X et A' ont la signification précédente par un produit de formule (III')

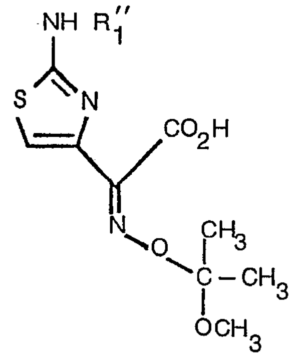

(III')

ou un dérivé fonctionnel de cet acide, formule (III') dans laquelle $R''_1$ représente un groupement protecteur du radical amino, pour obtenir un produit de formule (IV')

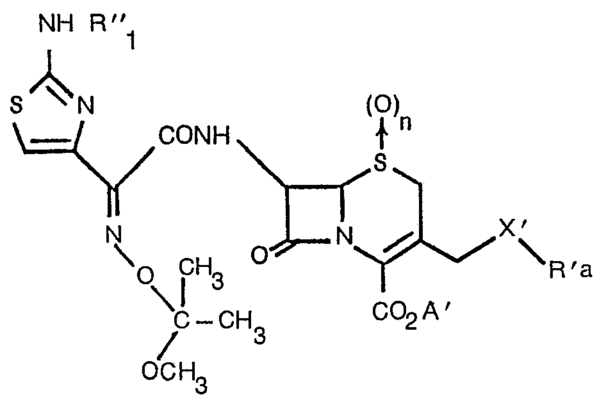

(IV')

dans laquelle A', $R''_1$, R'a, X' et n ont la signification précédente, produit de formule (IV') que l'on traite par un acide dans des conditions modérées, pour obtenir un produit de formule (VI)

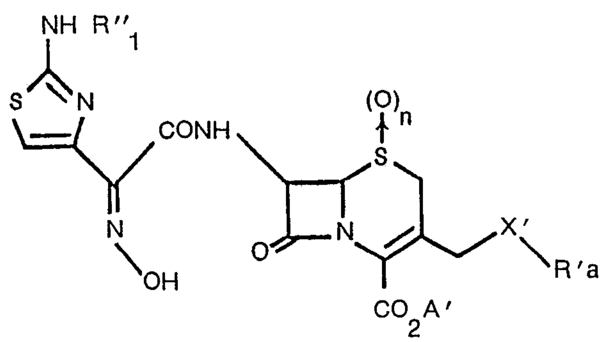

(VI)

produit que, si désiré, l'on estérifie ou salifie et produit de formule VI que l'on soumet à une hydrolyse, hydrogénolyse ou à l'action de la thiourée pour éliminer le radical $R''_1$, de protection du radical amino et, si désiré, ou si nécessaire, à l'une ou plusieurs des réactions suivantes, dans un ordre quelconque:

a) élimination du groupement ester,
b) estérification ou salification par une base, du radical carboxylique,
c) salification par un acide du radical amino.

Le groupement protecteur du radical amino que peut représenter $R''_1$ peut être, par exemple, un de ceux précédemment cités pour $R_1$.

Le dérivé fonctionnel du produit de formule III' peut être l'un de ceux précédemment cités pour le produit de formule III.

L'acide que l'on utilise pour obtenir le produit de formule VI à partir du produit de formule IV' est, de préférence, l'acide chlorhydrique aqueux.

Les traitements du produit de formule VI s'effectuent dans les conditions précédemment décrites pour les produits de formule IV.

Les produits de formule générale (I') possèdent une très bonne activité antibiotique sur les bactéries gram (+) telles que les staphylocoques, les streptocoques et, notamment, sur les staphylocoques pénicillino-résistants. Leur efficacité sur les bactéries gram (—), notamment, sur les bactéries coliformes, les klebsiella, les salmonella et les proteus est particulièrement remarquable.

Ces propriétés rendent aptes lesdits produits, ainsi que leurs sels d'acides, pharmaceutiquement acceptables à être utilisés comme médicaments dans le traitement des affections à germes sensibles et, notamment, dans celui des staphylococcies, telles que septicémies à staphylocoques, staphylococcies malignes de la face ou cutanée, pyodermites, plaies septiques ou suppurantes, anthrax, phlegmons, érésipèles, staphylococcies aigües primitives ou post grippales, bronchopneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (—).

La présente invention a donc également pour objet, à titre de médicaments et notamment de médicaments antibiotiques, les produits de formule (I), tels que définis ci-dessus, ainsi que leurs sels d'acides, pharmaceutiquement acceptables.

L'invention a particulièrement pour objet, à titre de médicaments et notamment de médicaments antibiotiques, les produits de formule (I) dans laquelle Ra représente un radical alkyle ayant au plus 6 atomes de carbone, et notamment parmi ceux-ci, ceux dans laquelle Ra représente un radical méthyle et n représente le nombre 0, y compris leurs sels d'acides, pharmaceutiquement acceptables.

L'invention a également plus particulièrement pour objet, à titre de médicaments et notamment de médicaments antibiotiques, les produits de formule (I), répondant à la formule ($I_A$)

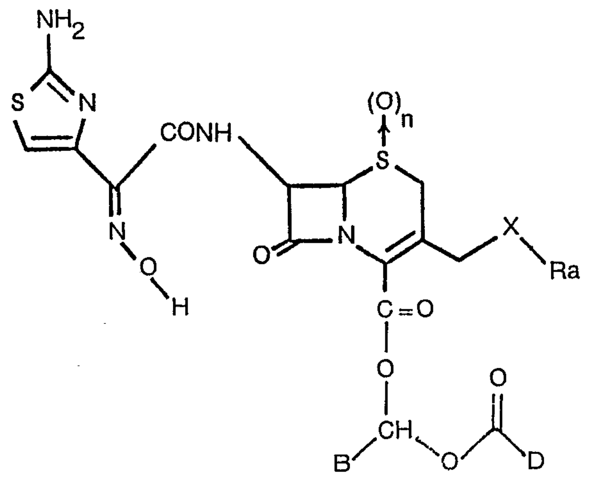

($I_A$)

dans laquelle n, X et Ra sont définis comme précédemment, B représente un atome d'hydrogène ou un radical alkyle linéaire ou ramifié, éventuellement substitué, renfermant de 1 à 5 atomes de carbone et D représente un radical alkyle ou alkyloxy linéaire ou ramifié, éventuellement substitué, renfermant de 1 à 15 atomes de carbone et notamment de 1 à 5 atomes de carbone, et, tout particulièrement parmi les produits de formule $I_A$, ceux dans lesquels B représente un atome d'hydrogène ou un radical méthyle ou éthyle et D représente un radical méthyle, éthyle, méthoxy ou éthoxy.

L'invention a également plus particulièrement pour objet, à titre de médicaments et notamment de médicaments antibiotiques, les produits décrits dans les exemples et parmi ceux-ci,

— l'acide 3-méthoxyméthyl 7-//2-(2-amino thiazol-4-yl) 2-hydroxyimino acétyl/amino/ceph-3-ème 4-carboxylique, ses sels pharmaceutiquement acceptables avec les métaux alcalins, alcalino-terreux, le magnésium, l'ammoniaque, les bases organiques aminées, les acides et ses esters facilement clivables,
— l'acide 3-méthylthiométhyl 7-//2-(2-aminothiazol-4-yl) 2-hydroxyimino acétyl/amino/ceph-3-ème 4-carboxylique, ses sels pharmaceutiquement acceptables, avec les métaux alcalins, alcalino-terreux, le magnésium, l'ammoniaque, les bases organiques aminées, les acides et ses esters facilement clivables,
— l'acide 3-éthoxyméthyl 7-//2-(2-aminothiazol-4-yl) 2-hydroxyimino acétyl/amino/ceph-3-ème 4-carboxylique, ses sels pharmaceutiquement acceptables, avec les métaux alcalins, alcalinoterreux, le magnésium, l'ammoniaque, les bases organiques aminées, les acides et ses esters facilement clivables,
— le 3-méthylthiométhyl 7-//2-(2-aminothiazol-4-yl) 2-hydroxyimino acétyl/amino/ceph-3-ème 4-carboxylate de 1-acétyloxyéthyle;
— le 3-méthoxyméthyl 7-//2-(2-aminothiazol-4-yl) 2-hydroxyimino acétyl/amino/ceph-3-ème 4-carboxylate de 1-acétyloxyéthyle;
— le 3-éthoxyéthyl 7-//2-(2-aminothiazol-4-yl) 2-hydroxyimino acétyl/amino/ceph-3-ème 4-carboxylate de 1-acétyloxyéthyle.

L'invention s'étend aux compositions pharmaceutiques renfermant comme principe actif au moins un des médicaments définis ci-dessus.

Ces compositions peuvent être administrées par voie buccale, rectale, parentérale, notamment intramusculaire, ou par voie locale en application topique sur la peu et les muqueuses.

Les produits de formule (I') dans laquelle A représente un ester clivable, et notamment parmi ces produits, ceux répondant à la formule ($I_A$), et, tout particulièrement, les esters de 1-méthoxycarbonyloxyéthyle ou de 1-acétyloxyéthyle peuvent être administrés par voie orale.

Les compositions selon l'invention peuvent être solides ou liquides et se présenter sous les formes pharmaceutiques couramment utilisées en médecine humaine comme par exemple, les comprimés simples ou dragéïfiés, les gélules, les granulés, les suppositoires, les préparations injectables les pommades, les crèmes, les gels; elles sont préparées selon les méthodes usuelles. Le ou les principes actifs peuvent y être incorporées à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent, notamment, se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec le produit décrit à l'exemple 5, ou encore comprise entre 0,500 g et 1 g trois fois par jour, par voie intramusculaire.

Les produits de formule (I') peuvent également être utilisés comme désinfectants des instruments chirurgicaux.

En plus des produits décrits dans les exemples qui illustrent l'invention les produits suivants constituent des produits pouvant être obtenus dans le cadre de la présente invention; les substituants n, X', R'a et A sont ceux indiqués dans la formule (I')

| n | X' | R'a | A |
|---|---|---|---|
| 0 | 0 | $-CH_2-CH_3$ | $-CH_2OC-C_2H_5$<br>‖<br>O |
| 0 | 0 | $-CH_2-CH_3$ | $-CH_2OC-C(CH_3)_3$<br>‖<br>O |
| 0 | 0 | $-CH_2-CH_3$ | $-CH-OC-CH_3$<br>\| ‖<br>$CH_3$ O |
| 0 | S | $-CH_2-CH_3$ | $-CH_2OCC_2H_5$<br>‖<br>O |
| 0 | S | $-CH_2-CH_3$ | $-CH_2OCC(CH_3)_3$<br>‖<br>O |
| 0 | S | $-CH_2-CH_3$ | $-CH-OC-CH_3$<br>\| ‖<br>$CH_3$ O |
| 0 | 0 | $-CH(CH_3)_2$ | H |
| 1 | 0 | $-CH_3$ | H |
| 0 | 0 | $-(CH_2)_2CH_3$ | H |
| 0 | 0 | $-CH_2-CH=CH_2$ | H |
| 0 | 0 | $-CH_2$ 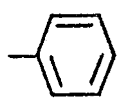 | H |
| 0 | S | $-CH(CH_3)_2$ | H |
| 0 | S | $-CH_3$ | H |
| 0 | S | $-(CH_2)_2CH_3$ | H |
| 0 | S | $-CH_2-CH=CH_2$ | H |
| 0 | S | $-CH_2$ 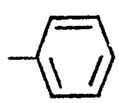 | H |

Les produits de formule (II) sont connus ou peuvent être préparés selon les procédés indiqués dans les brevets français 2 379 540 et 2 119 074. Les produits de formules (III) et (V) sont décrits par exemple dans les brevets français 2 346 014 et 2 385 722.

Les exemples suivants illustrent l'invention sans toutefois la limiter.

Exemple 1

Acide 3-méthoxyméthyl 7-/2-(2-aminothiazol-4-yl) 2-hydroxyimino acétamido/ceph-3-ème 4-carboxylique isomère syn.

Stade A

Anhydride para-toluène sulfonique 2-(2-tritylamino thiazol-4-yl)2-/(1-méthyl-1-méthoxy)-éthoxy-imino/acétique isomère syn.

On met en suspension 3,01 g de sel de triéthylamine de l'acide 2-(2-tritylaminothiazol-4-yl) 2-(1-méthyl 1-méthoxy éthoxy imino) acétique isomère syn dans 15 $cm^3$ d'acétone. On ajoute 1,05 g de chlorure de tosyle et agite pendant une heure trente minutes. On introduit 20 $cm^3$ d'éther éthylique dans le mélange, refroidit à $-10°C$, essore, lave à l'éther et obtient 2,90 g de produit composé de l'anhydride recherché et de chlorhydrate de triéthylamine.

Stade B

Acide 3-méthoxy méthyl 7-/2-(2-tritylamino thiazol-4-yl) 2-/(1-méthyl 1-méthoxy) éthoxy imino/acétamido/ceph-3-ème 4-carboxylique isomère syn.

On dissout 0,732 g d'acide 7-amino 3-méthoxy méthyl cèph-3-ème 4-carboxylique dans 10 cm3 de chlorure de méthylène et 0,84 cm3 de triéthylamine, refroidit à —20°C et ajoute 2,4 g du mélange obtenu au Stade A laisse revenir à température ambiante, ajoute 0,5 cm3 d'acide acétique, lave à l'eau, sèche, concentre à sec, triture avec de l'éther éthylique, essore et obtient 3,07 g de produit brut que l'on recristallise dans le méthanol pour obtenir 1,21 g de produit attendu

RMN ($CDCL_3$) ppm
1,52: $C\underline{H}_3$ géminés
3,22—3,25: —O—$C\underline{H}_3$
3,45: —$C\underline{H}_2$S
4,25: —$C\underline{H}_2$OMe
4,99 (d; J=5): H6
5,73 (dd; J=5 J=8): H7
6,70: $H_5$ du thiazole (syn)
7,28: —$C\emptyset_3$

Stade C

Acide 3-méthoxyméthyl 7-/2-(2-aminothiazol-4-yl) 2-hydroxyimino acétamido/ceph-3-ème-4-carboxylique isomère syn.

On agite pendant 10 minutes 1,1 g de produit obtenu ci-dessus avec 5 cm3 d'acide formique aqueux, à 45—50°C, ajoute 2 cm3 d'eau, essore, concentre à sec le filtrat sous pression réduite à 30°C, en entrainant l'eau à l'éthanol. Le résidu cristallise dans l'eau. On essore et obtient 0,54 g de produit brut que l'on dissout, à l'aide de triéthylamine, dans 5 cm3 d'éthanol aqueux à 50%. On acidifie à pH 2—3 avec de l'acide formique, essore le produit cristallisé, lave à l'éthanol puis à l'éther et obtient 0,44 g de produit solvaté.

Analyse: $C_{14}H_{15}O_6N_5S_2$ 1/2 $H_2O$ PM: 422,43

| | C% | H% | N% | S% |
|---|---|---|---|---|
| Calculé: | 39,9 | 3,82 | 16,58 | 15,18 |
| Trouvé: | 40,0 | 4,0 | 16,1 | 14,8 |

Spectre IR (Nujol)
C=O $\beta$ lactame 1757 $^{cm^{-1}}$
C=C, C=N: 1637—1638 $^{cm^{-1}}$
aromatiques 1605—1572—1488 $^{cm^{-1}}$

Spectre UV (ETOH, HCl N/10)
infl: 220 nm $E_{1\,cm}^{1\%}=288$
$\lambda$max: 262 nm $E_{1\,cm}^{1\%}=421$ $\varepsilon$: 17400

Spectre RMN (DMSO) ppm
3,20: $OC\underline{H}_3$
3,50: S—$C\underline{H}_2$
4,17: $C\underline{H}_2O$
5,14 (d; J=5): H6
5,77 (dd; J=5; J=8): H7
6,65: $H_5$ du thiazole
7,1: $NH_2$
9,43 (d; J=8) N$\underline{H}$CO

Exemple 2

3-méthoxyméthyl 7-/2-(2-tritylamino thiazol-4-yl) 2-/(1-méthyl 1-méthoxy)éthoxy imino/-acétamido/ceph-3-ème 4-carboxylate de 1-oxopropoxy méthyl isomère syn.

Stade A

3-méthoxyméthyl 7-/2-(2-tritylamino thiazol-4-yl) 2-/(1-méthyl 1-méthoxy)éthoxy imino/acétamido/ceph-3-ème 4-carboxylate de 1-oxopropoxy méthyle isomère syn.

On dissout à température ambiante 4,15 g d'acide 3-méthoxy méthyl 7-/2-(2-tritylamino thiazol-4-yl) 2-/(1-méthoxy 1-méthyl) éthoxy imino/acétamido ceph-3-ème 4-carboxylique, isomère syn et

0,456 g de carbonate de potassium sec dans 14 cm3 de diméthylformamide anhydre. On refroidit à 0°C, introduit, en 10 minutes, une suspension de propionate d'iodométhyle préparé comme ci-après et agite 30 minutes à 0°C puis 30 minutes à 20°C. On verse le milieu réactionnel dans un mélange constitué par 340 cm3 d'eau, 17 cm3 de solution aqueuse normale de bicarbonate de sodium et 50 cm3 d'acétate d'éthyle. On agite, décante, extrait à l'acétate d'éthyle, lave à l'eau, sèche, concentre à sec sous pression réduite à moins de 35°C. On reprend le résidu par 25 cm3 d'éther isopropylique et essore 4,42 g de produit attendu.

RMN ($CDCl_3$) ppm
1,15 (t, j=7); 2,40 (q, J=7) $C_2H_5$
3,34: $OC\underline{H}_3$
3,55: $SC\underline{H}_2$
4,33: $C\underline{H}_2OCH_3$
5,05: (d J=5): H6
6,71: $H_5$ du thiazole syn
7,33: trityle

Préparation du propionate d'iodométhyle
On porte au reflux pendant 10 minutes 1,4 g de propionate de chlorométhyle, 1,71 g d'iodure de sodium et 23 cm3 d'acétone anhydre. On obtient une suspension que l'on utilise immédiatement.

Stade B
3-méthoxyméthyl 7-/2-(2-aminothiazol-4-yl) 2-hydroxyimino acétamido/ceph-3-ème-4-carboxylate de 1-oxopropoxy méthyle isomère syn
On agite, à 45—50°C, pendant 15 minutes, 4.37 g du produit obtenu précédemment dans 22 cm3 d'acide formique aqueux à 65%. On dilue avec 90 cm3 d'eau à chaud, essore et distille le filtrat sous pression réduite, à moins de 30°C. On reprend le résidu par 100 cm3 de chlorure de méthylène, lave avec une solution saturée de chlorure de sodium diluée au 1/10 ème, et 7 cm3 d'une solution normale de bicarbonate de sodium puis avec une solution saturée de chlorure de sodium diluée au 1/10 ème. On sèche la phase organique, distille à sec sous pression réduite et reprend le résidu par 100 cm3 d'éther éthylique, essore et obtient 2,10 g de produit brut. On le reprend dans 15 cm3 d'acétate d'éthyle, agite 30 minutes, essore, rince à l'acétate d'éthyle puis à l'éther éthylique et obtient 1,69 g de produit. On en dissout 1,57 g dans 15 cm3 de chlorure de méthylène, filtre, distille à sec sous pression réduite, reprend le résidu par 10 cm3 d'acétate d'éthyle, agite 30 minutes, essore, rince à l'acétate d'éthyle puis à l'éther et obtient 1,29 g de produit attendu.

$/\alpha/_D$=+52°±1 C=1,5% DMSO
RMN ($CDCl_3$) ppm
1,13 (t; J=7); 2,41 (q; J=7): $C_2H_5$
3,30: $OC\underline{H}_3$
3,53: $SC\underline{H}_2$
4,3: $C\underline{H}_2$—$OCH_3$
5,02 (d; J=5): H6
6,92: $H_5$ du thiazole (syn)

Exemple 3
Acide 3-méthylthiométhyl 7-/2-(2-aminothiazol-4-yl) 2-hydroxyimino acétamido/ceph-3-ème 4-carboxylique isomère syn
Stade A
Acide 3-méthylthiométhyl 7-/2-(2-tritylaminothiazol-4-yl) 2-/(1-méthyl 1-méthoxy)éthoxy imino/-acétamido/ceph-3-ème 4-carboxylique isomere syn
On mélange 2,60 g d'acide 3-méthylthiométhyl 7-amino ceph-3-ème 4-carboxylique, 37,5 cm3 de chlorure de méthylène et 2,8 cm3 de triéthylamine, refroidit à —20°C et introduit 9,5 g d'anhydride para-toluène sulfonique 2-(2-tritylamino thiazol-4-yl) 2-/(1-méthyl 1-méthoxy) éthoxy imino/acétique préparé comme au stade A de l'exemple I. On agite à 0°C pendant deux heures, acidifie par 1,75 cm3 d'acide acétique, lave à l'eau la phase organique, sèche et concentre à sec sous pression réduite. On reprend le résidue par 50 cm3 d'éther éthylique, essore, rince à l'éther et obtient 8,45 g de produit brut. On le reprend par 45 cm3 de méthanol et agite pendant 30 minutes. On amorce la cristallisation, essore, rince au méthanol puis à l'éther éthylique et obtient 5,2 g du produit attendu.

RMN ($CDCl_3$) ppm
1,85=$CH_3S$
5,05 (d; J=5): H6
5,70 (d,d; J=5, J=8): H7
6,71 $H_5$ du thiazole (syn)
7,28=trityle

Stade B

Acide 3-méthylthiométhyl 7-/2-(2-aminothiazol-4-yl) 2-(hydroxyimino) acétamido/ceph-3-ème 4-carboxylique isomère syn

On agite à 50°C pendant 10 minutes 3,72 g du produit obtenu précédemment et 18,6 cm3 d'acide formique aqueux à 66%. On ajoute 7,4 cm3 d'eau et essore aussitôt. On distille le filtrat sous pression réduite à 30°C au maximum, effectue 2 entrainements avec un mélange éthanol eau (2/1), reprend le résidu par 10 cm3 d'eau, essore, rince à l'eau, puis à l'éther éthylique et obtient 1,945 g de produit brut. On le reprend par 136 cm3 d'éthanol aqueux à 50% et ajoute lentement 0,63 cm3 de triéthylamine. On essore l'insoluble, acidifie de filtrat à pH≃3—4 par 0,45 cm3 d'acide formique aqueux à 50%. On essore, rince à l'éthanol aqueux à 50%, à l'éthanol anhydre puis à l'éther éthlyique et obtient 1,392 g de produut purifié.

Spectre IR (Nujol)

| | |
|---|---|
| C=0 $\beta$ lactame | $1772^{cm^{-1}}$ |
| amide | $1693^{cm^{-1}}$ |
| $NH_2$ (de formation) | $1619^{cm^{-1}}$ |
| aromatique | $1595^{cm^{-1}}$ |
| | $1535^{cm^{-1}}$ |

Spectre UV (EtOH —HCl N/10)

infl 220 nm $E_1^1$=320

$\lambda$max 262 nm $E_1^1$=444 $\varepsilon$=19100

Spectre RMN (DMSO) ppm

1,98: $C\underline{H}_3S$

3,58: $C\underline{H}_2S$

5,17: (d; J=5) H6

5,72 (d,d; J=5, J=8): H7

6,66: $H_5$ du thiazole (syn)

9,43 (d; J=8): NHCO

Exemple 4

3-méthylthiométhyl 7-/2-(2-aminothiazol-4-yl) 2-hydroxyimino acétamido) ceph-3-ème 4-carboxylate de 1-oxopropoxyméthyle isomère syn

Stade A

3-méthylthiométhyl 7-/2-(2-tritylamino thiazol-4-yl) 2-/(1-méthyl 1-méthoxy) éthoxy imino/-acétamido/ceph-3-ème 4-carboxylate de 1-oxopropoxy méthyle isomère syn

On agite à 20°C 5,2 g d'acide 3-méthylthiométhyl 7-/2-(2-tritylamino thiazol-4-yl) 2-/(1-méthyl 1-méthoxy) éthoxyimino/acétamido/ceph-3-ème 4-carboxylique isomère syn et 0,56 g de carbonate de potassium dans 20 cm3 de diméthyl formamide anhydre, refroidit à +5°C et ajoute à +5+10°C 28 cm3 d'une suspension acétonique de propionate d'iodométhyle préparé extemporanément à partir de 1,715 g de propionate de chlorométhyle et comme décrit à l'exemple II. On agite 30 minutes à 5°C puis 30 minutes à 20°C. On verse le mélange réactionnel dans une solution constituée par 260 cm3 d'eau à +10+15°C, 20 cm3 d'une solution aqueuse N de bicarbonate de sodium et 50 cm3 d'acétate d'éthyle, on agite, décante, extrait à l'acétate d'éthyle, lave à l'eau, sèche et distille à sec sous pression réduite. On reprend le résidue par 50 cm3 d'éther isopropylique, essore et obtient 5,53 g de produit attendu.

Stade B

3-méthylthiométhyl 7-/2-(2-amino thiazol-4-yl) 2-hydroxyimino acétamido) ceph-3-ème 4-carboxylate de 1-oxopropoxyméthyle isomère syn

Pendant 10 minutes on agite à 50°C, 3,75 g du produit obtenu précédemment dans 18,7 cm3 d'acide formique aqueux à 66%. On ajoute 7,5 cm3 d'eau, essore, distille le filtrat sous pression réduite à 30°C maximum et effectue 2 entrainements par le mélange éthanol-eau 2/1. On reprend le résidu par 10 cm3 d'eau, essore, rince à l'eau, puis à l'éther éthylique et obtient 2,17 g de produit brut. On le chromatographie sur silice, élue par un mélange acétate d'éthyle-acétone (3/1) reprend le résidu sec par 10 cm3 d'éther isopropylique, essore 1,55 g de produit que l'on reprend par 4,5 cm3 d'acétate d'éthyle, ajoute 7,5 cm3 d'acétate d'éthyle, essore, rince à l'acétate d'éthyle puis à l'éther isopropylique et obtient 0,536 g de produit attendu.

Spectre UV

(éthanol)

$\lambda$max 222 nm $E_1^1$=384 $\varepsilon$=19.800

$\lambda$max 262 nm $E_1^1$=271 $\varepsilon$=14.000

(éthanol, HCl N/10)

infl 218 nm $E_1^1$=293

$\lambda$max 263 nm $E_1^1$=382 $\varepsilon$=19.700

Spectre RMN ($CDCL_3$) ppm

1,17 (t; J=7 } $C_2H_5$
2,42 (q; J=7)}
2,1: $CH_3S$
5,11 (d; J=5) H6
5,9: COO—$CH_2O$
7,1: $H_5$ du thiazole

Spectre IR (Nujol)

C=O $\beta$ lactame $1779^{cm^{-1}}$
ester+propionate $1738^{cm^{-1}}$ $1759^{cm^{-1}}$
amide $1664^{cm^{-1}}$
$NH_2$ (de formation) $1613^{cm^{-1}}$
thiazole+amide II 1528 $cm^{-1}$

Exemple 5

Acide 3-éthylthiométhyl 7-/2-(2-amino thiazol-4-yl) 2-hydroxyimino acétamido/ceph-3-ème 4-carboxylique isomère syn

Stade A

Acide 3-éthylthiométhyl 7-/2-(2-tritylamino thiazol-4-yl) 2-hydroxyimino acétamido/ceph-3-éme 4-carboxylique isomère syn

A température ambiante et sous atmosphère inerte, on dissout 1,37 g d'acide 3-éthylthiométhyl 7-amino ceph-3-ème 4-carboxylique dans 15 cm3 de chlorure de méthylene et 1,75 cm3 de triéthylamine. On refroidit à —30°C pour introduire, en une fois 3,97 g d'anhydride mixte tosylique de l'acide 2-(2-tritylamino thiazol-4-yl) 2-/(1-methyl 1-méthoxy) éthoxy imino/acétique isomère syn. On laisse revenir à 0°C, agite pendant 2 heures à cette température, puis neutralise par 0,75 cm3 d'acide acétique. On extrait au chlorure de méthylène, lave à l'eau la phase organique, sèche et concentre à sec sous pression réduite à ⩽30°C. On triture le résidu dans 20 cm3 d'éther, essore 3,55 g de produit brut. Aprés recristallisation dans le méthanol, on obtient 2,17 g de produit attendu.

Stade B

Acide 3-éthylthiométhyl 7-/2-(2-amino thiazol-4-yl) 2-hydroxyimino acétamido/ceph-3-ème 4-carboxylique isomère syn

On dissout 2,16 g de produit obtenu ci-dessus dans 10,8 cm3 d'acide formique aqueux (à 66%), agite 10 minutes à 50°C et ajoute 4,3 cm3 d'eau. On essore l'insoluble, concentre à sec le filtrat sous pression réduite à moins de 35°C et reprend le résidu, par 6 cm3 d'eau pour concréter le produit. On essore, sèche et obtient 1,168 g de produit brut. On le purifie par dissolution dans 6 cm3 d'eau et 0,4 cm3 de triéthylamine. Le sel de triéthylamine cristallise. On ajoute 10 cm3 d'eau. On filtre un insoluble, le lave avec 5 cm3 de méthanol ajoute au filtrat 11 cm3 d'éthanol et acidifie à pH: 3—4 par 1 cm3 d'acide formique à 50% d'eau. Après un repos de 30 minutes à 20°C, on essore le produit cristallisé, le rince à l'éthanol anhydre, enfin à l'éther éthylique. On obtient 0,885 g de produit attendu.

Spectre UV (EtOH, HCl N/10)

infl 218 nm $E_1^1$=320
max 263 nm $E_1^1$=448 $\varepsilon$=19900

Spectre RMN (DMSO) ppm

1,15 (t: J=7) } —S—Et
2,62 (q: J=70}
3,61 $CH_2S$—Et
11,4: N—OH
6,75 $H_5$ du thiazole
5,12—5,2 $H_6$

5,61—5,7 } $H_7$
5,75—5,83}
9,62—9,48 NH—C=O

L'acide 3-éthylthiométhyl 7-amino ceph 3-ème 4-carboxylique utilisé au début de l'exemple a été préparé comme suit:

On mélange 54,4 g d'acide 7-amino céphalosporanique et 544 cm3 d'acide acétique. On ajoute 170 cm3 d'éthérate de trifluorure de bore, puis 45 cm3 d'éthanediol et on agite pendant 2 heures à 45°—50°C. On refroidit à 20—30°C, ajoute 170 cm3 de triéthylamine et essore le précipité formé. On le rince à l'acide acétique, à l'acétone et à l'éther éthylique et obtient 32,45 g de produit attendu.

Spectre UV (EtOH, HCl N/10)
max 262 nm $E_1^1$: 217 $\varepsilon$=5950

Spectre RMN (DMSO) ppm
1,13 (t: J=7) } —S—Et
2,46 (q: J=7) }
3,6 —$CH_2S$
4,72—4,79 et 4,98—5,05 H du $\beta$ lactame

Exemple 6
Acide 3-éthoxyméthyl 7-/2-(2-amino thiazol-4-yl) 2-hydroxyimino acétamido/ceph 3-ème 4-carboxylique isomère syn
Stade A
Acide 3-éthoxyméthyl 7-/2-(2-tritylamino thiazol 4-yl) 2-(1-méthoxy 1-méthyl éthoxy) imino acetamido/ceph 3-ème 4-carboxylique isomère syn

On opère comme au stade A de l'exemple 5, avec 1,29 g d'acide 3-éthoxyméthyl 7-amino ceph 3-ème 4-carboxylique pour obtenir 3,74 g de produit brut que l'on recristallise dans de l'acétate d'éthyle pour isoler 1,791 g de produit attendu.

Spectre UV
1/(EtOH)
infl: 230 nm $E_1^1$: 370
infl: 260 nm $E_1^1$: 233 $\varepsilon$: 17.300
infl: 300 nm $E_1^1$: 80 $\varepsilon$: 5.900
2/(EtOH, HCl N/10)
max: 271 nm $E_1^1$: 256 $\varepsilon$: 19.000

Spectre RMN ($CDCl_3$) ppm
1,05—1, 13—1 21 et 3,23—3,31—3,38—3,47: $OCH_2$—$CH_3$
1,54: $CH_3$ géminé
3,24: $OCH_3$
3,51: —S—$CH_2$
4,15: —$CH_2$—O
4,98—5,05: $H_6$
de 5,68 à 5,87: $H_7$
6,75=$H_5$ du thiazole

Stade B
Acide 3-éthoxyméthyl 7-/2-(2-amino thiazol 4-yl) 2-hydroxyimino acétamido/ceph 3-ème 4-carboxylique isomère syn

On dissout, en agitant, 1,735 g d'acide 3-éthoxy méthyl 7-/2-(2-tritylamino thiazol 4-yl) 2-(1-méthoxy 1-méthyl) éthoxy imino acétamido/ceph 3-ème 4-carboxylique isomère syn et 8,6 cm3 d'acide formique aqueux (à 66%). On chauffe à 50°C pendant 10 minutes et ajoute à chaud 3,5 cm3 d'eau. On essore le triphénylcarbinol formé, concentre presqu'à sec le filtrat sous pression réduite, ajoute 5 cm3 d'éthanol à 50% d'eau et concentre à sec à moins de 35°C. On reprend le résidu par 6 cm3 d'eau, triture pour concréter le produit, essore, rince à l'eau puis à l'éther et obtient 0,915 g de produit brut. On dissout 0,813 g de ce dernier dans 10 cm3 d'éthanol à 50% d'eau et 0,45 cm3 de triéthylamine. On filtre, rince avec 6 cm3 d'éthanol aqueux acidifie le filtrat à pH: 3.4 en ajoutant 0,7 cm3 d'acide formique à 50%. On amorce la cristallisation, essore, rince à l'éthanol aqueux et à l'éther pour obtenir 0,688 g de produit attendu.

Spectre UV (EtOH, HCl N/10)
infl: 219 nm E: 288 $\varepsilon$: 12.300
max: 261 nm E: 409 $\varepsilon$: 17.500

Spectre RMN (DMSO) ppm
1,11 (t: J=7) } —OEt
3,40 (q: J=7) }
4,22 —$CH_2$—O—
5,11 —5,19 $H_6$
5,67—5,75—5,8—5,87 $H_7$
6,75 $H_5$ du thiazole
7,17 $NH_2$
9,47—9,6 NHCO

L'acide 3-éthoxyméthyl 7-amino ceph 3-ème 4-carboxylique utilisé au début de l'exemple peut être préparé de la façon suivante:

On agite 27,2 g d'acide 7-amino céphalosporanique et 272 cm3 d'acétonitrile anhydre, ajoute 128 cm3 d'éthérate de trifluorure de bore puis 71 cm3 d'éthanol et chauffe à 45°—50°C pendant 16 heures sous atmosphère inerte. On refroidit le mélange réactionnel à 15—20°C, et ajoute 98 cm3 de triéthylamine en 15 minutes. On essore le précipité, rince à l'acétonitrile, à l'acétone puis à l'éther éthylique pour obtenir 19,25 g de produit brut. On dissout 18 g de celui-ci dans 54 cm3 d'acide chlorhydrique 2N, chauffe à 45°C, traite au charbon actif, filtre et neutralise le filtrat par addition de 11 cm3 d'ammoniaque à chaud. On essore à 20°C le précipité obtenu, rince à l'eau, à l'acétone et à l'éther et obtient 8,6 g de produit. On reprend 5,86 g de ce dernier par 24 cm3 d'acide chlorhydrique 2N, agite pendant 1 heure à 45°C et ajoute 4 cm3 d'ammoniaque à chaud. On essore à 20°C, rince à l'eau, à l'acétone puis à l'éther éthylique et obtient 4,75 g de produit attendu.

Spectre UV (EtOH, HClN/10)
max: 259 nm E: 239 $\varepsilon$: 6.200

Spectre R.M.N (DMSO) ppm
1,08 (t: J=7) } —OEt
3,39 (q: J=7) }
4,18 —O—$CH_2$
4,72—4,8 et 4,93—5,03 $H_6$ et $H_7$

Exemple 7

Acide 3-méthoxy méthyl S-oxyde 7/2-(2-amino thiazol 4-yl) 2-hydroxyimino acetamido/ceph 3-ème 4-carboxylique isomère syn

Stade A

Acide 3-méthoxy méthyl S-oxyde 7/2-(2-tritylamino thiazol 4-yl) 2/(1-méthyl 1-méthoxy)éthoxy imino/acetamido/ceph 3-ème 4-carboxylique isomère syn.

On dissout à température ambiante 1,45 g d'acide 3-méthoxyméthyl 7/2-(2-tritylamino thiazol-4-yl) 2/(1-méthyl 1-méthoxy) éthoxyimino/acétamido/ceph-3-eme 4-carboxylique isomère syn dans 14 cm3 de chlorure de méthylène, refroidit à —20°C, ajoute 440 mg d'acide métachloroperbenzoïque, agite 15 mn et laisse remonter à la température ambiante. On ajoute 10 cm3 d'éther isopropylique chasse le chlorure de méthylène, ajoute de nouveau 10 cm3 d'éther isopropylique, essore 1,47 g de produit brut.

Stade B

Acide 3-méthoxy méthyl S-oxyde 7/2-(2-amino thiazol 4-yl) 2-hydroxyimino acetamido/ceph 3-ème 4-carboxylique isomère syn

On mélange 1,47 g de produit obtenu au stade A et 5 cm3 d'une solution aqueuse d'acide formique à 66%. On ajoute 50 cm3 d'éther éthylique, essore le précipité, obtient 0,785 g de produit brut que l'on reprend par 1 cm3 d'eau et 2 gouttes de pyridine, ajoute 10 cm3 d'éthanol, essore, lave à l'éthanol puis à l'éther et obtient 0,51 g de produit attendu.

Spectre UV (EtOH, HCl N/10)
inf 218 nm $E_1^1$=303
max 261 nm $E_1^1$=423 $\varepsilon$ 18.200
inf 375 nm $E_1^1$=2

Spectre RMN (DMSO) ppm
3,25: $OCH_3$
4,3—4,5 et 4—4,21: $CH_2$—O
4,98—5,06: $H_6$
6,83: $H_5$ du thiazole
5,95—5,98 et 6,05—6,13: $H_7$

Exemple 8

Acide 3-méthyl sulfinylméthyl S-oxyde 7/2-(2-amino thiazol 4-yl) 2-hydroxyimino acetamido/ceph-3-ème 4-carboxylique isomère syn

On opère comme à l'exemple 7 à partir de 0,744 g d'acide 3-méthylthiométhyl 7-/2-(2-tritylamino thiazol 4-yl) 2-/(1-méthyl 1-méthoxy) éthoxyimino/acetamido/ceph 3-ème 4-carboxylique isomère syn et 0,404 g d'acide métachloroperbenzoïque. On obteient 0,250 g de produit attendu.

Spectre UV (EtOH, HCl N/10)
max: 269 nm $E_1^1$=396 $\varepsilon$: 18.300

Spectre RMN (DMSO) ppm
2,6: $CH_3$—S—O
5—5,08: $H_6$
5,88—5,96 } $H_7$
6,03—6,12 }
6,78: $H_5$ du thiazole

Exemple 9
3-méthylthiométhyl 7-/2-(2-tritylamino thiazol-4-yl) 2-(1-méthyl 1-méthoxy) éthoxy imino/ acétamido/ceph-3-ème 4-carboxylate de 1/-(1-oxopropyl) oxy/propyle isomère syn
Stade A
3-méthylthiométhyl 7-/2-(2-tritylamino thiazol-4-yl) 2-/(1-méthyl 1-méthoxy) éthoxy imino/acétamido/ceph-3-ème 4-carboxylate de 1/-(1-oxopropyl) oxy/propyle isomère syn

On mélange sous atmosphère inerte 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn dans 16,5 cm3 de diméthylformamide et 0,49 g de carbonate de potassium. On ajoute goutte à goutte 3 cm3 de propionate de bromopropyle préparé comme ci-après et agite pendant 15 minutes. On verse le mélange réactionnel dans 200 cm3 d'eau puis extrait à l'acétate d'éthyle. On lave la phase organique à l'aide d'une solution aqueuse saturée en chlorure de sodium, sèche, concentre à sec sous pression réduite à une température inférieure à 40°C et obtient 6,3 g de produit brut.

Stade B
3-méthylthiométhyl 7-/2-(2-amino thiazol-4-yl) 2-hydroxyimino acétamido/ceph-3-ème 4-carboxylate de 1-/(1-oxopropyl) oxy/propyle isomère syn

On agite pendant 1 heure 45 6,3 g de produit obtenu au stade précédent dans 53 cm3 d'une solution d'acide formique à 90%, filtre, ajoute au filtrat 500 cm3 d'eau, agite 5 minutes et extrait à l'acétate d'éthyle. On lave la phase organique à l'aide d'une solution aqueuse saturée en chlorure de sodium, sèche, concentre à ses sous pression réduite et obtient 2,66 g de produit attendu que l'on purifir par chromatographie sur silice (éluant: chlorure de méthylène-méthanol 92—8). On évapore à sec, dissout le résidu dans le méthanol et le recristallise dans l'éther isopropylique. On obtient 1,11 g de produit attendu pur. F=140°C (décomposition).

Spectre UV (EtOH, HCl N/10)
max.: 264 nm $E_1^1$=347 $\varepsilon$=18800

Spectre RMN ($CDCl_3$) ppm
2,06: $SCH_3$
1,03 à 1,26 et 2,21 à 2,56: C—Et
‖
O
6,83—7,08: $CO_2$—CH—
0,85 à 1,83: Et.

Le propionate de bromopropyle utilisé au stade A de l'exemple 9 a été préparé comme suit:

On mélange sous agitation 180 mg de chlorure de zinc et 30 cm3 de bromure de propionyle, refroidit à +5°C et ajoute en 25 minutes 26,4 cm3 de propionaldéhyde. On laisse 15 heures sous agitation à température ambiante puis distille sous pressio réduite et recueille 14,27 g de produit attendu utilisé tel quel pour le stade suivant.

Exemple 10
3-méthylthiométhyl 7-/2-(2-amino thiazol-4-yl) 2-hydroxyimino acétamido/ceph-3-ème 4-carboxylate de cyanométhyle isomère syn

On opère comme à l'exemple 9 à partir de 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn et 2,2 cm3 de bromure d'acétonitrile. On obtient 0,767 g de produit cristallisé que l'on purifie par addition d'éther isopropylique. F~=145°C.

Spectre UV (EtOH, HCl N/10)
max.: 265 nm $E_1^1$=367 $\varepsilon$=17200

Spectre RMN (DMSO) ppm
2,01: $SCH_3$
5,23: $COOCH_2$
6,75: $H_5$ du thiazole

Exemple 11
3-méthylthiométhyl 7-/2-(2-amino thiazol-4-yl) 2-hydroxyimino acétamido/ceph-3-ème 4-carboxylate de 1-/(1-oxoéthyl oxy/propyle isomère syn

On opère comme à l'exemple 9 à partir de 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn et 7 cm3 d'acétate de 3-bromopropyle en solution chloroformique. On obtient 0,965 g de produit pur. F=130°C (décomposition).

Spectre UV (EtOH, HCl N/10)
max.: 264 nm $E_1^1$=347 $\varepsilon$=18400

Spectre RMN ($CDCl_3$) ppm
0,85 à 1,08: $CH_2$—$CH_3$
1,88 et 1,93: $SCH_3$ et 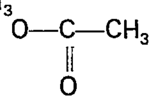
7,01: $H_5$ du thiazole
6,83 à 7,16: COOCH—O

L'acétate de 3-bromopropyle utilisé à l'exemple 11 a été préparé comme suit:

On mélange 37 cm3 de bromure d'acétyle et 0,242 g de chlorure de zinc puis ajoute en 12 minutes à 15°C, 40 cm3 de propionaldéhyde. On laisse 15 heures sous agitation à température ambiante puis distille à 53°—58°C (80/90 mm Hg). On mélange sous agitation et à température ambiante 5 cm3 du produit recueilli et 5 cm3 de chloroforme, ajoute par petites fractions 840 mg d'hexaméthylène tétramine, agite 10 minutes et obtient une solution chloroformique d'acétate de 3-bromopropyle utilisé immédiatement.

Exemple 12
3-méthylthiométhyl 7-/2-(2-amino thiazol-4-yl) 2-hydroxyimino acétamido/ceph-3-ème 4-carboxylate de l'acétyloxy éthyl isomère syn

On opère comme à l'exemple 9 à partir de 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn et 1,55 cm3 d'acétate de bromoéthyl et obtient 0,867 g de produit attendu. F~150°C (décomposition).

Spectre UV (EtOH, HCl N/10)
max.: 265 nm $E_1^1$=347 $\varepsilon$=17800

Spectre RMN ($CDCl_3$) ppm
2,26—2,35: $\underline{CH_3}$—CH
3,06—3,12: S—$CH_3$ et OAc
5,38: les $CH_2S$
10,4: $H_5$ du thiazole et 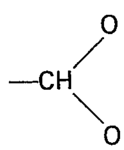

Exemple 13
3-méthylthiométhyl 7-/2-(2-amino thiazol-4-yl) 2-hydroxyimino acétamido/ceph-3-ème 4-carboxylate de 2-chloro 2-propényle isomère syn

Stade A
3-méthylthiométhyl 7-/2-(2-tritylamino thiazol-4-yl) 2-(1-méthyl 1-méthoxy) éthoxy imino/acétamido ceph-3-ème 4-carboxylate de 2-chloro 2-propényle isomère syn

On dissout sous atmosphère inerte 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn dans 16 cm3 de diméthylformamide et 0,477 g de carbonate de potassium. On ajoute goutte à goutte la solution acétonique de 3-iodo 2-chloro 1-propène préparée ci-dessous. Après 30 minutes, on distille l'acétone sous pression réduite à une température inférieure à 40°C. On mélange la solution de diméthylformamide avec 250 ml d'éther isopropylique, décante, sèche le résidu sous pression réduite et recueille 7,8 g de produit brut.

Stade B
3-méthylthiométhyl 7-/2-(2-amino thiazol-4-yl) 2-hydroxyimino acétamido/ceph-3-ème 4-carboxylate de 2-chloro 2-propényle isomère syn

On opère comme au stade B de l'exemple 9 à partir de 7,8 g de produit obtenu au stade précédent, et recueille 1,18 g de produit attendu. F~146°C (décomposition).

Spectre UV (EtOH, HCl N/10)
max.: 265 nm $E_1^1$=377 ε=19000

Spectre RMN ($CDCl_3$) ppm
2,03: S—$CH_3$
3,63: S—$CH_2$
4,83 à 5,6: $CO_2CH_2$ et $CH_2$=
5,1—5,18: $H_6$

La solution acétonique de 3-iodo 2-chloro 1-propène utilisée au stade A de l'exemple a été préparée comme suit:

On mélange sous atmosphère inerte 6,1 cm3 de 2,3-dichloro 1-propène et 10 g d'iodure de sodium dans 43 ml d'acétone. On porte au reflux pendant 40 minutes, ramène à température ambiante et essore le chlorure de sodium formé. La solution acétonique de 3-iodo 2-chloro 1-propène est utilisée immédiatement.

Exemple 14
3-méthylthiométhyl 7-/2-(2-amino thiazol-4-yl) 2-hydroxyimino acétamido/ceph-3-ème 4-carboxylate de méthylthiométhyle isomère syn
Stade A
3-méthylthiométhyl 7-/2-(2-tritylamino thiazol-4-yl) 2-/(1-méthyl 1-méthoxy) éthoxy imino/acétamido/ceph-3-ème 4-carboxylate de méthylthiométhyle isomère syn

On dissout sous atmosphère inerte 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn dans 16 cm3 de diméthylformamide et 480 mg de carbonate de potassium. On ajoute goutte à goutte 3,4 cm3 de chlorométhyl méthyl sulfure et agite pendant 25 minutes. On verse le mélange dans 200 cm3 d'une solution aqueuse saturée en chlorure de sodium essore le précipité formé, le dissout dans le chloroforme, lave avec une solution saturée en chlorure de sodium, sèche et concentre à sec sous pression réduite à une température inférieure à 40°C. On reprend le résidu à l'éther de pétrole (Eb: 60°—80°C) jusqu'à concrétion du produit attendu. On obtient 5,2 g de produit utilisé tel quel pour le stade suivant.

Stade B
3-méthylthiométhyl 7-/2-(2-amino thiazol-4-yl) 2-hydroxyimino acétamido/ceph-3-ème 4-carboxylate de méthylthiométhyle isomère syn

On dissout 5,05 g du produit obtenu au stade A dans 50 cm3 de méthanol et laisse 3 jours à température ambiante. On concentre sous pression réduite à une température inférieure à 40°C, reprend le résidu par 15 cm3 d'éther isopropylique et agite 20 minutes à température ambiante. On essore le précipité formé, le rince à l'éther isopropylique et obtient 4,5 g de produit que l'on purifie par chromatographie sur silice (éluant: chlorure de méthylène-méthanol 92—8). On concentre à sec, reprend le résidu dans le chloroforme, précipite par addition d'éther isopropylique, essore, sèche et obtient 0,969 g de produit attendu F=140°C (décomposition).

Spectre UV: (EtOH, HCl N/10)
max.: 265 nm $E_1^1$=379 ε=18600

Spectre RMN ($CDCl_3$) ppm
2,08: S—$CH_3$ en 3
2,3: S—$CH_3$ en 4
5,51: $CO_2$—$CH_2$—S

Exemple 15
3-méthylthiométhyl 7/2-(2-amino thiazol-4-yl) 2-hydroxyimino acétamido/ceph-3-ème 4-carboxylate de 1-/(2,2-diméthyl 1-oxopropyl) oxy/éthyle isomère syn
Stade A
3-méthylthiométhyl 7-/2-(tritylamino thiazol-4-yl) 2-/(1-méthyl 1-méthoxy) éthoxyimino/acétamido/ceph-3-ème 4-carboxylate de 1-/(2,2-diméthyl 1-oxopropyl) oxy/éthyle isomère syn

On introduit lentement sous atmosphère inerte, 3,72 g d'acide 3-méthylthiométhyl 7-/2-(2-tritylamino thiazol-4-yl) 2-/(1-méthyl méthoxy) éthoxyimino/acétamido/ceph-3-ème 4-carboxylique isomère syn dans 7,5 cm3 de tétraméthyl urée. On ajoute 0,375 g de carbonate de potassium puis le tert-butyl carboxylate de 1-iodo éthyle, préparé ci-dessous et de nouveau en 1 heure 10, 1,125 g de carbonate de potassium. On agite 10 minutes puis verse le mélange réactionnel sur un mélange composé de 500 cm3 d'eau, 250 g de glace et 25 cm3 d'acide chlorhydrique N/10. On agite 15 minutes, extrait à l'acétate d'éthyle. On lave la phase organique à l'eau puis à l'aide d'une solution aqueuse saturée en

chlorure de sodium, sèche et concentre à sec à une température inférieure à 30°C. On obtient 8,69 g de produit brut que l'on reprend dans l'éther de pétrole (60°—80°C) jusqu'à concrétion. On recueille 5,42 g de produit attendu.

Stade B

3-méthylthiométhyl 7-/2-(2-amino thiazol-4-yl) 2-hydroxyimino acétamido/ceph-3-ème 4-carboxylate de 1-/(2,2-diméthyl 1-oxopropyl) oxy/éthyle isomère syn

On reprend le produit obtenu au stade précédent dans 55 cm3 d'acide formique à 88%, agite pendant 1 heure 30, filtre, ajoute au filtrate 650 cm3 d'eau glacée et extrait au chloroforme. On lave la phase organique à l'eau puis avec une solution aqueuse saturée en chlorure de sodium, sèche et concentre à sec sous pression réduite à une température inférieure à 35°C. On recueille 3,04 g de produit que l'on chromatographie sur silice (éluant: chlorure de méthylène-méthanol 98—2). On concentre à sec et reprend le résidu dans le chloroforme et précipite par addition d'éther isopropylique, sèche et obtient 0,733 g de produit attendu. F~140°C.

Spectre UV: (EtOH, HCl N/10)

infl: 219 nm $E_1^1$=265

max: 265 nm $E_1^1$=337 $\varepsilon$=18800

Spectre RMN ($CDCl_3$) ppm

2,06 $CH_3$—S

1,19—1,23: t—Bu

1,5—1,6: $\underline{CH_3}$—CH

3,33 et 3,9: $CH_2$—S

Le tert-butyl carboxylate de 1-iodo éthyle utilisé au stade A de l'exemple a été préparé comme suit:

Stade a) Pivalate de 2-chloroéthyle

On mélange 61,4 cm3 de chlorure de pivaloyle et 250 mg de chlorure de zinc, agite 5 minutes, refroidit à +15°C et ajoute en 1 heure 40 cm3 d'acétaldéhyde. Après 15 heures à température ambiante, on distille sous pression réduite et recueille 29,01 g de produit distillant entre 56 et 58°C (27 mmHg)

Stade b) tert-butyl carboxylate de 1-iodo éthyle

On introduit lentement dans 20 cm3 de tétraméthylurée 7,45 g d'iodure de sodium, agite 10 minutes et ajoute 5 cm3 de pivalate de 2-chloroéthyle. On agite 30 minutes à 18°C et utilise ce réactif immédiatement.

Exemple 16

3-méthylthiométhyl 7-/2-(2-amino thiazol-4-yl) 2-hydroxyimino acétamido/ceph-3-ème 4-carboxylate de phénylméthyle isomère syn

Stade A

Sel de sodium de l'acide 3-méthylthiométhyl 7-/2-(2-amino thiazol-4-yl) 2-hydroxyimino acétamido/ceph-3-ème 4-carboxylique isomère syn

On dissout 1 g d'acide 3-méthylthiométhyl 7-/2-(2-amino thiazol-4-yl) 2-hydroxyimino acétamido/ceph-3-ème 4-carboxylique isomère syn dans 2 cm3 de diméthylformamide puis ajoute 3,5 cm3 d'une solution méthanolique d'acétate de sodium et 30 cm3 d'éthanol. On essore le précipité, rince à l'éthanol puis à l'éther éthylique et recueille 0,804 g de produit attendu.

Stade B

3-méthylthiométhyl 7-/2-(2-amino thiazol-4-yl) 2-hydroxyimino acétamido/ceph-3-ème 4-carboxylate de phénylméthyle isomère syn

On mélange 0,84 g de produit préparé au stade A et 4 cm3 de diméthylformamide, ajoute lentement 0,21 cm3 de bromure de benzyle et laisse 1 heure à température ambiante. On ajoute 40 cm3 d'eau, essore le précipité, rince à l'eau puis à l'éther isopropylique, sèche et obtient 0,605 g de produit brut que l'on chromatographie sur silice (éluant: chlorure de méthylèneméthanol 95—5). Après cristallisation dans l'éther isopropylique, on obtient 0,225 g de produit pur. F=114°C.

Spectre UV. (EtOH, HCl N/10)

Max. 263—264 nm $\varepsilon$: 19400

Spectre RMN: ($CDCl_3$) ppm.
2,01: S—$CH_3$
7,08: $H_5$ du thiazole
7,43: H du phényle
5,3: $CO_2$ $C\underline{H}_2$ $\phi$

Exemple 17
3-méthylthiométhyl 7-/2-(2-amino thiazol-4-yl) 2-hydroxyimino acétamido/ceph-3-ème 4-carboxylate de 1-(oxo hexadecanoxy) méthyle isomère syn.

On opère comme à l'exemple 9 à partir de 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn et 20 cm3 de solution acétonique de palmitate d'iodométhyle préparé ci-dessous. On obtient 2,02 g de produit après recristallisations dans l'éther de pétrole (60°—80°C). F=125°C.

Spectre UV. (EtOH, HCl N/10)
Max. 264 nm $E_1^1$=268 $\varepsilon$=18700

Spectre RMN ($CDCl_3$)
0,89: $CH_3$ de l'ester
2,37: $CH_2$ de l'ester
2,04: S—$CH_3$

Le palmitate d'iodométhyle utilisé dans l'exemple a été préparé comme suit:

Stade a: Palmitate de chlorométhyle

On chauffe à 80°C sous atmosphère inerte pendant 20 minutes 33 cm3 de chlorure de palmitolye, 3 g de paraformaldéhyde et 50 mg de chlorure de zinc. On ramène à température ambiante, essore le précipité et le dissout dans 10 cm3 de chloroforme, ajoute 100 cm3 d'éthanol, essore, obtient 5 g de produit cristallisé.

Stade b: Palmitate d'iodométhyle

On porte au reflux pendant 20 minutes sous atmosphère inerte, le mélange de 4,116 g de produit obtenu au stade a et de 2,025 g d'iodure de sodium dans 20 cm3 d'acétone. On laisse revenir à température ambiante, filtre l'insoluble et recueille la solution acétonique que l'on utilise immédiatement.

Exemple 18
3-méthylthiométhyl 7-/2-(2-amino thiazol-4-yl) 2-hydroxyimino acétamido/ceph-3-ème 4-carboxylate de (2,2-diméthyl 1-oxopropoxy) méthyle isomère syn

On opère comme au stade A de l'exemple 9 à partir de 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn et la solution de pivalate d'iodométhyle préparée à partir de pivalate de chlorométhyle et d'iodure de sodium dans l'acétone. On obtient après concrétion dans l'éther isopropylique 4,1 g de produit brut utilisé tel quel pour la suite de la synthèse. On opère comme au stade B de l'exemple 9 et obtient 1,98 g de produit attendu. F=140°C.

Spectre UV. (EtOH, HCl N/10)
Max. 264 nm $E_1^1$=325 $\varepsilon$=17700

Spectre RMN ($CDCl_3$) ppm
2,02: $CH_3$—S
3,61: $CH_2$—S
6,93: $H_5$ du thiazole
1,24: t.Bu

Exemple 19
3-méthylthiométhyl 7-/2-(2-amino thiazol-4-yl) 2-hydroxyimino acétamido/ceph-3-ème 4-carboxylate de 1-oxopentoxy méthyle isomère syn

On opère comme au stade A de l'exemple 9 à partir de 3,72 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn et de la solution de n-valérate d'iodométhyle préparée ci-dessous. Le mélange réactionnel est extrait au chloroforme. La phase organique est lavée avec une solution aqueuse saturée en chlorure de sodium et concentrée à sec. On reprend à l'éther de pétrole (Eb: 60°—80°C), concentre à sec, reprend à l'éther de pétrole jusqu'à concrétion et recueille 4,8 g de produit attendu.

On opère comme au stade B de l'exemple 9 à partir de 4,7 g du produit obtenu ci-dessus et 28,2

cm3 d'une solution d'acide formique à 90%. Après extraction au chloroforme et cristallisations dans l'éther isopropylique, on obtient 0,6 g de produit attendu. F~158°C (décomposition).

Spectre UV (EtOH, HCl N/10)
Max.: 265 nm $E_1^1$=360 ε=19600

Spectre RMN ($CDCl_3$) ppm.
2,03: S—$CH_3$
0,82 à 0,97: $C\underline{H}_3$—$CH_2$
2,3 à 2,46: C—$CH_2$
‖
O
6,96: $H_5$ du thiazole

Le n'valérate d'iodométhyle utilisé dans l'exemple a été préparé comme suit:

On agite pendant 4 heures à 90°C le mélange de 12 g de paraformaldéhyde et 48,2 g de chlorure de l'acide n-valérique auquel on a ajouté 0,3 g de chlorure de zinc. On distille sous pression réduite à 71,5°—73°C (20 mmHg). On introduit 2,94 cm3 du produit recueilli et 2,25 g d'iodure de sodium dans 15 cm3 d'acétone, porte au reflux 20 minutes, laisse revenir à température ambiante, essore l'insoluble et recueille la solution acétonique de n-valérate d'iodométhyle que l'on utilise immédiatement.

Exemple 20

3-méthylthiométhyl 7-/2-(2-amino thiazol-4-yl) 2-hydroxyimino acétamido/ceph-3-ème 4-carboxylate de 1-oxobutoxy méthyle isomère syn.

On opère comme au stade A de l'exemple 15, à partir de 3,72 g d'acide 3-méthylthiométhyl 7-/2-(2-tritylamino thiazol-4-yl) 2-(1-méthyl 1-méthoxy) éthoxy imino/acétamido ceph-3-ème 4-carboxylique isomère syn et la solution de n-butyrate d'iodométhyle préparée ci-dessous. On obtient 4,52 g de produit que l'on mélange à 45 cm3 d'acide formique à 90%. On poursuit la synthèse comme au stade B de l'exemple 15 et recueille 0,627 g de produit attendu. F=160°C (décomposition).

Spectre UV (EtOH, HCl N/10)
inf.: 261 nm $E_1^1$=290 ε=15300
max.: 264 nm $E_1^1$=355 ε=18800

Spectre RMN (ppm)
0,86 à 1,02: $CH_3$ du propyle
2,27 à 2,43: CO—$CH_2$
5,88: C—O—$CH_2$—O—
‖
O
6,94: $H_5$ du thiazole

Le n-butyrate d'iodométhyle a été préparé comme suit:

On mélange 12 g de paraformaldéhyde et 42,6 g de chlorure de n-butyryle et ajoute 1 g de chlorure de zinc, agite 3 heures à température ambiante puis 4 heures à 90°C. On distille (sous pression de 180—190 mm Hg) et recueille la fraction dont le point d'ébullition est compris entre 104—107°C. On introduit lentement 2,8 cm3 du produit recueilli dans le mélange de 6 cme de tétraméthylurée et de 3,4 g d'iodure de sodium, agite pendant 2 heures, essore et utilise le filtrat immédiatement.

Exemple 21

3-méthylthiométhyl 7-/2-(2-amino thiazol-4-yl) 2-hydroxyimino acétamido/ceph-3-ème 4-carboxylate d'acétyloxyméthyle isomère syn

On opère comme à l'exemple 9 à partir de 5 g d'acide 3-méthylthiométhyl 7-/2-(2-tritylamino thiazol-4-yl) 2-/(1-méthyl 1-méthoxy) éthoxy imino/Acétamido/ceph-3-ème 4-carboxylique isomère syn et 20 cm3 de solution acétonique d'acétate d'iodométhyle contenant 1,02 g d'acétate de chlorométhyle et 2,67 g d'iodure de sodium, et préparée extemporément. Avant la purification par chromatographie, on élimine l'acide formique résiduel par entraînement au nitrométhane. On recueille 1,677 g de produit attendu pur. F~120°C (décomposition).

Spectre UV (EtOH, HCl N/10)
max.: 263 nm $E_1^1$=354 ε=17800

Spectre RMN (DMSO) ppm
2: $CH_3S$
2,04: OAC
5;86: $COOCH_2O$
6,72: $H_5$ du thiazole

Exemple 22
3-méthylthiométhyl 7-/2-(2)amino thiazol-4-yl) 2-hydroxyimino acétamido/ceph-3-ème 4-carboxylate de 3,3-diméthyl 2-oxobutyle isomère syn

Stade A
3-(méthylthio) méthyl 7-/2-(2-tritylamino thiazol-4-yl) 2-/(1-méthyl 1-méthoxy) éthoxy imino/acétamido/ceph-3-ème 4-carboxylate de 3,3-diméthyl 2-oxobutyle isomère syn

On opère comme au stade A de l'exemple 15 à partir de 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn, 0,47 g de carbonate de potassium, 27 cm3 de tétraméthylurée et 0,95 cm3 de bromo-pinacolone. On obtient 7,5 g de produit utilisé tel quel au stade suivant.

Stade B
3-méthylthiométhyl 7-/2-(2-amino thiazol-4-yl) 2-hydroxyimino acétamido/ceph-3-ème 4-carboxylate de 3,3-diméthyl 2-oxobutyle isomère syn

On opère comme au stade B de l'exemple 15 à partir du produit obtenu au stade A et de 53 cm3 d'acide formique à 90%. Avant la purification par chromatographie, on effectue un entraînement au nitrométhane pour éliminer l'acide formique résiduel. On obtient 1,392 g de produit attendu. F~160°C (décomposition).

Spectre UV (EtOH, HCl N/10)
max.: 264 nm $E_1^1$=388 $\varepsilon$=20500

Spectre RMN ($CDCl_3$) ppm
1,23: t.Bu
4,82—5,1 et 5,13—5,41: COO—$CH_2CO$

Exemple 23
3-méthylthiométhyl 7-/2-(2-amino thiazol-4-yl) 2-hydroxyimino acétamido/ceph-3-ème 4-carboxylate del-(1-oxopropyloxy) éthyle isomère syn

On opère comme à l'exemple 9 à partir de 10 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn et 5,4 cm3 de propionate de bromoéthyle préparé extemporément. Après traitement à l'acide formique, on effectue un entraînement au nitrométhane pour éliminer l'acide formique residuel. On obtient 1,5 g de produit attendu, qui sont de nouveau purifiés par chromatographie sur silice (éluant: chlorure de méthylène-méthanol 92,5—7,5). On recueille 0,566 g de produit pur. F~130°C.

Spectre UV (EtOH, HCl N/10)
max.: 264 nm $E_1^1$=364 $\varepsilon$=19300

Spectre RMN ($CDCl_3$) ppm
1,5—1,6: $C\underline{H}_3$—CH
7,05: $H_5$ du thiazole

Ce propionate de bromoéthyle a été préparé comme suit:

On mélange sous pression réduite 9 cm3 de bromure de propionyle et 50 mg de chlorure de zinc, refroidit la solution à −15°C, ajoute goutte à goutte 5,4 cm3 d'acétaldéhyde puis laisse revenir la température ambiante.

Exemple 24
3-méthylthiométhyl 7-/2-(2-amino thiazol-4-yl) 2-hydroxyimino acétamido/ceph-3-ème 4-carboxylate de 1-/1-(oxobutyl) oxy/éthyle isomère syn

On opère comme à l'exemple 9 à partir de 5 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn dans le diméthylacétamide à la place du diméthylformamide et de la solution de butyrate d'iodo éthyle préparée comme ci-dessous. Avant la purification par chromatographie, on élimine l'acide formique résiduel par entraînement au nitrométhane. On obtient 0,530 g de produit attendu pur. F=127°C.

Spectre UV (EtOH, HCl N/10)
max.: 264 nm $E_1^1$=353 $\varepsilon$=19200

Spectre RMN ($CDCl_3$) ppm
0,87 à 1,03: $C\underline{H}_3$—$(CH_2)_2$
1,54—1,6: $C\underline{H}_3$—CH=
2,09: $C\underline{H}_3$—S
7,08: $H_5$ du thiazole

Le butyrate d'iodo éthyle utilisé dans l'exemple a été préparé comme suit:

Stade a: Butyrate de chloroéthyle

On mélange à 16°C sous atmosphère inerte, 52 cm3 de chlorure de butyryl et 31 cm3 d'acétaldéhyde, verse dans un flacon ajoute 0,11 g de chlorure ferrique, ferme hermétiquement. Après 18 heures de repos à température ambiante, on verse le mélange réactionnel dans 200 cm3 d'éther de pétrole (Eb: 60°—80°C), traite au charbon actif, filtre et distille le filtrat sous pression réduite à une température inférieure à 30°C. On recueille la fraction distillant entre 64 et 68°C (34 mm Hg).

Stade b: Butyrate d'iodoéthyle

On mélange 4 g de butyrate de chloroéthyle dans 16 cm3 de diméthylacétamide et ajoute peu à peu 4 g d'iodure de sodium. On agite 15 minutes et utilise le mélange immédiatement.

Exemple 25

3-méthylthiométhyl 7-/2-(2-amino thiazol-4-yl) 2-hydroxyimino acétamido/ceph-3-ème 4-carboxylate de 1-/(1-oxohexadécyl) oxy/éthyle isomère syn

On opère comme à l'exemple 15 partir de 744 mg d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn, 1,5 cm3 de tétraméthylurée, 78 mg de carbonate de potassium, le palmitate d'iodoéthyl dans la tétraméthylurée préparé extemporément, et de nouveau 210 mg de carbonate de potassium. Après extraction au chloroforme, le produit recueilli est repris par 45 cm3 d'acide formique à 98%. Après extraction au chloroforme et concrétion dans l'éther de pétrole (Eb: 60°—80°C), on obtient 0,353 g de produit attendu. F=108°C.

Spectre UV (EtOH, HCl N/10)
Inf.: 218 nm $E_1^1$=215
max.: 265 nm $E_1^1$=266 $\varepsilon$=18900

Spectre RMN ($CDCl_3$) ppm
0,88: $CH_3(CH_2)_n$
1,5—1,6: $C\underline{H}_3$—CH=
2,06: $C\underline{H}_3$—S
3,63: les $CH_2S$

Exemple 26

3-méthylthiométhyl 7-/2-(2-amino thiazol-4-yl) 2-hydroxyimino acétamido/ceph-3-ème 4-carboxylate de 2-propényle isomère syn

On opère comme à l'exemple 9 à partir de 3,72 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn dans le diméthylacétamide à la place du diméthylformamide et 1,85 cm3 d'iodure d'allyle. On obtient 1,128 g de produit pur. F~137°C (décomposition).

Spectre UV (EtOH, HCl N/10)
max.: 262 nm $E_1^1$=393 $\varepsilon$=18500

Spectre RMN ($CDCl_3$) ppm
2,08: $CH_3S$
4,73—4,83: COO—$CH_2$
5,08 à 6,5: CH=$CH_2$ et H du $\beta$ lactame
7,08: $H_5$ du thiazole

Exemple 27

3-méthylthiométhyl 7-/2-(2-amino thiazol-4-yl) 2-hydroxyimino acétamido/ceph-3-ème 4-carboxylate de 2,2-diméthyléthyle isomère syn

Stade A

3-méthylthiométhyl 7-/2-(2-tritylamino thiazol-4-yl) 2-/(1-méthyl 1-méthoxyéthoxyimino/ acetamido/ceph-3-ème 4-carboxylate de 2,2-diméthyléthyle isomère syn

On porte au reflux une solution de 1,49 g d'acide 3-méthyl thiométhyl 7-/2-(2-tritylamino thiazol-4-yl) 2-/(1-méthyl 1-méthoxy) éthoxy imino/acétamido/ceph-3-ème 4-carboxylique dans 30 cm3

d'acétate d'éthyle et 0,8 g de O (terbutyl) N-N'-diisopropylurée. On glace, essore et concentre à sec le filtrat sous pression réduite.

Stade B

3-méthylthiométhyl 7-/2-(2-amino thiazol-4-yl) 2-hydroxyimino acétamido/ceph-3-ème 4-carboxylate de 2,2-diméthyléthyle isomère syn

On reprend le résidu dans 7,5 cm3 d'acide formique à 67%, agite 15 minutes à 45°C, ajoute 3 cm3 d'eau, essore, élimine l'insoluble. On chasse le solvant du filtrat sous pression reduite à 35°C, reprend le résidu à l'aide d'une solution aqueuse d'éthanol (1-1) puis à l'eau, sèche et obtient 1,237 g de produit brut que l'on purifie par chromatographie sur silice (éluant: chlorure de méthylène-méthanol 9-1). On obtient 0,47 g de produit pur. F=107°C (décomposition).

Spectre UV (EtOH)
max.: 223 nm $\varepsilon$=18100
max.: 260 nm $\varepsilon$=13500
(EtOH, HCl N/10)
inf.: 220 nm
max.: 264 nm $\varepsilon$=18600

Spectre RMN ($CDCl_3$) ppm
1,54: t.Bu
2,11: $C\underline{H}_3$—S
7,08: $H_5$ du thiazole

Exemple 28

3-méthylthiométhyl 7-/2-(2-amino thiazol-4-yl) 2-hydroxyimino acétamido/ceph-3-ème 4-carboxylate de (2-méthoxy 2-oxoéthyle isomère syn

On opère comme à l'exemple 9 à partir de 3,44 g d'acide 3-méthylthiométhyl 7-/2-(2-tritylamino thiazol-4-yl) 2-/(1-méthyl 1-méthoxy) éthoxy imino/acétamido/ceph-3-ème 4-carboxylique isomère syn, et 10 cm3 de solution acétonique d'iodométhyl acétate préparée ci-dessous. On obtient 2,8 g de produit que l'on triture 10 minutes à 60°C avec 5,6 cm3 d'acide formique pur et 16,8 cm3 d'acide formique à 50%, élimine le précipité, effectue un entraînement au nitrométhane sur le filtrat et concentre à sec. On reprend le résidu par du chloroforme et précipite à l'éther isopropylique, sèche et recueille 1,65 g de produit brut. On reprend le résidu dans 90 cm3 d'acétate d'éthyle et 90 cm3 d'acide chlorhydrique 0,1N, extrait à l'acétate d'éthyle, concentre à sec les phases organiques réunies, reprend le résidu au chloroforme et précipite par addition d'éther isopropylique. On purifie de nouveau par chromatographie sur silice (éluant: chlorure de méthylène-méthanol 92,5—7,5) et obtient 0,4 g de produit attendu pur. F=156°C (décomposition).

Spectre UV (EtOH, HCl N/10)
max.: 264 nm $E_1^1$=383 $\varepsilon$=19200

Spectre RMN ($CDCl_3$) ppm
2,07: S—$CH_3$
3,68: les S—$CH_2$
3,8: COO—$C\underline{H}_3$
7: $H_5$ du thiazole.

L'iodométhyle acétate a été préparé comme suit:

On mélange sous atmosphère inerte 1,6 cm3 de monochloroacétate de méthyle, 2,72 g d'iodure de sodium dans 20 cm3 d'acétone et chauffe 25 minutes, au reflux, essore et recueille le filtrat que l'on emploie immédiatement.

Exemple 29

3-méthoxyméthyl 7-/2-(2-amino thiazol-4-yl) 2-hydroxyimino acétamido/ceph-3-ème 4-carboxylate de 1-(acétyloxy) éthyle isomère syn

Stade A

3-méthoxyméthyl 7-/2-(2-tritylamino thiazol-4-yl) 2-/(1-méthyl 1-méthoxy éthoxy) imino/ acétamido/ceph-3-ème 4-carboxylate de 1-(acétyloxyéthyle) isomère syn

On agite 15 minutes à 20°C un mélange de 10,92 g d'acide 3-méthoxyméthyl 7-/2-(2-tritylamino thiazol-4-yl) 2-/(1-méthyl 1-méthoxyéthoxy) imino/acétamido/ceph-3-ème 4-carboxylique isomère syn, 55 cm3 de diméthylformamide et 1,28 g de carbonate de potassium. On refroidit la solution à +5°C et ajoute 3,4 cm3 d'acétate de 1-bromoéthyle, agitate 20 minutes, ajoute 0,1 g de carbonate de potassium et 0,3 cm3 d'acétate de 1-bromoéthyle, agite 25 minutes, ajoute 550 cm3 d'eau, 150 cm3 d'acétate d'éthyle et 25 cm3 de solution aqueuse de bicarbonate de sodium (M), décante, extrait à

l'acétate d'éthyle, sèche, concentre à sec sous pression réduite à une température inférieure à 30°C. On recueille 14,3 g de produit.

Stade B

3-méthoxyméthyl 7-/2-(2-(2-amino thiazol-4-yl) 2-hydroxyimino acétamido/ceph-3-ème 4-carboxylate de 1-(acétyloxy) éthyle isomère syn

On dissout 14,3 g du produit du stade A dans 61 cm3 d'acide formique à 66%, agite 10 minutes à 50°C, ajoute 24 cm3 d'eau, élimine le précipité, concentre le filtrat sous pression réduite reprend par 50 cm3 de chlorure de méthylène et 350 cm3 d'eau et 10 cm3 d'une solution aqueuse de bicarbonate de sodium, décante, extrait au chlorure de méthylène, lave à l'eau, sèche, distille à sec sous pression réduite, reprend à l'acétate d'éthyle, essore et, sèche 4,87 g de produit attendu.

Spectre UV (EtOH)
max.: 223 nm $E_1^1$=387 ε=19300
max.: 259 nm $E_1^1$=285 ε=14200

Spectre UV (EtOH, HCl N/10)
inf.: 219 nm $E_1^1$=286
max.: 261—262 nm $E_1^1$=390 ε=19500

Spectre RMN (DMSO) ppm
1,43—1,51: $C\underline{H}_3$—CH—
2,08: oAc
3,58: $CH_2$—S
3,25: O—$CH_3$
6,75: $H_5$ du thiazole

Exemple 30

3-(éthoxy) méthyl 7-/2-(2-amino thiazol-4-yl) 2-hydroxyimino acétamido/ceph-3-ème 4-carboxylate de 1-/(1-oxoéthyl) oxy/éthyle isomère syn

On opère comme à l'exemple 29 à partir de 6 g d'acide 3-(éthoxy) méthyl 7-/2-(2-tritylamino thiazol-4-yl) 2-/(1-méthyl 1-méthoxy) éthoxy imino/acétamido/ceph-3-ème 4-carboxylique préparé à l'exemple 6 et 2,1 cm3 d'acétate de 1-bromoéthyle. On obtient 1,76 g de produit attendu.

Spectre UV (EtOH)
inf.: 220 nm $E_1^1$=268
max.: 264 nm $E_1^1$=369 ε=18900
(EtOH, HCl N/10)
inf.: 219 nm $E_1^1$=286
max.: 261 nm $E_1^1$=390 ε=19500

Spectre RMN (DMSO) ppm
1—1, 12—1, 23: $CH_3$ du O—Et
1,45—1,53: $C\underline{H}_3$—CH
2,06: OAc
6,73: $H_5$ du thiazole

Exemple 31

3-méthylthiométhyl 7-/2-(2-amino thiazol-4-yl) 2-hydroxyimino acétamido ceph-3-ème 4-carboxylate de 1-(méthoxy carbonyl) oxy/éthyle isomère syn

On opère comme à l'exemple 9 à partir de 3,72 g d'acide 3-méthylthiométhyl 7-/2-(2-tritylamino) thiazol-4-yl 2-/(1-méthyl 1-méthoxy éthoxy imino/acétamido/ceph-3-ème 4-carboxylique isomère syn et 3,2 g de carbonate d'iodoéthyle et de méthyle préparé ci-dessous et obtient 1,12 g de produit attendu pur.

Spectre UV (EtOH, HCl N/10)
max.: 265 nm $E_1^1$=365 ε=19400

Spectre RMN ($CDCl_3$) ppm
1,53—1,63: $C\underline{H}_3$—CH
2,06: S—$CH_3$
3,83: $COOCH_3$
7,05: $H_5$ du thiazole
6,75 à 7,16: $C\underline{H}$—$CH_3$

Le carbonate d'iodoéthyle et de méthyle utilisé dans l'exemple a été préparé comme suit:

a) chloroformiate de 1-chloroéthyle

On fait barboter du chlorure dans 200 cm3 de chloroformiate d'éthyle pendant 8 heures, bouche hermétiquement et laisse 5 jours au repos. On concentre sous pression réduite (50 mm Hg) et recueille la fraction distillant entre 42° et 46°C.

b) carbonate de chloroéthyle et de méthyle

On mélange sous atmosphère inerte 5 cm3 de chloroformiate d'éthyle et 16 cm3 de méthanol et agite 1 heure à température ambiante.

c) carbonate d'iodoéthyle et de méthyle

On reprend le mélange par 38 cm3 d'acétone, ajoute 10,9 g d'iodure de sodium, porte 5 minutes à reflux puis laisse revenir à température ambiante. On distille sous pression réduite à une température inférieure à 40°C, reprend le résidu par 100 cm3 d'éther et 75 cm3 d'eau. On décante, extrait la phase aqueuse à l'éther, lave la phase organique à l'eau puis avec une solution aqueuse de métabisulfite de sodium (0,25 M) puis à l'eau et enfin avec une solution aqueuse saturée en chlorure de sodium. On sèche, concentre à sec sous pression réduite (température max. 40°C) et utilise le mélange immédiatement.

Exemple 32

3-méthylthiométhyl 7-/2-(2-amino thiazol-4-yl) 2-hydroxyimino acétamido/ceph-3-ème 4-carboxylate de 1-/(éthoxycarbonyl) oxy/éthyle isomère syn

On opère comme à l'exemple 9 à partir d'acide 3-méthylthiométhyl 7-/2-(2-tritylamino) thiazol-4-yl 2-/(1-méthyl 1-méthoxy) éthoxy imino/acétamido/ceph-3-ème 4-carboxylique isomère syn et de carbonate d'iodoéthyle et d'éthyle.

Le carbonate d'iodoéthyle et d'éthyle a été préparé suivant un mode opératoire identique à celui donné à l'exemple 31 pour la préparation du carbonate d'iodoéthyle et de méthyle.

Spectre RMN ($CDCl_3$) ppm

1,2 à 1,45: $CH_3$ du COO—Et.

1,55—1,64: $\underline{CH_3}$—CH—

6,9: $H_5$ du thiazole

2,08: S—$CH_3$

Exemple 33

3-méthylthiométhyl 7-/2-(2-amino thiazol-4-yl) 2-hydroxyimino acétamido/ceph-3-ème 4-carboxylate de/(méthoxy carbonyl) oxy/méthyle isomère syn

On opère comme à l'exemple 9 à partir de 5 g d'acide 3-méthylthiométhyl 7-/2-(2-tritylamino thiazol-4-yl) 2-(1-méthyl 1-méthoxy) éthoxy imino/acétamido/ceph-3-ème 4-carboxylique isomère syn et de 7,93 g de carbonate monoiodé préparé ci-dessous. On obtient 0,537 g de produit pur.

Spectre UV (EtOH, HCl N/10)

max.: 265 nm $E_1^1$=371 $\varepsilon$=19200

Spectre RMN ($CDCl_3$) ppm

2,03: S—$CH_3$

3,85: $CO_2CH_3$

6,96: $H_5$ du thiazole

5,91: $CO_2CH_2$

Le carbonate monoiodé a été préparé comme suit:

a) carbonate monochloré

Sous atmosphère inerte on chauffe à 65°C 30 cm3 de diméthyl carbonate et fait barboter du chlore pendant 5 heures, puis abandonne 56 heures au repos. On concentre sous pression réduire et recueille 7,8 cm3 de produit distillant entre 59° et 61°C (50 mm Hg).

b) carbonate monoiodé

On chauffe 1 heure 30 à 30°C 5 cm3 du produit obtenu précédemment, 7,8 g d'iodure de sodium et 30 cm3 d'acétone. On concentre à sec, reprend le résidu par 100 cm3 d'éther et 100 cm3 d'eau, agite 5 minutes, décante et extrait la phase aqueuse à l'éther. On lave la phase organique avec une solution aqueuse de métabisulfite de sodium (0,25M) puis à l'eau et enfin avec une solution aqueuse saturée en chlorure de sodium. On sèche et évapore à sec sous pression réduite (température maximum 40°C). On recueille 7,93 g de réactif que l'on utilise immédiatement.

Exemple 34

On a réalisé une préparation pour injection de formule:

| | |
|---|---|
| —Acide 3-méthoxyméthyl 7-/2-(2-amino thiazol-4-yl) 2-hydroxyimino acétamido/ceph-3-ème 4-carboxylique isomère *syn* | 500 mg |
| —excipient aqueux stérile q.s.p. | 5 cm3 |

Exemple 35

On a réalisé des gélules répondant à la formule:

| | |
|---|---|
| A—3-méthoxyméthyl 7-/2-(2-amino thiazol-4-yl) 2-hydroxyimino acétamido/ceph-3-ème 4-carboxylate de (1-oxopropoxy) méthyle isomère *syn* | 250 mg |
| —excipient q.s.p. une gélule terminée à | 400 mg |
| B—3-méthylthiométhyl 7-/2-(2-amino thiazol-4-yl) 2-hydroxyimino acétamido/ceph-3-ème 4-carboxylate de 1-oxopropoxyméthyle isomère *syn* | 250 mg |
| —excipient q.s.p. une gélule terminée à | 400 mg |
| C—3-méthylthiométhyl 7-/2-(2-amino thiazol-4-yl) 2-hydroxyimino acétamido/ceph-3-ème 4-carboxylate de 1-(acétyloxy) éthyl isomère syn | 250 mg |
| —excipient q.s.p. une gélule terminée à | 400 mg |
| D—3-méthylthiométhyl 7-/2-(2-amino thiazol-4-yl) 2-hydroxyimino acétamido ceph-3-ème 4-carboxylate de 1-(méthoxycarbonyl) oxy/éthyle isomère syn | 250 mg |
| —excipient q.s.p. une gélule terminée à | 400 mg |
| E—3-méthylthiométhyl 7-/2-(2-amino thiazol-4-yl) 2-hydroxyimino acétamido ceph-3-ème 4-carboxylate de 1-(éthoxycarbonyl) oxy/éthyle isomère syn | 250 mg |
| —excipient q.s.p. une gélule terminée à | 400 mg. |

Etude pharmacologique des produits de l'invention

Activité in vitro, méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre ou quarante-huit heures à l'étuve à 37°C, l'inhibition de la croissance est appréciée par transillumination ce qui permet de déterminer les concentrations minimales inhibitrices (C.M.I.) exprimées en $\mu$g/cm3.

Les résultats suivants sont obtenus:

Produit de l'exemple: 1

| SOUCHES | C.M.I. 24 h | en $\mu$g/ml 48 h |
|---|---|---|
| Staphylococcus aureus ATCC 6 538 Pen-Sensibles | 1 | 1 |
| Staphylococcus aureus UC 1 128 Pen-Résistant | 0,5 | 1 |
| Staphylococcus aureus exp. n° 54 146 | 0,5 | 2 |
| Streptococcus pyrogènes A 561 | 0,2 | 0,2 |
| Streptococcus faecalis 5 432 | 10 | 10 |
| Streptococcus faecalis 99 F74 | 10 | 20 |
| Bacillus subtilis ATCC 6 633 | 1 | 2 |
| Escherichia Coli Sensible Tétracycline ATCC 9 637 | 1 | 1 |
| Escherichia Coli Résistant Tétracycline ATCC 11 303 | 0,5 | 0,5 |
| Escherichia Coli Exp. $TO_{26}B_6$ | 0,5 | 1 |
| Escherichia Coli Résistant Gentamicine Tobramycine R 55 123 D | 1 | 1 |
| Klebsiella pneumoniae Exp. 52 145 | 0,2 | 0,5 |
| Klebsiella pneumoniae 2 536 Résistant Gentamycine | 0,5 | 1 |
| Proteus mirabilis (indol-) A 235 | 0,3 | 0,5 |
| Salmonella typhimurium 420 | 0,5 | 1 |

Produit de l'exemple: 2

| SOUCHES | C.M.I. 24 h | en $\mu$g/ml 48 h |
|---|---|---|
| Staphylococcus aureus ATCC 6 538 Pen-Sensibles | 3 | 5 |
| Staphylococcus aureus UC 1 128 Pen-Résistant | 5 | 10 |
| Staphylococcus aureus exp. n° 54 146 | 3 | 5 |
| Streptococcus pyrogènes A 561 | 0,3 | 0,5 |
| Bacillus subtilis ATCC 6 633 | 10 | 20 |
| Escherichia Coli Sensible Tétracycline ATCC 9 637 | 20 | 20 |
| Escherichia Coli Résistant Tétracycline ATCC 11 303 | 3 | 3 |
| Escherichia Coli Exp. $TO_{26}B_6$ | 2 | 3 |
| Escherichia Coli Résistant Gentamicine Tobramycine R 55 123 D | 5 | 10 |
| Klebsiella pneumoniae Exp. 52 145 | 3 | 3 |
| Klebsiella pneumoniae 2 536 Résistant Gentamycine | 20 | 20 |
| Proteus mirabilis (indol-) A 235 | 3 | 3 |
| Salmonella typhimurium 420 | 5 | 5 |

Produit de l'exemple: 3

| SOUCHES | C.M.I. 24 h | en $\mu$g/ml 48 h |
|---|---|---|
| Staphylococcus aureus ATCC 6 538 Pen-Sensibles | 0,2 | 0,5 |
| Staphylococcus aureus UC 1 128 Pen-Résistant | 0,5 | 0,5 |
| Staphylococcus aureus exp. n° 54 146 | 0,2 | 0,5 |
| Streptococcus pyrogènes A 561 | ⩽0,02 | ⩽0,02 |
| Streptococcus faecalis 5 432 | 5 | 10 |
| Streptococcus faecalis 99 F 74 | 3 | 10 |
| Bacillus subtilis ATCC 6 633 | 0,5 | 1 |
| Escherichia Coli Sensible Tétracycline ATCC 9 637 | 2 | 3 |
| Escherichia Coli Résistant Tétracycline ATCC 11 303 | 0,2 | 0,5 |
| Escherichia Coli Exp. $TO_{26}B_6$ | 0,5 | 0,5 |
| Escherichia Coli Résistant Gentamicine Tobramycine R 55 123 D | 1 | 1 |
| Klebsiella pneumoniae Exp. 52 145 | 0,2 | 0,2 |
| Klebsiella pneumoniae 2 536 Résistant Gentamycine | 2 | 3 |
| Proteus mirabilis (indol-) A 235 | 0,5 | 1 |
| Salmonella typhimurium 420 | 1 | 1 |

Produit de l'exemple 5

| SOUCHES | C.M.I. | en $\mu$g/ml |
|---|---|---|
| | 24 H | 48 H |
| Staphylococcus aureus ATCC 6 538 Pen-Sensibles | 0,2 | 0,5 |
| Staphylococcus aureus UC 1 128 Pen-Résistant. | 0,5 | 0,5 |
| Staphylococcus aureus exp. n° 54 146 | 0,2 | 0,5 |
| Streptococcus pyrogènes A 561 | $\leqslant$0,02 | $\leqslant$0,02 |
| Streptococcus faecalis 5 432 | 10 | 20 |
| Streptococcus faecalis 99 F 74 | 3 | 20 |
| Escherichia Coli Sensible Tétracycline 7624 | 3 | 3 |
| Escherichia Coli Résistant Tétracycline ATCC 11 303 | 1 | 1 |
| Escherichia Coli Exp. $TO_{26}B_6$ | 2 | 2 |
| Escherichia Coli Résistant Gentamicine, Tobramycine R 55 123 D | 5 | 5 |
| Klebsiella pneumoniae Exp. 52 145 | 1 | 1 |
| Klebsiella pneumoniae 2 536 Résistant Gentamycine | 10 | 10 |
| Proteus mirabilis (indol-) A 235 | 1 | 2 |
| Salmonella typhimurium 420 | 5 | 5 |

Produit de l'exemple 6

| SOUCHES | C.M.I. 24 H | en $\mu$g/ml 48 H |
|---|---|---|
| Staphylococcus aureus ATCC 6 538 Pen-Sensibles | 0,5 | 0,5 |
| Staphylococcus aureus UC 1 128 Pen-Résistant | 1 | 1 |
| Staphylococcus aureus exp. n° 54 146 | 0,5 | 1 |
| Streptococcus pyrogènes A 561 | 0,05 | 0,05 |
| Streptococcus faecalis 5 432 | 5 | 10 |
| Streptococcus faecalis 99 F 74 | 2 | 10 |
| Escherichia Coli Sensible Tétracycline 7624 | 1 | 1 |
| Escherichia Coli Résistant Tétracycline ATCC 11 303 | 0,5 | 0,5 |
| Escherichia Coli Exp. $TO_{26}B_6$ | 0,5 | 1 |
| Escherichia Coli Résistant Gentamicine, Tobramycine R 55 123 D | 1 | 1 |
| Klebsiella pneumoniae Exp. 52 145 | 0,5 | 0,5 |
| Klebsiella pneumoniae 2 536 Résistant Gentamycine | 2 | 3 |
| Proteus mirabilis (indol-) A 235 | 0,5 | 1 |
| Salmonella typhimurium 420 | 1 | 1 |

2°) Activite antibiotique in vivo per os chez la souris

On utilise des souris mâles (CDI Charles River) d'un poids moyen de 21 à 22 g.

L'infection a été réalisée par voie intra-péritonéale avec 0,5 ml d'une culture de 22 heures d'une souche de Staphylococcus aureus n° 54 146 (Antibiotic Medium 3 à PH 7,0) diluée au 1/6 en eau physiologique.

Les produits ont été administrés à diverses doses sous 0,5 ml d'eau distillée, per os, par intubation, à des lots de 10 animaux 1 h, 5 h et 24 h après l'injection infestante.

On obtient les résultats suivants.

| Produit | Dose ×3 Per os | Souris survivantes au 10° Jour |
|---|---|---|
| Exemple 4 | 0,1 | 2 |
| | 0,25 | 9 |
| | 0,5 | 10 |
| Exemple 12 | 0,1 | 9 |
| | 0,25 | 10 |
| | 0,5 | 10 |
| Exemple 18 | 0,1 | 1 |
| | 0,25 | 7 |
| | 0,5 | 9 |
| Exemple 19 | 0,1 | 0 |
| | 0,25 | 5 |
| | 0,5 | 9 |
| Exemple 20 | 0,1 | 0 |
| | 0,25 | 7 |
| | 0,5 | 10 |

**Revendications pour les Etats Contractants BE CH DE GB IT LI LU NL SE**

1. Les produits de formule générale (I'):

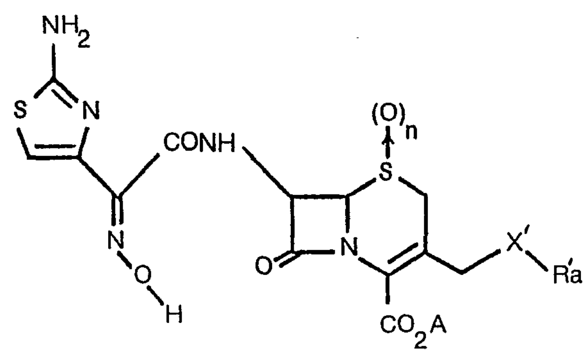

Isomères *syn*

(I').

dans laquelle

A représente un atome d'hydrogène, un équivalent de métal alcalin, alcalino-terreux, de magnésium, d'ammonium ou d'une base organique aminée, ou A représente le reste d'un groupement ester facilement clivable,

R'a représente un radical alkyle éventuellement interrompu par un hétéroatome, alkényle ou alkynyle ayant au plus 6 atomes de carbone linéaire ou ramifié, ou un radical benzyle ou phényléthyle, éventuellement substitué par un radical carboxy, amino, aminoalkyle, alkylamino, dialkylamino, dialkylaminoalkyle.

n est égal à 0, 1 ou 2,

X' représente un atome de soufre éventuellement oxydé sous forme de sulfoxyde ou de sulfone, ou un atome d'oxygène,

ainsi que les sels des produits de formule (I') avec les acides minéraux ou organiques.

2. Les produits selon la revendication 1, répondant à la formule générale (I)

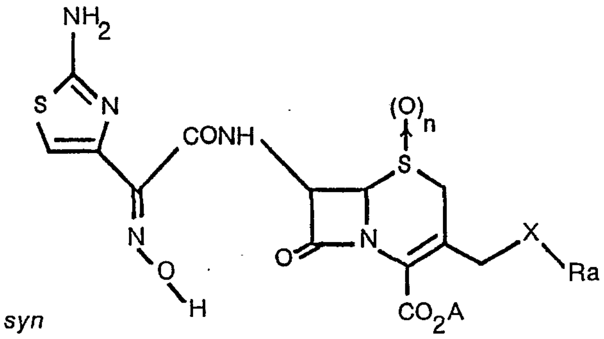

(I)

isomères *syn*

dans laquelle A et n sont définis comme à la revendication 1, Ra représente un radical alkyle, alkényle ou alkynyle ayant au plus 6 atomes de carbone linéaire ou ramifié, ou un radical benzyle ou phényléthyle, éventuellement substitué par un radical carboxy, amino aminoalkyle, alkylamino, dialkylamino, dialkylaminoalkyle, et X représente un atome de soufre ou un atome d'oxygène, ainsi que les sels des produits de formule (I) avec les acides minéraux ou organiques.

3. Les produits de formule générale (I) telle que définie à la revendication 2, dans laquelle Ra représente un radical alkyle ayant au plus 6 atomes de carbone.

4. Les produits de formule générale (I) telle que définie à la revendication 2 ou 3, dans laquelle Ra représente un radical méthyle et n représente le nombre 0.

5. Les produits selon la revendication 2, répondant à la formule générale ($I_A$):

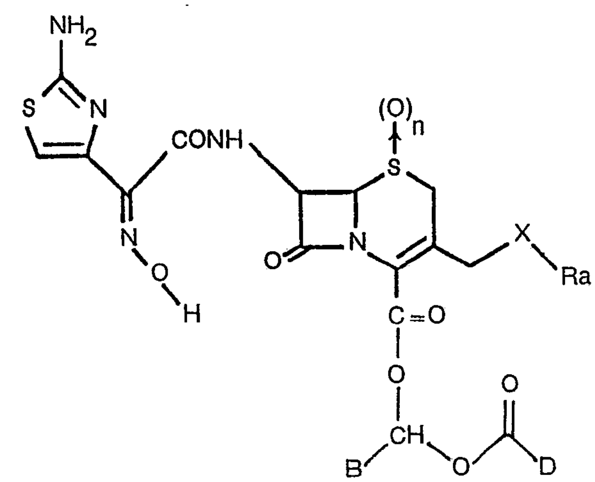

($I_A$)

dans laquelle n, X et Ra sont définis comme à la revendication 2, B représente un atome d'hydrogène ou un radical alkyle linéaire ou ramifié, renfermant de 1 à 5 atomes de carbone et D représente un radical alkyle ou alkoxy linéaire ou ramifié, renfermant de 1 à 15 atomes de carbone ainsi que leurs sels avec les acides minéraux ou organiques.

6. Les produits selon la revendication 5, caractérisé en ce que D représente un radical alkyle ou alkoxy, linéaire ou ramifié, renfermant de 1 à 5 atomes de carbone.

7. L'acide 3-méthoxyméthyl 7-//2-(2-amino thiazol-4-yl) 2-hydroxyimino acétyl/amino/ceph-3-ème 4-carboxylique, ainsi que ses sels avec les métaux alcalins, alcalino-terreux, le magnésium, l'ammoniaque, les bases organiques aminées, les acides et ses esters facilement clivables.

8. Le 3-méthoxyméthyl 7-//2-(2-amino thiazol-4-yl) 2-hydroxyimino acétyl/amino/ceph-3-ème 4-carboxylate de 1-acétyloxyéthyle, ainsi que ses sels avec les acides minéraux ou organiques.

9. Procédé de préparation des produits de formule (I') telle que définie à la revendication 1, caractérisé en ce que l'on traite un produit de formule (II):

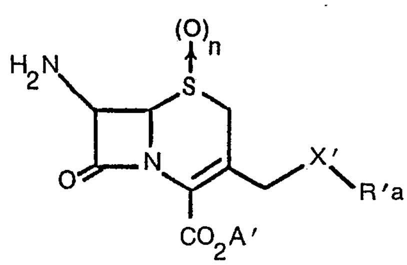

(II)

dans laquelle n, X′ et R′a ont la signification indiquée à la revendication 1 et A′ représente un atome d'hydrogène ou le reste d'un groupement ester facilement éliminable, par un produit de formule (III)

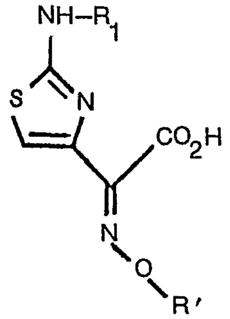

(III)

ou un dérivé fonctionnel de cet acide, formule (III) dans laquelle $R_1$ représente un atome d'hydrogène ou un groupement protecteur du radical amino et R′ représente un atome d'hydrogène ou un groupement protecteur du radical hydroxyle, pour obtenir un produit de formule (IV):

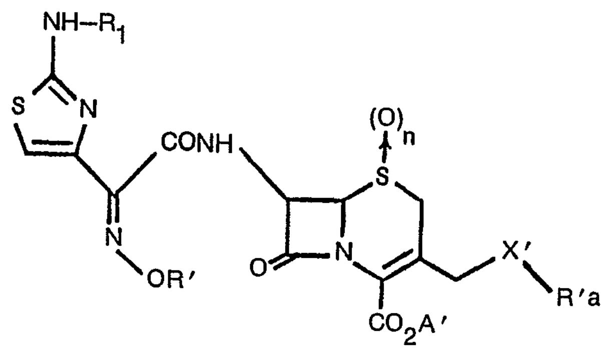

(IV)

dans laquelle $R_1$, R′, A′, R′a, X′ et n ont la signification précédente, produit que, si désiré, dans le cas où n est égal à 0 et X′ représente un atome de soufre ou d'oxygène, l'on traite par un agent d'oxydation, pour obtenir un produit de formule (IV) dans laquelle n'est égal à 1 ou 2 et X′ représente un atome de soufre oxydé sous forme de sulfoxyde ou de sulfone ou un atome d'oxygène et, produit de formule IV que l'on soumet si nécessaire ou si désiré à l'une ou plusieurs des réactions suivantes, dans un ordre quelconque:

a) coupure par hydrolyse, hydrogénolyse ou par action de la thiourée du groupement ester ou du groupement de protection du radical amino,
b) estérification ou salification par une base du radical carboxylique,
c) salification par un acide du radical amino.

10. Procédé de préparation des produits de formule (I′) telle que définie à la revendication 1, caractérisé en ce que l'on traite un produit de formule (V):

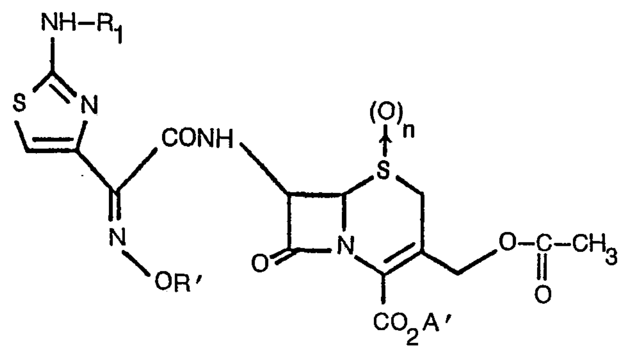

(V)

dans laquelle $R_1$, R′ et A′ ont la signification indiquée à la revendication 9 et n a la signification indiquée à la revendication 1, *ou bien* par un produit de formule R′a—SH dans laquelle R′a a la signification indiquée à la revendication 1, *ou bien* d'abord par le 2-mercapto pyridine N-oxyde puis par un produit de formule R′aOH dans laquelle R′a a la signification précédente, pour obtenir un produit de formule (IV), tel que défini précédemment, produit de formule IV que, si désiré, dans le cas où n est égal à 0 et X′ représente un atome de soufre ou d'oxygène, l'on traite par un agent d'oxydation, pour obtenir un produit de formule (IV) dans laquelle n est égal à 1 ou 2 et X′ représente un atome de soufre oxydé sous

forme de sulfoxyde ou de sulfone ou un atome d'oxygène et, produit de formule IV que l'on soumet si nécessaire ou si désiré, à l'une ou plusieurs des réactions suivantes, dans un ordre quelconque:

a) coupure par hydrolyse, hydrogénolyse ou par action de la thiourée du groupement ester ou des groupements de protection du radical amino ou du radical hydroxyle,
b) estérification ou salification par une base du radical carboxylique,
c) salification par un acide du radical amino.

11. Procédé selon la revendication 9 de préparation des produits de formule (I'), caractérisé en ce que l'on utilise, pour la mise en oeuvre du procédé, un produit de formule (III) dans laquelle $R_1$ représente un groupement protecteur du radical amino et en ce que le dérivé fonctionnel de l'acide de formule (III) est un anhydride mixte carboxylique sulfonique.

12. Procédé de préparation des produits de formule (I'), telle que définie à la revendication 1, caractérisé en ce que l'on traite un produit de formule (II'):

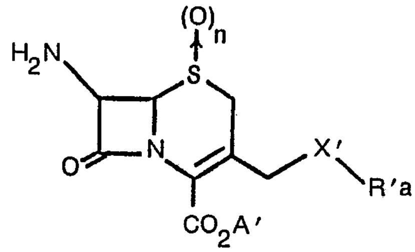

(II')

dans laquelle R'a, n, X' et A' ont la signification indiquée à la revendication 1, par un produit de formule (III'):

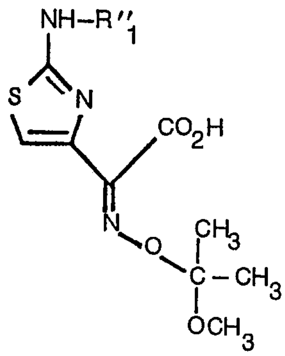

(III')

ou un dérivé fonctionnel de cet acide, formule (III') dans laquelle $R''_1$ représente un groupement protecteur du radical amino, pour obtenir un produit de formule (IV'):

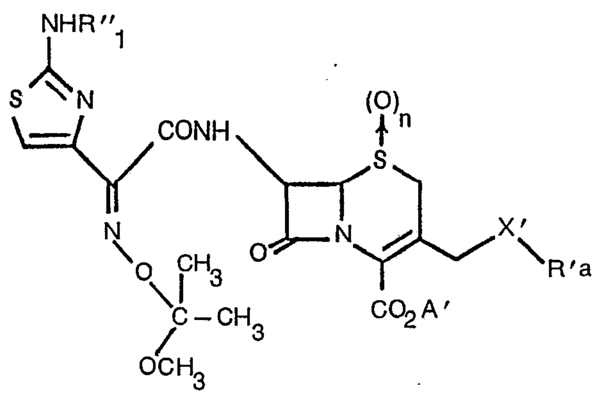

(IV')

dans laquelle A', $R''_1$, R'a, X' et n ont la signification précédente, produit de formule (IV') que l'on traite par un acide dans des conditions modérées, pour obtenir un produit de formule VI:

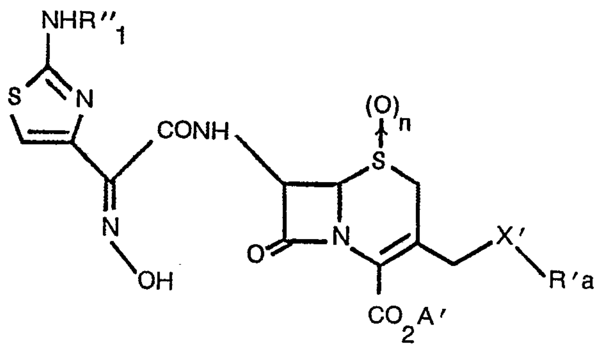

(VI)

produit que, si désiré, l'on estérifie ou salifie et produit de formule VI que l'on soumet à une hydrolyse, hydrogénolyse ou à l'action de la thiourée pour éliminer le radical $R''_1$ de protection du radical amino et, si désiré, ou si nécessaire, à l'une ou plusieurs des réactions suivantes, dans un ordre quelconque:

a) élimination du groupement ester,
b) estérification ou salification par une base du radical carboxylique,
c) salification par un acide du radical amino.

13. A titre de médicaments, les produits répondant à la formule (I') telle que définie à la revendication 1, ainsi que leurs sels d'acides, pharmaceutiquement acceptables.

14. A titre de médicaments, les produits de formule (I) telle que définie à l'une quelconque des revendications 2, 3 et 4 ainsi que leurs sels d'acides, pharmaceutiquement acceptables.

15. A titre de médicaments, les produits tels que définis à la revendication 5 ou 6, ainsi que leurs sels d'acides, pharmaceutiquement acceptables.

16. A titre de médicaments, les produits tels que définis à la revendication 7 ou 8, ainsi que leurs sels pharmaceutiquement acceptables.

17. Compositions pharmaceutiques contenant, à titre de principe actif, ou moins un des médicaments selon l'une des revendications 13 à 16.

**Revendications pour l'Etat Contractant AT**

1. Procédé pour préparer les produits de formule générale (I')

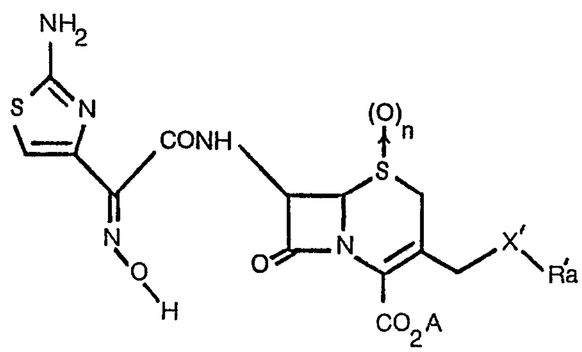

Isomères *syn*

(I')

dans laquelle

A représente un atome d'hydrogène, un équivalent de métal alcalin, alcalino-terreux, de magnésium, d'ammonium ou d'une base organique aminée, ou A représente le reste d'un groupement ester facilement clivable,

R'a représente un radical alkyle éventuellement interrompu par un hétéroatome, alkényle ou alkynyle ayant au plus 6 atomes de carbone linéaire ou ramifié, ou un radical benzyle ou phényléthyle, éventuellement substitué par un radical carboxy, amino, aminoalkyle, alkylamino, dialkylamino, dialkylaminoalkyle.

n est égal à 0, 1 ou 2,

X' représente un atome de soufre éventuellement oxydé sous forme de sulfoxyde ou de sulfone, ou un atome d'oxygène, ainsi que les sels des produits de formule (I') avec les acides minéraux ou organiques, caractérisé en ce que l'on traite un produit de formule (II)

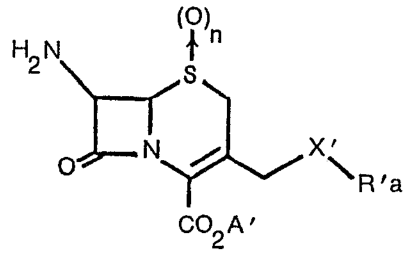

(II)

dans laquelle n, X' et R'a ont la signification indiquée, précédemment et A' représente un atome d'hydrogène ou le reste d'un groupement ester facilement éliminable, par un produit de formule (III):

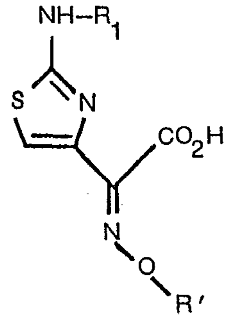

(III)

ou un dérivé fonctionnel de cet acide, formule (III) dans laquelle $R_1$ représente un atome d'hydrogène ou un groupement protecteur du radical amino et R' représente un atome d'hydrogène, ou un groupement protecteur du radical hydroxyle, pour obtenir un produit de formule (IV):

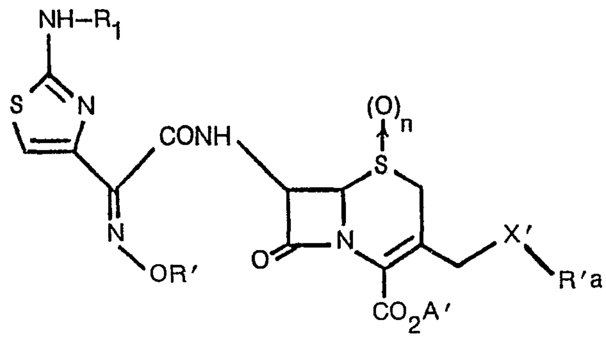

(IV)

dans laquelle $R_1$, R', A', R'a, X' et n ont la signification précédente, produit que, si désiré dans le cas où n est égal à O et X' représente un atome de soufre ou d'oxygène, l'on traite par un agent d'oxydation, pour obtenir un produit de formule (IV) dans laquelle n est égal à 1 ou 2 et X' représente un atome de soufre oxydé sous forme de sulfoxyde ou de sulfone ou un atome d'oxygène et, produit de formule IV que l'on soumet si nécessaire ou si désiré à l'une ou plusieurs des réactions suivantes, dans ou ordre quelconque:

a) coupure par hydrolyse, hydrogènolyse ou par action de la thiourée du groupement ester ou des groupements de protection du ou des radicaux amino,
b) estérification ou salification par une base du ou des radicaux carboxyliques,
c) salification par un acide du ou des radicaux amino.

2. Procédé de préparation des produits de formule (I') telle que définie à la revendication 1, caractérisé en ce que l'on traite un produit de formule (V):

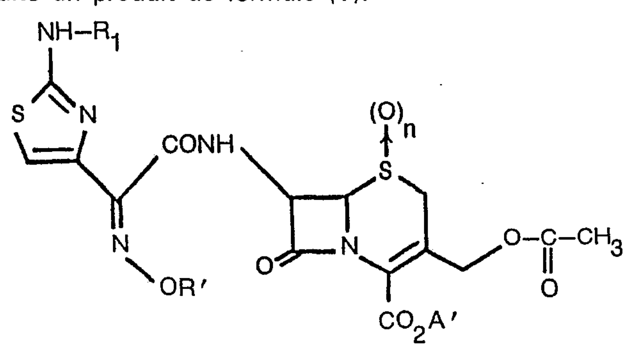

(V)

dans laquelle $R_1$, R', A' et n ont la signification indiquée à la revendication 1, *ou bien* par un produit de formule R'a—SH dans laquelle R'a a la signification indiquée à la revendication 1, *ou bien* d'abord par le 2-mercapto pyridine N-oxyde puis par un produit de formule R'aOH dans laquelle R'a a la signification précédente, pour obtenir un produit de formule (IV), tel que défini précédemment, produit de formule IV que, si désiré, dans le cas où n est égal à O et X' représente un atome de soufre ou d'oxygène, l'on traite par un agent d'oxydation, pour obtenir un produit de formule (IV) dans laquelle n est égal à 1 ou 2 et X' représente un atome de soufre oxydé sous forme de sulfoxyde ou de sulfone ou un atome d'oxygène et, produit de formule IV que l'on soumet si nécessaire ou si désiré, à l'une ou plusieurs des réactions suivantes, dans un ordre quelconque:

a) coupure par hydrolyse, hydrogénolyse ou par action de la thiourée du groupement ester ou des groupements de protection du radical amino ou du radical hydroxyle,
b) estérification ou salification par une base du radical carboxylique,
c) salification par un acide du radical amino.

3. Procédé selon la revendication 1 de préparation des produits de formule (I'), caractérisé en ce que l'on utilise, pour la mise en oeuvre du procédé, un produit de formule (III) dans laquelle $R_1$ représente un groupement protecteur du radical amino et en ce que le dérivé fonctionnel de l'acide de formule (III) est un anhydride mixte carboxylique sulfonique.

4. Procédé selon l'une quelconque des revendications 1, 2 et 3, pour préparer les produits selon la revendication 1, répondant à la formule générale (I):

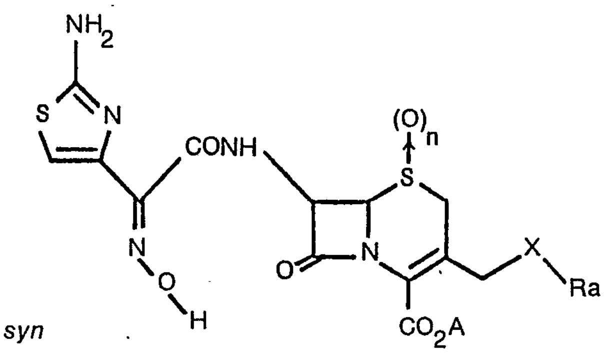

isomères *syn*

(I)

dans laquelle A et n sont définis comme à la revendication 1, Ra représente un radical alkyle; alkényle ou alkynyle ayant au plus 6 atomes de carbone linéaire ou ramifié, ou un radical benzyle ou phényléthyle éventuellement substitué par un radical carboxy, amino, aminoalkyle, alkylamino, dialkylamino, dialkylamino alkyle et X représente un atome de soufre ou un atome d'oxygène, ainsi que les sels des produits de formule (I) avec les acides minéraux ou organiques, caractérisé en ce que l'on utilise au départ des produits de formule (II), R'aSH et R'aOH dans lesquelles X' et R'a ont les valeurs précédemment indiquées pour X et Ra.

5. Procédé de préparation des produits de formule (I'), telle que définie à la revendication 1, caractérisé en ce que l'on traite on produit de formule (II'):

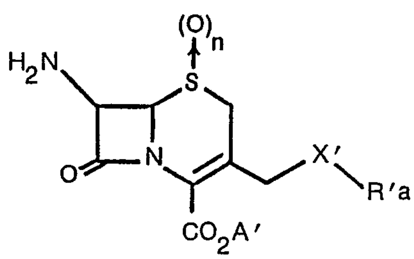

(II')

dans laquelle R'a, n, X' et A' ont la signification indiquée à la revendication 1, par un produit de formule (III'):

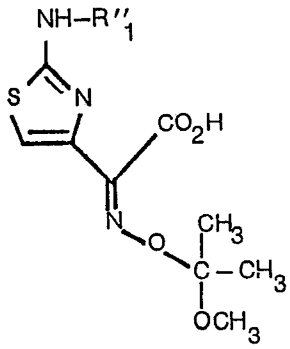

(III')

ou un dérivé fonctionnel de cet acide, formule (III') dans laquelle $R''_1$ représente un groupement protecteur du radical amino, pour obtenir un produit de formule (IV'):

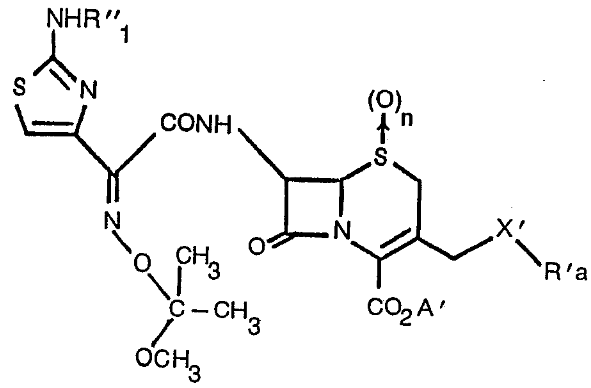

(IV')

dans laquelle A', $R''_1$, R'a, X' et n ont la signification précédente, produit de formule (IV') que l'on traite par un acide dans des conditions modérées, pour obtenir un produit de formule VI:

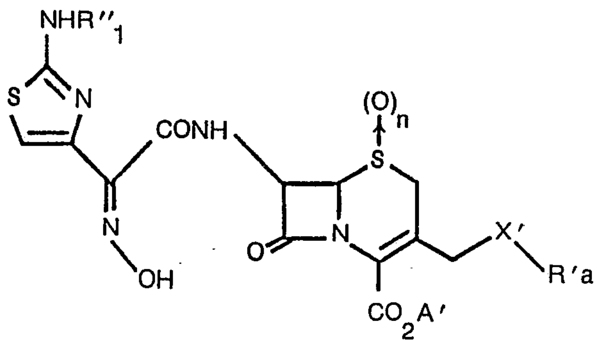

(VI)

produit que, si désiré, l'on estérifie ou salifie et produit de formule VI que l'on soumet à une hydrolyse, hydrogénolyse ou à l'action de la thiourée pour éliminer le radical $R''_1$ de protection du radical amino et, si désiré, ou si nécessaire, à l'une ou plusieurs des réactions suivantes, dans un ordre quelconque:

a) élimination du groupement ester,
b) estérification ou salification par une base du radical carboxylique,
c) salification par un acide du radical amino.

6. Procédé selon la revendication 4, caractérisé en ce que dans le produit de formule (III), R' représente un atome d'hydrogène, un groupement protecteur du radical hydroxyle, et en ce que dans le produit de formule (II) ou dans le produit de formule R'aSH ou R'aOH, R'a représente un radical alkyle ayant au plus 6 atomes de carbone.

7. Procédé selon la revendication 5, caractérisé en ce que R'a représente un radical méthyle et en ce que dans le produit de formule (II'), n est égal à 0.

8. Procédé selon la revendication 4, caractérisé en ce que l'estérification finale éventuelle est effectuée par un dérive fonctionnel du groupement de formule

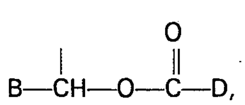

dans lequel B représente un atome d'hydrogène ou un radical alcoyle linéaire ou ramifié, renfermant de 1 à 5 atomes de carbone et D représente un radical alcoyle ou alkoxy linéaire ou ramifié, renfermant de 1 à 15 atomes de carbone.

9. Procédé selon la revendication 8, caractérisé en ce que D représente un radical alcoyle ou alkoxy, linéaire ou ramifié, renfermant de 1 à 5 atomes de carbone.

10. Procédé de préparation de produits de formule (I'), tels qu'obtenus à l'une quelconque des revendications 6 à 9, caractérisé en ce que l'on opère selon le procédé de la revendication 5.

11. Procédé selon la revendication 4 ou 10, caractérisé en ce que l'on prépare le produit répondant à la formule (I) suivante:

— l'acide 3-méthoxyméthyl 7-//2-(2-amino thiazol-4-yl) 2-hydroxyimino acétyl/amino/ceph-3-ème 4-carboxylique, ainsi que ses sels avec les métaux alcalins, alcalino-terreux, le magnésium, l'ammoniaque, les bases organiques aminées, les acides et ses esters facilement clivables.

12. Procédé selon la revendication 4 ou 10, caractérisé en ce que l'on prépare le produit répondant à la formule (I) suivante:

— le 3-méthoxyméthyl 7-//2-(2-amino thiazol-4-yl) 2-hydroxyimino acétyl/amino/ceph-3-ème 4-carboxylate de 1-acétyloxyéthyle, ainsi que ses sels avec les acides minéraux ou organiques.

**Patentansprüche für die Vertragsstaaten: BE, CH, DE, GB, IT, LI, LU, NL, SE**

1. Produkte der allgemeinen Formel (I')

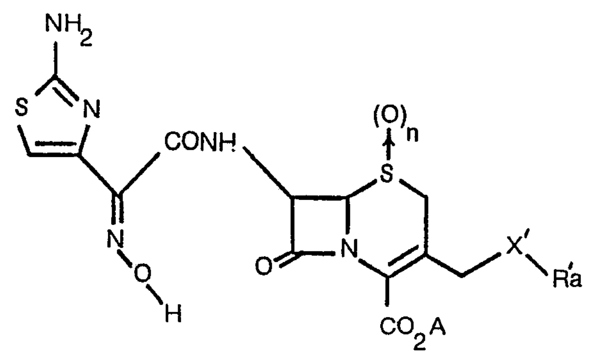

syn-Isomere

(I')

worin

A ein Wasserstoffatom, ein Äquivalent eines Alkali- oder Erdalkalimetalls, von Magnesium, Ammonium oder einer organischen Stickstoffbase bedeutet oder A den Rest einer leicht spaltbaren Estergruppe darstellt,

R'a einen linearen oder verzweigten Alkylrest, der gegebenenfalls durch ein Heteroatom unterbrochen ist, Alkenyl- oder Alkinylrest mit jeweils höchstens 6 Kohlenstoffatomen oder einen Benzyl- oder Phenethylrest, der gegebenenfalls durch einen Carboxy-, Amino-, Aminoalkyl-, Alkylamino-, Dialkylamino-, Dialkylaminoalkylrest substituiert ist, bedeutet,

n für 0, 1 oder 2 steht,

X' ein gegebenenfalls in Form des Sulfoxids oder Sulfons oxidiertes Schwefelatom oder ein Sauerstoffatom bedeutet, sowie die Salze der Produkte der Formel (I') mit Mineraloder organischen Säuren.

2. Produkte gemäß Anspruch 1 der allgemeinen Formel (I)

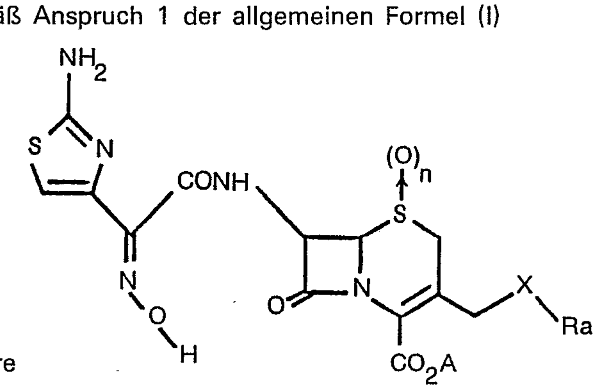

syn-Isomere

(I)

worin A und n wie in Anspruch 1 definiert sind, Ra einen linearen oder verzweigten Alkyl-, Alkenyl- oder Alkinyl- rest mit höchstens 6 Kohlenstoffatomen oder einen Benzyl- oder Phenethylrest, der gegebenenfalls durch einen Carboxy- Amino-, Aminoalkyl-, Alkylamino-, Dialkylamino- oder Dialkylaminoalkylrest substituiert ist, bedeutet und X ein Schwefelatom oder ein Sauerstoffatom darstellt, sowie die Salze der Produkte der Formel (I) mit Mineral- oder organischen Säuren.

3. Produkte der Formel (I) gemäß Anspruch 2, worin Ra einen Alkylrest mit höchstens 6 Kohlenstoffatomen bedeutet.

4. Produkte der Formel (I) gemäß Anspruch 2 oder 3, worin Ra einen Methylrest bedeutet und n die Zahl O derstellt.

5. Produkte gemäß Anspruch 2 der allgemeinen Formel ($I_A$)

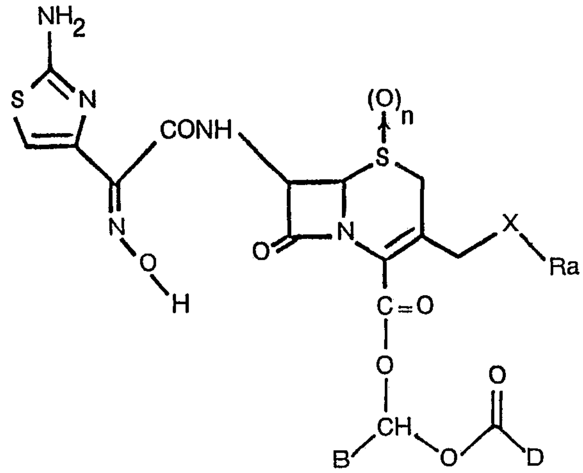

($I_A$)

worin n, X und Ra wie in Anspruch 2 definiert sind, B ein Wasserstoffatom oder einen linearen oder verzweigten Alkylrest mit 1 bis 5 Kohlenstoffatomen bedeutet und D einen linearen oder verzweigten Alkyl- oder Alkoxyrest mit 1 bis 15 Kohlenstoffatomen darstellt, sowie deren Salze mit Mineral- oder organischen Säuren.

6. Produkte gemäß Anspruch 5, dadurch gekennzeichnet, daß D einen linearen oder verzweigten Alkyl- oder Alkoxyrest mit 1 bis 5 Kohlenstoffatomen bedeutet.

7. 3-Methoxymethyl-7-[[2-(2-aminothiazol-4-yl)-2-hydroxyiminoacetyl]-amino]-ceph-3-em-4-carbonsäure sowie ihre Salze mit Alkali- oder Erdalkalimetallen, Magnesium, Ammoniak, organischen Stickstoffbasen, Säuren und ihren leicht spaltbaren Estern.

8. 1-Acetyloxyethyl-3-methoxymethyl-7-[[2-(2-aminothiazol-4-yl)-2-hydroxyiminoacetyl]-amino]-ceph-3-em-4-carboxylat sowie seine Salze mit Mineral- oder organischen Säuren.

9. Verfahren zur Herstellung der Produkte der Formel (I') gemäß Anspruch 1, dadurch gekennzeichnet, daß man ein Produkt der Formel (II).

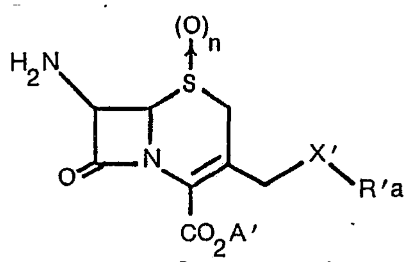

(II)

worin n, X' und R'a die in Anspruch 1 angegebene Bedeutung besitzen und A' ein Wasserstoffatom oder den Rest einer leicht eliminierbaren Estergruppe bedeutet, mit einem Produkt der Formel (III)

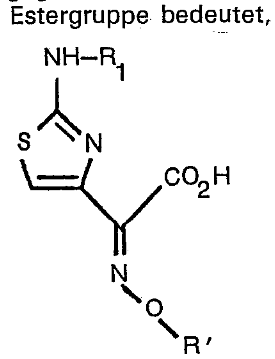

(III)

oder einem funktionellen Derivat dieser Säure umsetzt, wobei in der Formel (III) $R_1$ ein Wasserstoffatom oder eine Aminoschutzgruppe bedeutet und R' ein Wasserstoffatom oder eine Schutzgruppe für den Hydroxylrest darstellt, um ein Produkt der Formel (IV)

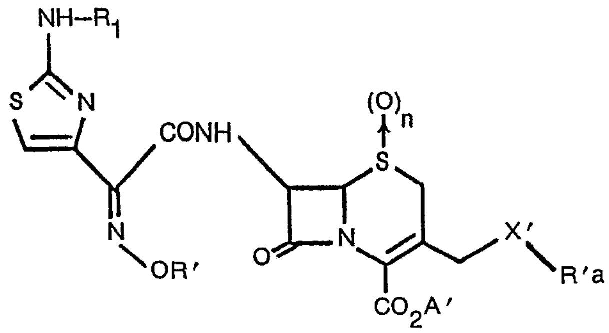

(IV)

worin $R_1$, R', A', R'a, X' und n die vorstehende Bedeutung besitzen, zu erhalten, welches Produkt man gewünschtenfalls, wenn n für 0 steht und X' ein Schwefel- oder Sauerstoffatom bedeutet, mit einem Oxidationsmittel behandelt, um ein Produkt der Formel (IV) zu erhalten, worin n für 1 oder 2 steht und X' ein in Form des Sulfoxids oder Sulfons oxidiertes Schwefelatom oder ein Sauerstoffatom bedeutet, und das Produkt der Formel (IV) nötigen- oder gewünschtenfalls einer oder mehreren der folgenden Reaktionen in beliebiger Reihenfolge unterzieht:

(a) Spaltung durch Hydrolyse, Hydrogenolyse oder durch Umsetzung mit Thioharnstoff der Estergruppe oder der Amino-Schutzgruppe,
(b) Veresterung der Carboxylgruppe oder ihre Überführung in ein Salz mit einer Base,
(c) Überführung der Aminogruppe in ein Salz mit einer Säure.

10. Verfahren zur Herstellung der Produkte der Formel (I') gemäß Anspruch 1, dadurch gekennzeichnet, daß man ein Produkt der Formel (V)

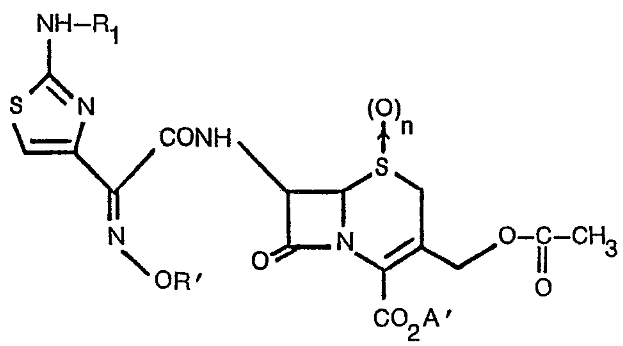

(V)

worin $R_1$, R' und A' die in Anspruch 9 angegebene Bedeutung besitzen und n die in Anspruch 1 angegebene Bedeutung besitzt, *entweder* mit einem Produkt der Formel R'a—SH, worin R'a die in Anspruch 1 angegebene Bedeutung besitzt, *oder* zunächst mit 2-Mercaptopyridin-N-oxid, danach mit einem Produkt der Formel R'aOH, worin R'a die vorstehende Bedeutung besitzt, behandelt, um ein Produkt der Formel (IV), wie vorstehend definiert, zu erhalten, das Produkt der Formel (IV) gewünschtenfalls, wenn n für 0 steht und X' ein Schwefel- oder Sauerstoffatom bedeutet, mit einem Oxidationsmittel behandelt, um ein Produkt der Formel (IV) zu erhalten, worin n für 1 oder 2 steht und X' ein in Form des Sulfoxids oder Sulfons oxidiertes Schwefelatom oder ein Sauerstoffatom bedeutet, und das Produkt der Formel (IV) nötigen- oder gewünschtenfalls einer oder mehreren der folgenden Reaktionen in beliebiger Reihenfolge unterzieht:

(a) einer Spaltung durch Hydrolyse, Hydrogenolyse oder durch Umsetzung mit Thioharnstoff der Estergruppe oder der Amino- oder Hydroxyl-Schutzgruppen,
(b) Veresterung des Carboxylrestes oder seine Überführung in ein Salz mit einer Base,
(c) Überführung des Aminorestes in ein Salz durch eine Säure.

11. Verfahren gemäß Anspruch 9 zur Herstellung der Produkte der Formel (I'), dadurch gekennzeichnet, daß man für die Durchführung des Verfahrens ein Produkt der Formel (III) verwendet, worin $R_1$ eine Amino-Schutzgruppe bedeutet, und daß das funktionelle Derivat der Säure der Formel (III) ein gemischtes Carbonsäure-Sulfonsäureanhydrid ist.

12. Verfahren zur Herstellung der Produkte der Formel (I') gemäß Anspruch 1, dadurch gekennzeichnet, daß man ein Produkt der Formel (II')

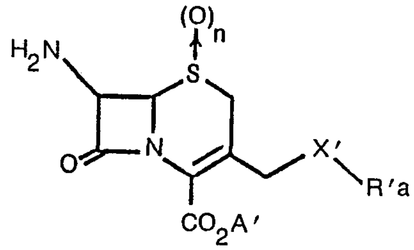

(II')

worin R'a, n, X' und A' die in Anspruch 1 angegebene Bedeutung besitzen, mit einem Produkt der Formel (III')

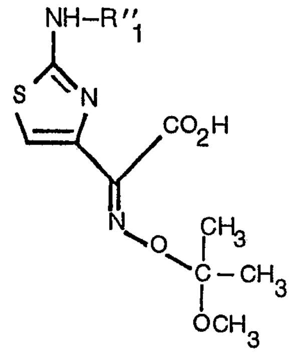

(III')

oder einem funktionellen Derivat dieser Säure behandelt, wobei in der Formel (III') $R''_1$ eine Amino-Schutzgruppe bedeutet, um ein Produkt der Formel (IV')

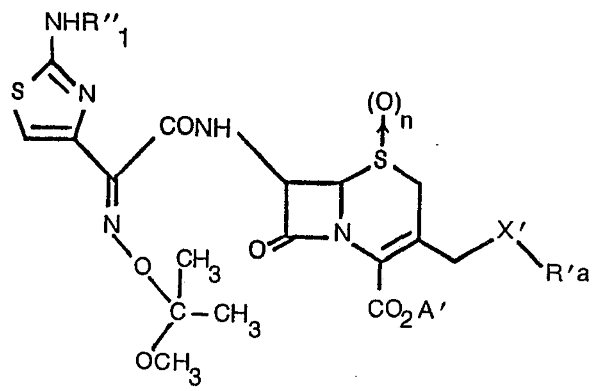

(IV')

zu erhalten, worin A', $R''_1$, R'a, X' und n die vorstehende Bedeutung besitzen, das Produkt der Formel (IV') unter mäßigen Bedingungen mit einer Säure behandelt, um ein Produkt der Formel (VI)

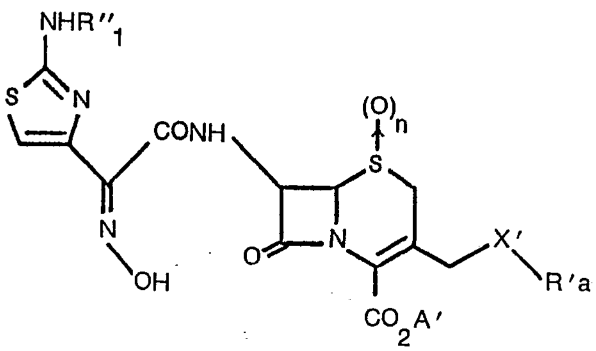

(VI)

zu erhalten, welches Produkt man gewünschtenfalls verestert oder in ein Salz überführt und das Produkt der Formel (VI) einer Hydrolyse, Hydrogenolyse oder Umsetzung mit Thioharnstoff unterzieht, um die Amino-Schutzgruppe $R''_1$ zu eliminieren, und gewünschten- oder erforderlichenfalls einer oder mehreren der folgenden Reaktionen in beliebiger Reihenfolge unterzieht:

(a) Eliminierung der Estergruppe,
(b) Veresterung der Carboxylgruppe oder ihre Überführung in ein Salz mit einer Base,
(c) Überführung des Aminorestes in ein Salz mit einer Säure.

13. Als Arzneimittel die Produkte der Formel (I') gemäß Anspruch 1 sowie deren pharmazeutisch verträgliche Salze mit Säuren.

14. Als Arzneimittel die Produkte der Formel (I) gemäß einem der Ansprüche 2, 3 und 4 sowie deren pharmazeutisch verträgliche Salze mit Säuren.

15. Als Arzneimittel die Produkte gemäß Anspruch 5 oder 6 sowie deren pharmazeutisch verträgliche Salze mit Säuren.

16. Als Arzneimittel die Produkte gemäß Anspruch 7 oder 8 sowie deren pharmazeutisch verträgliche Salze.

17. Pharmazeutische Zusammensetzungen, enthaltend als Wirkstoff zumindest eines der Arzneimittel gemäß einem der Ansprüche 13 bis 16.

**Patentansprüche für den Vertragsstaat: AT**

1. Verfahren zur Herstellung der Produkte der allgemeinen Formel (I')

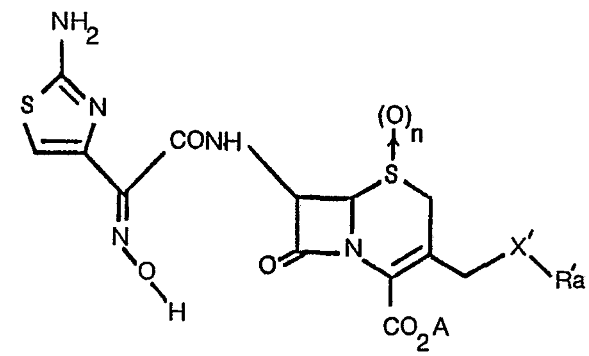

(I')

syn-Isomere

worin

A ein Wasserstoffatom, ein Äquivalent eines Alkali- oder Erdalkalimetalls, von Magnesium, Ammonium oder einer organischen Stickstoffbase bedeutet oder A den Rest einer leicht spaltbaren Estergruppe darstellt,

R'a einen linearen oder verzweigten, gegebenenfalls durch ein Heteroatom unterbrochenen Alkylrest, Alkenyl- oder Alkinylrest mit höchstens 6 Kohlenstoffatomen oder einen Benzyl- oder Phenethylrest, der gegebenenfalls durch einen Carboxy-, Amino-, Aminoalkyl-, Alkylamino-, Dialkylamino- oder Dialkylaminoalkylrest substituiert ist, bedeutet,

n für 0, 1 oder 2 steht,

X' ein gegebenenfalls in Form des Sulfoxids oder Sulfons oxidiertes Schwefelatom oder ein Sauerstoffatom bedeutet, sowie die Salze der Produkte der Formel (I') mit Mineraloder organischen Säuren, dadurch gekennzeichnet, daß man ein Produkt der Formel (II)

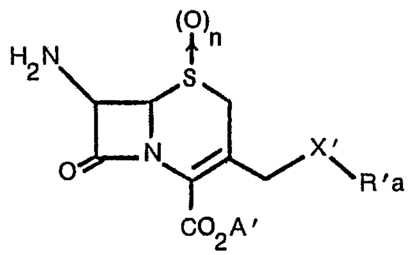

(II)

worin n, X' und R'a die vorstehend angegebene Bedeutung besitzen und A' ein Wasserstoffatom oder den Rest einer leicht eliminierbaren Estergruppe bedeutet, mit einem Produkt der Formel (III)

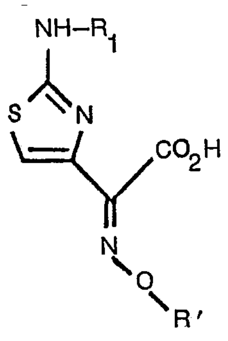

(III)

oder einem funktionellen Derivat dieser Säure behandelt, wobei in der Formel (III) $R_1$ ein Wasserstoffatom oder eine Amino-Schutzgruppe bedeutet und R' ein Wasserstoffatom oder eine Hydroxyl-Schutzgruppe darstellt, um ein Produkt der Formel (IV)

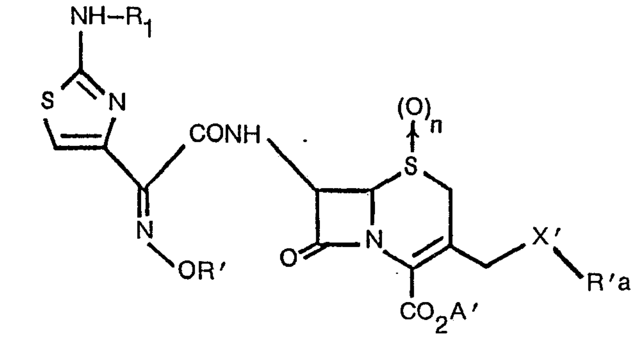

(IV)

worin $R_1$, R', A', R'a, X' und n die vorstehende Bedeutung besitzen, zu erhalten, welches Produkt man gewünschtenfalls, wenn n für 0 steht und X' ein Schwefel- oder Sauerstoffatom bedeutet, mit einem Oxidationsmittel behandelt, um ein Produkt der Formel (IV) zu erhalten, worin n für 1 oder 2 steht und X' ein in Form des Sulfoxids oder Sulfons oxidiertes Schwefelatom oder ein Sauerstoffatom bedeutet, und das Produkt der Formel (IV) erforderlichen- oder gewünschtenfalls einer oder mehreren der folgenden Reaktionen in beliebiger Reihenfolge unterzieht

(a) Spaltung durch Hydrolyse, Hydrogenolyse oder durch Umsetzung mit Thioharnstoff der Estergruppe oder der Amino-Schutzgruppe oder Amino-Schutzgruppen,
(b) Veresterung oder Salzbildung mit einer Base des Carboxylrestes oder der Carboxylreste,
(c) Überführung des Aminorestes oder der Aminoreste in ein Salz mit einer Säure.

2. Verfahren zur Herstellung der Produkte der Formel (I') gemäß Anspruch 1, dadurch gekennzeichnet, daß man ein Produkt der Formel (V)

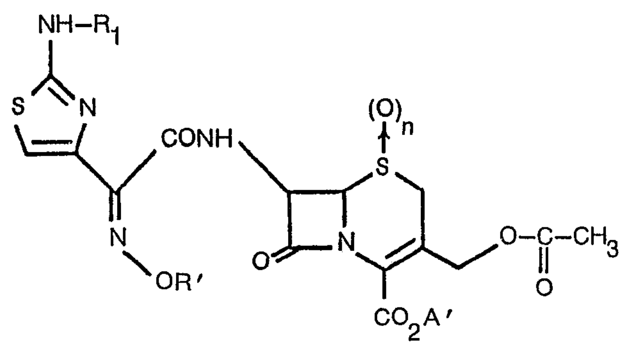

(V)

worin $R_1$, R', A' und n die in Anspruch 1 angegebene Bedeutung besitzen, *entweder* mit einem Produkt der Formel R'a—SH, worin R'a die in Anspruch 1 angegebene Bedeutung besitzt, *oder* zunächst mit 2-Mercaptopyridin-N-oxid und dann mit einem Produkt der Formel R'aOH, worin R'a die vorstehende Bedeutung besitzt, behandelt, um ein Produkt der Formel (IV), wie vorstehend definiert, zu erhalten, das Produkt der Formel (IV) gewünschtenfalls, wenn n für 0 steht und X' ein Schwefel- oder Sauerstoffatom bedeutet, mit einem Oxidationsmittel behandelt, um ein Produkt der Formel (IV) zu erhalten, worin n für 1 oder 2 steht und X' ein in Form des Sulfoxids oder Sulfons oxidiertes Schwefelatom oder ein Sauerstoffatom bedeutet, und das Produkt der Formel (IV) erforderlichenoder gewünschtenfalls einer oder mehreren der folgenden Reaktionen in beliebiger Reihenfolge unterzieht

(a) Spaltung durch Hydrolyse, Hydrogenolyse oder durch Umsetzung mit Thioharnstoff der Estergruppe oder der Amino- oder Hydroxyl-Schutzgruppen,
(b) Veresterung des Carboxylrestes oder seine Überführung in ein Salz mit einer Base,
(c) Überführung des Aminorestes in ein Salz mit einer Säure.

3. Verfahren gemäß Anspruch 1 zur Herstellung von Produkten der Formel (I'), dadurch gekennzeichnet, daß man für die Durchführung des Verfahrens ein Produkt der Formel (III) verwendet, worin $R_1$ eine Amino-Schutzgruppe bedeutet, und daß das funktionelle Derivat der Säure der Formel (III) ein gemischtes Carbonsäure-Sulfonsäureanhydrid ist.

4. Verfahren gemäß einem der Ansprüche 1, 2 und 3 zur Herstellung der Produkte gemäß Anspruch 1 der Formel (I)

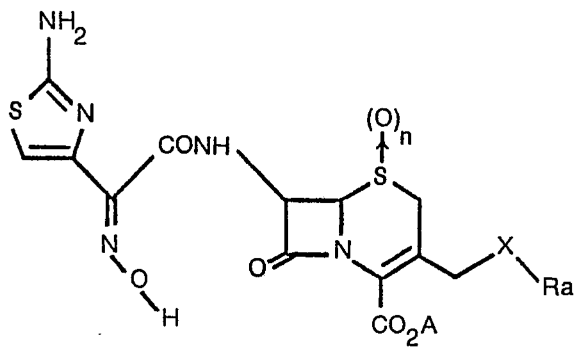

(I)

syn-Isomere

worin A und n wie in Anspruch 1 definiert sind, Ra einen linearen oder verzweigten Alkyl-, Alkenyl- oder Alkinylrest mit höchstens 6 Kohlenstoffatomen oder einen Benzyloder Phenethylrest, der gegebenenfalls mit einem Carboxy-, Amino-, Aminoalkyl-, Alkylamino-, Dialkylamino-, Dialkylaminoalkylrest substituiert ist, bedeutet und X ein Schwefel- oder Sauerstoffatom darstellt, sowie der Salze der Produkte der Formel (I) mit Mineral- oder organischen Säuren, dadurch gekennzeichnet, daß man von Produkten der Formel (II), R'aSH und R'aOH ausgeht, worin X' und R'a die vorstehend für X und Ra angegebenen Bedeutungen besitzen.

5. Verfahren zur Herstellung der Produkt der Formel (I') gemäß Anspruch 1, dadurch gekennzeichnet, daß man ein Produkt der Formel (II')

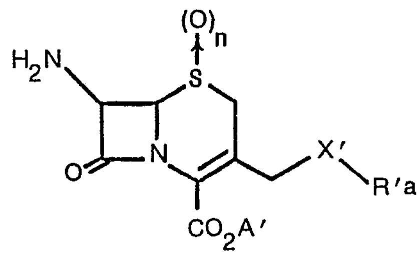

(II')

worin R'a, n, X' und A' die in Anspruch 1 angegebenen Bedeutungen besitzen, mit einem Produkt der Formel (III')

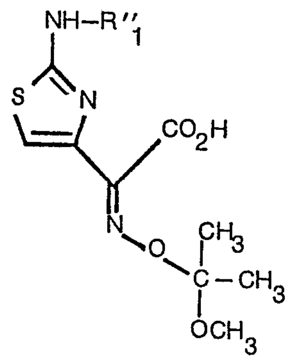

(III')

oder einem funktionellen Derivat dieser Säure behandelt, wobei in der Formel (III') $R''_1$ eine Amino-Schutzgruppe bedeutet, um ein Produkt der Formel (IV')

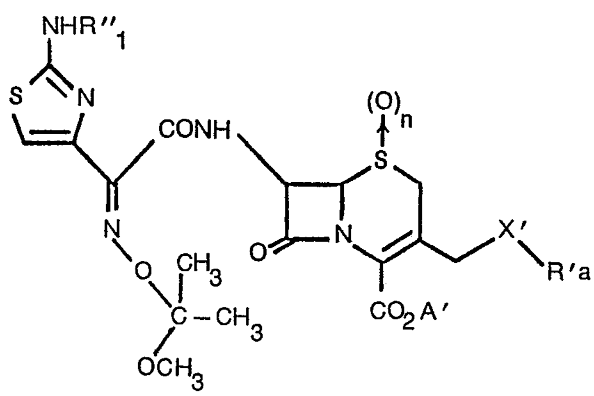

(IV')

zu erhalten, worin A', R''$_1$, R'a, X' und n die vorstehenden Bedeutung besitzen, welches Produkt der Formel (IV') man mit einer Säure unter mäßigen Bedingungen behandelt, um ein Produkt der Formel (VI)

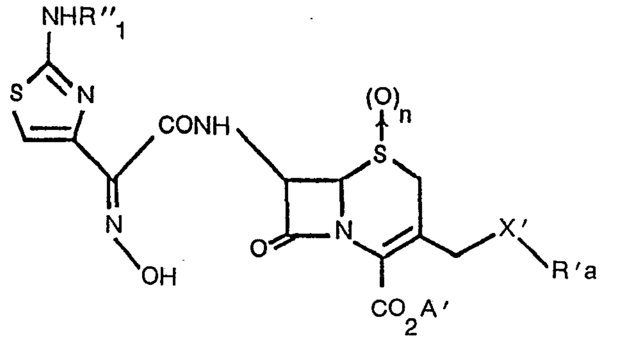

(VI)

zu erhalten, welches Produkt man gewünschtenfalls verestert oder in ein Salz überführt, und das Produkt der Formel (VI) einer Hydrolyse, Hydrogenolyse oder einer Umsetzung mit Thioharnstoff unterzieht, um die Amino-Schutzgruppe R''$_1$ zu eliminieren und gewünschten- oder erforderlichenfalls einer oder mehreren der folgenden Reaktionen in beliebiger Reihenfolge unterzieht

(a) einer Eliminierung der Estergruppe,
(b) einer Veresterung des Carboxylrestes oder seine Überführung in ein Salz mit einer Base,
(c) Salzbildung des Aminorestes mit einer Säure.

6. Verfahren gemäß Anspruch 4, dadurch gekennzeichnet, daß in dem Produkt der Formel (III) R' ein Wasserstoffatom oder eine Hydroxyl-Schutzgruppe bedeutet und daß in dem Produkt der Formel (II) oder in dem Produkt der Formel R'aSH oder R'aOH R'a einen Alkylrest mit höchstens 6 Kohlenstoffatomen bedeutet.

7. Verfahren gemäß Anspruch 5, dadurch gekennzeichnet, daß R'a einen Methylrest bedeutet und daß in dem Produkt der Formel (II') n für 0 steht.

8. Verfahren gemäß Anspruch 4, dadurch gekennzeichnet, daß die eventuelle letztendliche Veresterung mit einem funktionellen Derivat der Gruppe der Formel

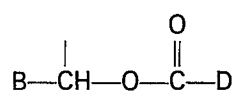

durchgeführt wird, worin B ein Wasserstoffatom oder einen linearen oder verzweigten Alkylrest mit 1 bis 5 Kohlenstoffatomen bedeutet und D einen linearen oder verzweigten Alkyl- oder Alkoxyrest mit 1 bis 15 Kohlenstoffatomen darstellt.

9. Verfahren gemäß Anspruch 8, dadurch gekennzeichnet, daß D einen linearen oder verzweigten Alkyl- oder Alkoxyrest mit 1 bis 5 Kohlenstoffatomen bedeutet,

10. Verfahren zur Herstellung der Produkte der Formel (I') gemäß einem der Ansprüche 6 bis 9, dadurch gekennzeichnet, daß man gemäß dem Verfahren des Anspruchs 5 arbeitet.

11. Verfahren gemäß Anspruch 4 oder 10, dadurch gekennzeichnet, daß man das folgende, der Formel (I) entsprechende Produkt herstellt: 3-Methoxymethyl-7-[[2-(2-aminothiazol-4-yl)-2-hydroxyiminoacetyl]-amino]-ceph-3-em-4-carbonsäure sowie ihre Salze mit Alkali- oder Erdalkalimetallen, Magnesium, Ammoniak, organischen Stickstoffbasen, Säuren und leicht spaltbaren Estern.

12. Verfahren gemäß Anspruch 4 oder 10, dadurch gekennzeichnet, daß man das folgende, der Formel (I) entsprechende Produkt herstellt: 1-Acetyloxyethyl-3-methoxymethyl-7-[[2-(2-aminothiazol-4-yl)-2-hydroxyiminoacetyl]-amino]-ceph-3-em-4-carboxylat sowie dessen Salze mit Mineral- oder organischen Säuren.

**Claims for the Contracting States: BE, CH, DE, GB, IT, LI, LU, NL, SE.**

1. The products with the general formula (I'):

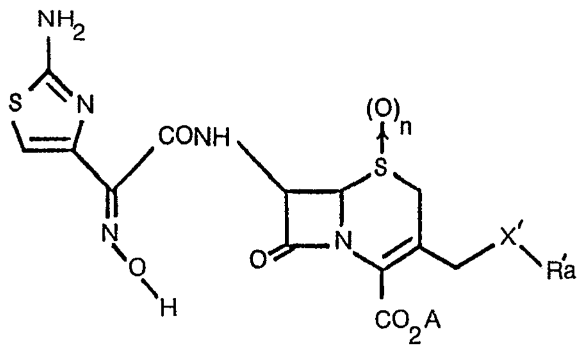

*syn* isomers

(I')

in which

A represents a hydrogen atom, an equivalent of an alkali or alkaline earth metal, magnesium, ammonium or aminated organic base, or A represents the residue of an easily cleavable ester group,

R'a represents an alkyl radical, possibly interrupted by a hetero-atom, an alkenyl or alkynyl radical, with at most 6 carbon atoms, linear or branched or a benzyl or phenylethyl radical, possibly substituted by a carboxy, amino, aminoalkyl, alkylamino, dialkylamino, or dialkylaminoalkyl radical,

n is 0, 1 or 2,

X' represents a sulphur atom, possibly oxidized in the form of sulphoxide or sulphone, or an oxygen atom, as well as the salts of the products with the formula (I') with mineral or organic acids.

2. The products according to Claim 1, corresponding to the formula (I):

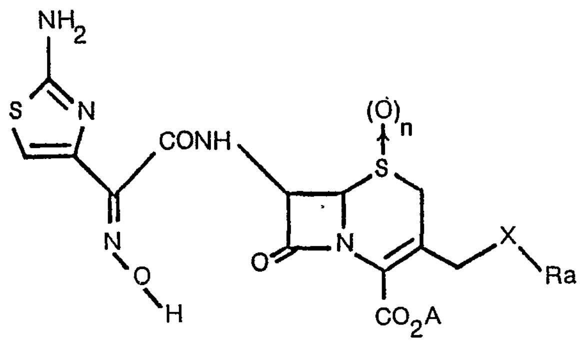

*syn* isomers

(I)

in which A and n are defined as in Claim 1, Ra represents an alkyl, alkenyl or alkynyl radical with at most 6 carbon atoms, linear or branched, or a benzyl or phenylethyl radical, possibly substituted by a carboxy, amino, aminoalkyl, alkylamino, dialkylamino or dialkylaminoalkyl radical, and X represents a sulphur atom or an oxygen atom, as well as the salts of the products with the formula (I) with mineral or organic acids.

3. The products with the general formula (I) as defined in Claim 2, in which Ra represents an alkyl radical with at most 6 carbon atoms.

4. The products with the general formula (I) as defined in Claim 2 or 3, in which Ra represents a methyl radical and n represents 0.

5. The products according to Claim 2, corresponding to the general formula ($I_A$):

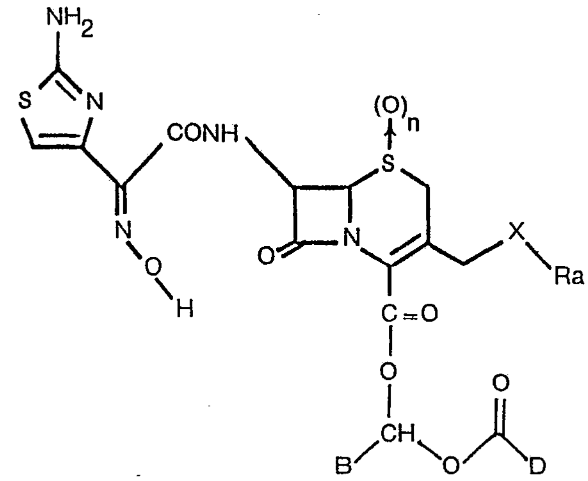

$(I_A)$

in which n, X and Ra are defined as in Claim 2, B represents a hydrogen atom or an alkyl radical, linear or branched, containing 1—5 carbon atoms and D represents an alkyl or alkoxy radical, linear or branched, containing 1—15 carbon atoms, as well as their salts with mineral or organic acids.

6. The products according to Claim 5, characterized in that D represents an alkyl or alkoxy radical, linear or branched, containing 1—5 carbon atoms.

7. 3-methoxymethyl-7-[[2-(2-aminothiazol-4-yl)-2-hydroxyiminoacetyl]-amino]-ceph-3-em-4-carboxylic acid, as well as its salts with alkali metals, alkaline earth metals, magnesium, ammonium, aminated organic bases, the acids and its easily cleavable esters.

8. 3-methoxymethyl-7-[[2-(2-aminothiazol-4-yl)-2-hydroxyiminoacetyl]-amino]-ceph-3-em-4-carboxylate of 1-acetyloxyethyl, as well as its salts with mineral or organic acids.

9. Preparation process for the products with the formula (I') as defined in Claim 1, characterized in that a product with the formula (II):

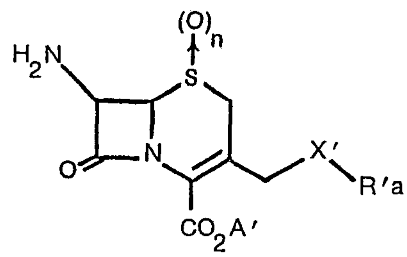

(II)

in which n, X' and R'a have the significance indicated in Claim 1 and A' represents a hydrogen atom or the residue of an easily eliminated ester group, is treated with a product with the formula (III):

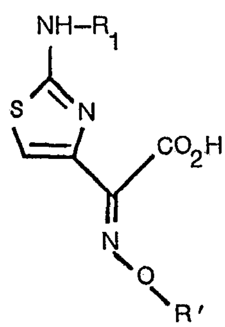

(III)

or a functional derivative of this acid, in which formula (III) $R_1$ represents a hydrogen atom or a protective group of the amino radical and R' represents a hydrogen atom or a protective group of the hydroxyl radical, so as to obtain a product with the formula (IV):

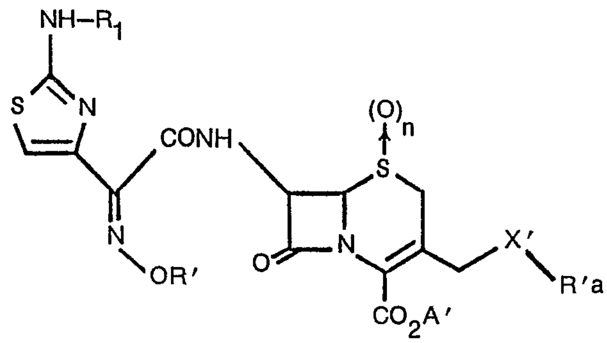

(IV)

in which $R_1$, R′, A′, R′a, X′ and n have the preceding significance, which product, if desired, when n is 0 and X′ represents a sulphur or oxygen atom, is treated with an oxidation agent, so as to obtain a product with the formula (IV) in which n is 1 or 2 and X′ represents an oxidized sulphur atom in the form of sulphoxide or sulphone or an oxygen atom and, which product with the formula (IV) is submitted, if necessary, or if desired, to one or more of the following reactions, in any order:

a) cleavage by hydrolysis, hydrogenolysis or by action of thiourea of the ester group or of the protective group of the amino radical,
b) esterification or salification of the carboxyl radical by a base,
c) salification of the amino radical by an acid.

10. Preparation process for the products with the formula (I′) as defined in Claim 1, characterized in that a product with the formula (V):

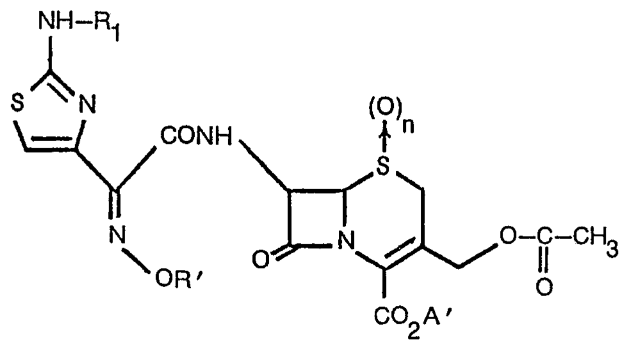

(V)

in which $R_1$, R′ and A′ have the significance indicated in Claim 9 and n has the significance indicated in Claim 1, is treated *either* by a product with the formula R′a—SH in which R′a has the significance indicated in Claim 1, *or* firstly with 2-mercapto pyridine-N-oxide then with a product with the formula R′aOH in which R′a has the preceding significance, so as to obtain a product with the formula (IV), as previously defined, which product with the formula (IV), if desired, when n is 0 and X′ represents a sulphur or oxygen atom, is treated with an oxidation agent, so as to obtain a product with the formula (IV) in which n is 1 or 2 and X′ represents an oxidized sulphur atom in the form of sulphoxide or sulphone or an oxygen atom and, which product with the formula (IV) is submitted, if necessary or if desired, to one or more of the following reactions, in any order:

a) cleavage by hydrolysis, hydrogenolysis or by action of thiourea of the ester group or of the protective groups of the amino radical or the hydroxyl radical,
b) esterification or salification of the carboxyl radical by a base,
c) salification of the amino radical by an acid.

11. Process according to Claim 9 for preparation of the products with the formula (I′), characterized in that a product with the formula (III) is used to put the process into operation, in which $R_1$ represents a protective group of the amino radical and in that the functional derivative of the acid of formula (III) is a mixed carboxylic sulphonic anhydride.

12. Preparation process for the products with the formula (I′), as defined in Claim 1, characterized in that a product with the formula (II′):

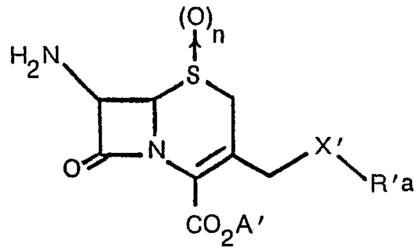

(II')

in which R'a, n, X' and A' have the significance indicated in Claim 1, is treated with a product with the formula (III'):

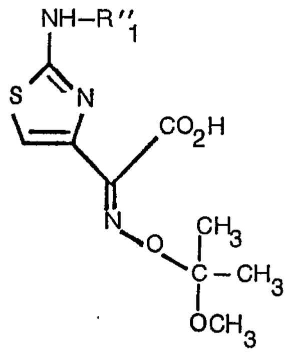

(III')

or a functional derivative of this acid, in which formula (III') $R''_1$ represents a protective group of the amino radical, so as to obtain a product with the formula (IV'):

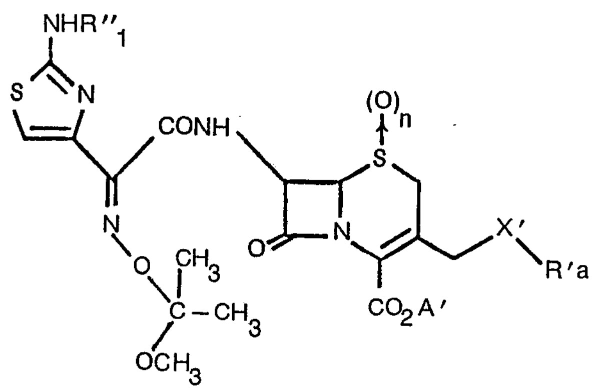

(IV')

in which A', $R''_1$, R'a, X' and n have the preceding significance, which product with the formula (IV') is treated with an acid in moderate conditions, so as to obtain a product with the formula (VI):

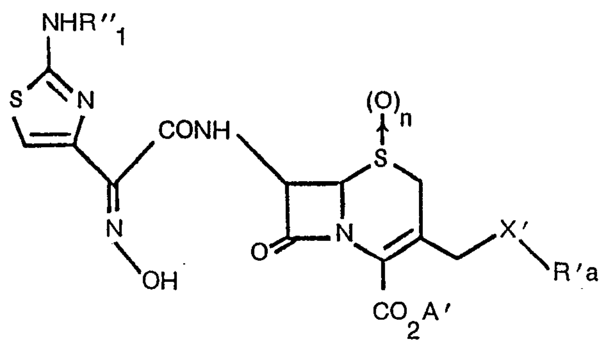

(VI)

which product, if desired, is esterified or salified and which product with the formula (VI) is submitted to hydrolysis, hydrogenolysis or to the action of thiourea so as to eliminate the protective radical $R''_1$ of the amino radical and, if desired or if necessary, to one or more of the following reactions in any order:

a) elimination of the ester group,
b) esterification or salification of the carboxyl radical by a base,
c) salification of the amino radical by an acid.

13. As medicaments, the products corresponding to the formula (I') as defined in Claim 1, as well as their pharmaceutically acceptable salts of acids.

14. As medicaments, the products with the formula (I) as defined in any one of Claims 2, 3 and 4 as well as their pharmaceutically acceptable salts of acids.

15. As medicaments, the products as defined in Claim 5 or 6, as well as their pharmaceutically acceptable salts of acids.

16. As medicaments, the products as defined in Claim 7 or 8 as well as their pharmaceutically acceptable salts.

17. Pharmaceutical compositions containing as active principle at least one of the medicaments according to one of Claims 13 to 16.

**Claims for the Contracting State: AT**

1. Process for preparing the products with the general formula (I')

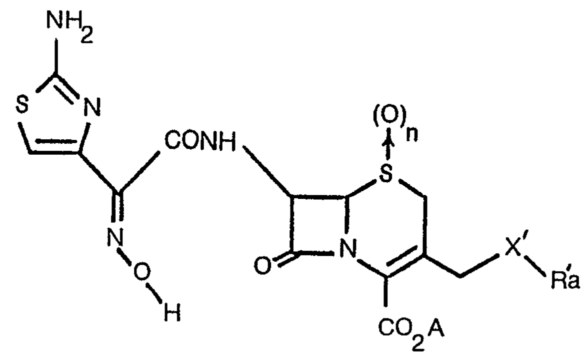

(I')

*syn* isomers

in which

A represents a hydrogen atom, an equivalent of an alkali or alkaline earth metal, magnesium, ammonium or aminated organic base, or A represents the residue of an easily cleavable ester group,

R'a represents an alkyl radical possibly interrupted by a hetero-atom, an alkenyl or alkynyl radical with at most 6 carbon atoms, linear or branched, or a benzyl or phenylethyl radical, possibly substituted by a carboxy, amino, aminoalkyl, alkylamino, dialkylamino, dialkylaminoalkyl radical,

n is 0, 1 or 2,

X' represents a sulphur atom, possibly oxidized in the form of sulphoxide or sulphone, or an oxygen atom, as well as the salts of the products with the formula (I') with mineral or organic acids, characterized in that a product with the formula (II):

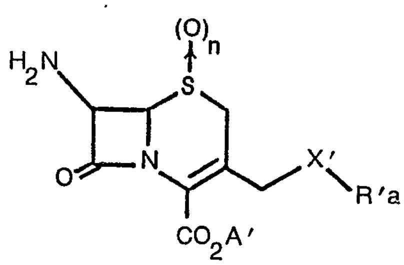

(II)

in which n, X' and R'a have the significance previously indicated and A' represents a hydrogen atom or the residue of an easily eliminated ester group, is treated with a product with the formula (III):

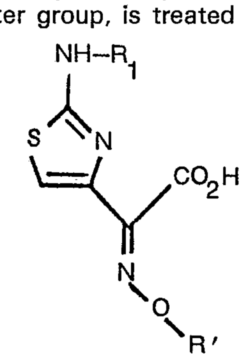

(III)

or a functional derivative of this acid, in which formula (III) $R_1$ represents a hydrogen atom or a protective group of the amino radical, R' represents a hydrogen atom or a protective group of the hydroxyl radical, so as to obtain a product with the formula (IV):

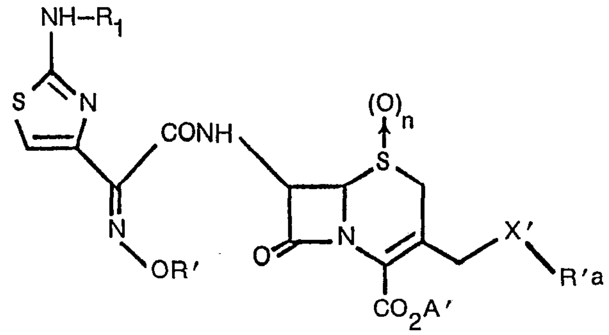

(IV)

in which $R_1$, R', A', R'a, X' and n have the preceding significance, which product, if desired, when n is 0 and X' represents a sulphur or oxygen atom, is treated with an oxidation agent, so as to obtain a product with the formula (IV) in which n is 1 or 2 and X' represents an oxidized sulphur atom in the form of sulphoxide or sulphone or an oxygen atom, which product with the formula (IV) is submitted, if necessary or if desired, to one or more of the following reactions, in any order:

a) cleavage by hydrolysis, hydrogenolysis or by action of thiourea of the ester group or the protective groups of the amino radical(s),
b) esterification or salification of the carboxyl radical(s) by a base,
c) salification of the amino radical(s) by an acid.

2. Process for preparing the products with the formula (I') as defined in Claim 1, characterized in that a product with the formula (V):

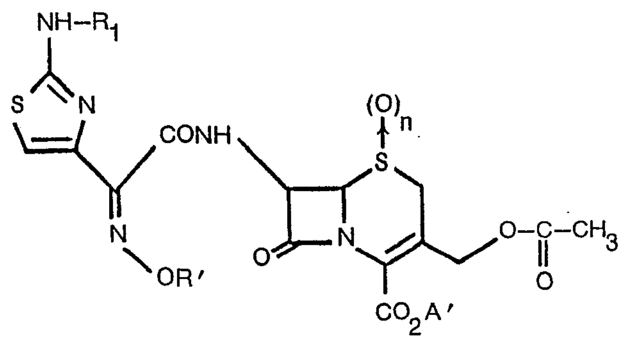

(V)

in which $R_1$, R', A' and n have the significance indicated in Claim 1 is treated *either* with a product with the formula R'a—SH in which R'a has the significance indicated in Claim 1, *or* firstly with 2-mercapto pyridine-N-oxide, then with a product with the formula R'aOH in which R'a has the preceding significance, so as to obtain a product with the formula (IV), as defined previously, which product with the formula (IV), if desired, when n is 0 and X' represents a sulphur or oxygen atom, is treated with an oxidation agent, so as to obtain a product with the formula (IV) in which n is 1 or 2 and X' represents an oxidized sulphur atom in the form of sulphoxide or sulphone or an oxygen atom and, which product with the formula (IV) is submitted, if necessary or if desired, to one or more of the following reactions, in any order:

a) cleavage by hydrolysis, hydrogenolysis or by action of thiourea of the ester group of the protective groups of the amino radical or the hydroxyl radical,
b) esterification or salification of the carboxyl radical by a base,
c) salification of the amino radical by an acid.

3. Process according to claim 1 for the preparation of products with the formula (I'), characterized in that for putting the process into operation a product with the formula (III) is used in which $R_1$ represents a protective group of the amino radical and in that the functional derivative of the acid with the formula (III) is a mixed carboxylic sulphonic anhydride.

4. Process according to any one of Claims 1, 2 and 3, for preparing the products according to claim 1, corresponding to the general formula (I):

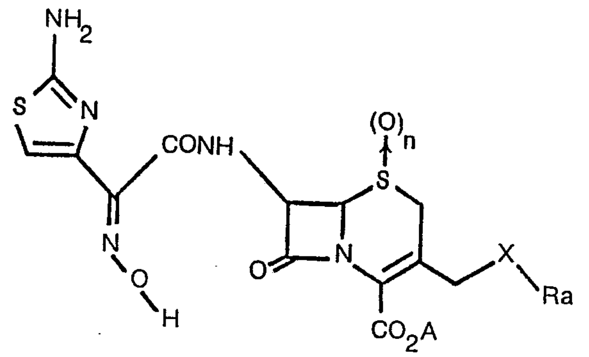

syn isomers

(I)

in which A and n are defined as in Claim 1, Ra represents an alkyl, alkenyl or alkynyl radical with at most 6 carbon atoms, linear or branched, or a benzyl or phenylethyl radical, possibly substituted by a carboxy, amino, aminoalkyl, alkylamino, dialkylamino, or dialkylaminoalkyl radical and X represents a sulphur atom or an oxygen atom, as well as the salts of the products with the formula (I) with mineral or organic acids, characterized in that products with the formulae (II), R'aSH and R'aOH are used at the start, in which X' and R'a have the values previously indicated for X and Ra.

5. Preparation process for the products with the formula (I'), as defined in Claim 1, characterized in that a product with the formula (II'):

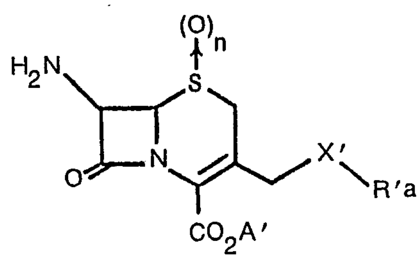

(II')

in which R'a, n, X' and A' have the significance indicated in Claim 1, is treated with a product with the formula (III'):

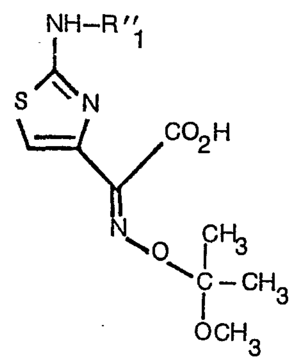

(III')

or a functional derivative of this acid, in which formula (III'), $R''_1$ represents a protective group of the amino radical, so as to obtain a product with the formula (IV'):

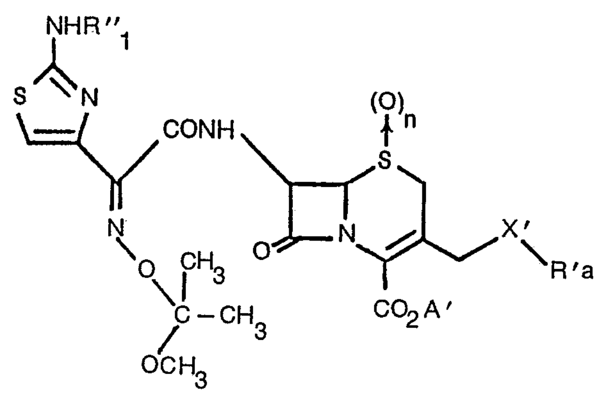

(IV')

in which A′, R″$_1$, R′a, X′ and n have the preceding significance, which product with the formula (IV′) is treated with an acid in moderate conditions, so as to obtain a product with the formula (VI):

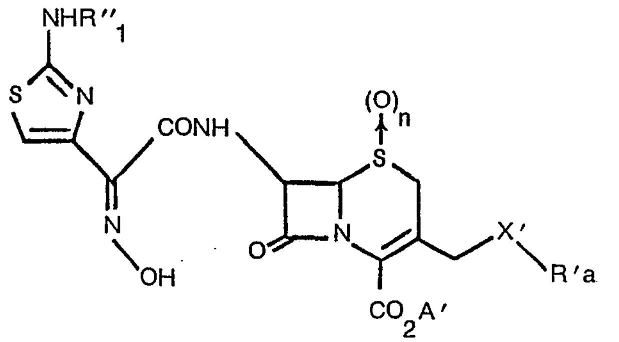

(VI)

which product, if desired, is esterified or salified and which product with the formula (VI) is submitted to hydrolysis, hydrogenolysis or the action of thiourea so as to eliminate the protective radical R″$_1$ of the amino radical and, if desired, or if necessary, to one or more of the following reactions, in any order:

a) elimination of the ester group,
b) esterification or salification of the carboxyl radical by a base,
c) salification of the amino radical by an acid.

6. Process according to claim 4, characterized in that in the product with the formula (III), R′ represents a hydrogen atom, or a protective group of the hydroxyl radical and in that in the product with the formula (II) or in the product with the formula R′aSH or R′aOH, R′a represents an alkyl radical with at most 6 carbon atoms.

7. Process according to Claim 5, characterized in that R′a represents a methyl radical and in that in the product with the formula (II′) n is 0.

8. Process according to claim 4, characterized in that the possible final esterification is carried out by a functional derivative of the group with the formula

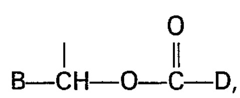

in which B represents a hydrogen atom or an alkyl radical, linear or branched, containing 1—5 carbon atoms, and D represents an alkyl or alkoxy radical, linear or branched, containing 1—15 carbon atoms.

9. Process according to Claim 8, characterized in that D represents an alkyl or alkoxy radical, linear or branched, containing 1—5 carbon atoms.

10. Preparation process for the products with the formula (I′), as obtained in any one of Claims 6—9, characterized in that the operation is carried out according to the process of Claim 5.

11. Process according to Claim 4 or 10, characterized in that the product is prepared corresponding to the following formula (I):

— 3-methoxymethyl-7-[[2-(2-aminothiazol-4-yl)-2-hydroxyiminoacetyl]-amino]-ceph-3-em-4-carboxylic acid as well as its salts with alkali metals, alkaline earth metals, magnesium, ammonium, aminated organic bases, the acids and its easily cleavable esters.

12. Process according to claim 4 or 10, characterized in that the product is prepared corresponding to the following formula (I):

— 3-methoxymethyl-7-[[2-(2-aminothiazol-4-yl)-2-hydroxyiminoacetyl]-amino]-ceph-3-em-4-carboxylate of 1-acetyloxyethyl, as well as its salts with mineral or organic acids.